(19) Europäisches Patentamt
European Patent Office
Office européen des brevets

(11) Publication number: **0 522 504 A1**

(12) **EUROPEAN PATENT APPLICATION**

(21) Application number: **92111497.1**

(22) Date of filing: **07.07.92**

(51) Int. Cl.5: **C07D 477/00**, A61K 31/40

(30) Priority: **09.07.91 JP 194767/91**
**07.12.91 JP 349591/91**

(43) Date of publication of application:
**13.01.93 Bulletin 93/02**

(84) Designated Contracting States:
**AT BE CH DE DK ES FR GB GR IT LI LU MC NL PT SE**

(71) Applicant: **Banyu Pharmaceutical Co., Ltd.**
**2-3, 2-chome Nihonbashi Honcho**
**Chuo-ku Tokyo(JP)**

(72) Inventor: **Nakagawa, Susumu, c/o Banyu Pharmaceut. Co. Ltd.**
**Tsukuba Research Inst., 3, Okubo**
**Tsukuba-shi, Ibaraki-ken 300-33(JP)**
Inventor: **Ohtake, Norikazu, c/o Banyu Pharmaceut. Co. Ltd.**
**Tsukuba Research Inst., 3, Okubo**
**Tsukuba-shi, Ibaraki-ken 300-33(JP)**
Inventor: **Okada, Shigemitsu, c/o Banyu Pharmaceut. Co. Ltd.**
**Tsukuba Research Inst., 3, Okubo**
**Tsukuba-shi, Ibaraki-ken 300-33(JP)**
Inventor: **Ushijima, Ryosuke, c/o Banyu Pharmaceut. Co. Ltd.**
**Tsukuba Research Inst., 3, Okubo**
**Tsukuba-shi, Ibaraki-ken 300-33(JP)**

(74) Representative: **Wächtershäuser, Günter, Dr.**
**Tal 29**
**W-8000 München 2(DE)**

(54) **2-(2-substituted pyrrolidinylthio)-carbapenem derivatives.**

(57) A compound of the formula (I):

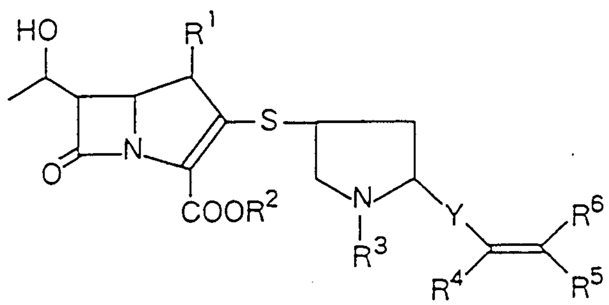

(I)

wherein $R^1$ is a hydrogen atom or a methyl group, $R^2$ is a hydrogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula $-X-N(R^7)R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula $-X-N(R^7)R^8$ wherein $R^7$, $R^8$ and X are as

EP 0 522 504 A1

defined above, and Y is a lower alkylene group or a single bond; or a pharmaceutically acceptable salt or ester thereof.

## TECHNICAL FIELD

The present invention relates to novel carbapenem (7-oxo-1-azabicyclo[3.2.0]hept-2-en-2-carboxylic acid) compounds, antibacterial agents containing such compounds as active ingredients, and a process for producing such compounds.

## BACKGROUND ART

In recent years, new β-lactam antibiotic substances have been found in nature which have the same β-lactam rings as penicillin derivatives and as cephalosporin derivatives, but which have different basic structures.

For example, naturally derived carbapenem compounds such as thienamycin isolated from the fermentation of Streptomyces cattleya (J. Am. Chem. Soc., vol. 100, p.6491 (1978)), may be mentioned. Thienamycin has an excellent antibacterial spectrum and strong antibacterial activities over a wide range against gram positive bacteria and gram negative bacteria. Therefore, its development as a highly useful β-lactam agent has been expected. However, thienamycin itself is chemically unstable, and it has been reported that it is likely to be decomposed by a certain enzyme in vivo such as renal dehydropeptidase I (hereinafter referred to simply as DHP-I), whereby the antibacterial activities tend to decrease, and the recovery rate in the urine is low (Antimicrob. Agents Chemother., vol. 22, p.62 (1982); ditto, vol. 23, p.300 (1983)).

Merck & Co., Inc. have synthesized many thienamycin analogues with an aim to maintain the excellent antibacterial activities of thienamycin and to secure chemical stability. As a result, imipenem, (5R,6S,8R)-3-[[2-(formimidoylamino)ethyl]thio]-6-(1-hydroxyethyl)-7-oxo-1-azabicyclo[3.2.0]hept-2-en-2-carboxylic acid monohydrate, obtained by formimidation of the amino group of thienamycin, has been practically developed as a pharmaceutical product (J. Med. Chem., vol. 22, p. 1435 (1979)). Imipenem has antibacterial activities of an equal or higher level than thienamycin against various types of bacteria and has β-lactamase resistance. Especially against Pseudomonas aeruginosa, its antibacterial activities are superior to thienamycin by from 2 to 4 times. Further, the stability of imipenem in the solid form or in an aqueous solution is remarkably improved over thienamycin.

However, like thienamycin, imipenem is likely to be decomposed by DHP-I in the human kidney. Therefore, it can not be used for treatment of the urinary-tract infection. Further, it presents toxicity against the kidney due to the decomposition products. Therefore, imipenem can not be administered alone and is required to be used in combination with a DHP-I inhibitor like cilastatin (J. Antimicrob. Chemother., vol. 12 (Suppl. D), p. 1 (1983)). In recent years, imipenem has been frequently used for the treatment and prevention of infectious diseases. Consequently, highly methicillin resistant Staphylococcus aureus which is resistant to imipenem and imipenem resistant Pseudomonas aeruginosa are increasing in the clinical field. Imipenem does not show adequate treating effects against these resistant bacteria.

As the prior art closest to the present invention, EP 411664A may be mentioned. This publication discloses carbapenem compounds having a 2-[2-(aminocarbonyl)vinyl]pyrrolidin-4-ylthio group at the 2-position of the carbapenem structure, represented by (1R,5S,6S)-2-[(2S,4S)-2-[(E)-2-(aminocarbonyl)vinyl]-pyrrolidin-4-ylthio]-6-[(R)-1-hydroxyethyl]-1-methyl-1-carbapen-2-em-3-carboxylic acid (Compound No. 145, compound of Example 3; hereinafter referred to simply as BO-2171), as a typical compound.

β-Lactam antibiotics exhibit selective toxicity against bacteria and show no substantial effects against animal cells. Therefore, they are widely used for treatment of infectious diseases caused by bacteria, as rare antibiotics having little side effects, and thus are highly useful drugs.

However, in recent years, highly methicillin resistant Staphylococcus aureus and resistant Pseudomonas aeruginosa have been isolated frequently from patients with the immunity decreased, as bacteria causing hardly curable infectious diseases. This is regarded as a clinically serious problem. Accordingly, it is strongly desired to develop an antibacterial agent having improved antibacterial activities against such resistant bacteria. Especially with respect to carbapenem compounds, it is desired to improve the antibacterial activities, to improve the stability against DHP-I, to reduce the toxicity against the kidney and to reduce side effects against the central nervous system.

The compounds disclosed in EP 411664A, have a certain level of stability against DHP-I. However, the antibacterial activities against the above-mentioned highly methicillin resistant Staphylococcus aureus and the stability against DHP-I are not adequate, and a carbapenem compound having superior antibacterial activities and stability against DHP-I, is desired.

## DISCLOSURE OF THE INVENTION

The present inventors have made extensive researches with an aim to provide novel carbapenem compounds which have excellent antibacterial activities and which are resistant against DHP-I. As a result, they have found that carbapenem compounds of the present invention having, at the 2-position of the carbapenem structure, a group of the formula:

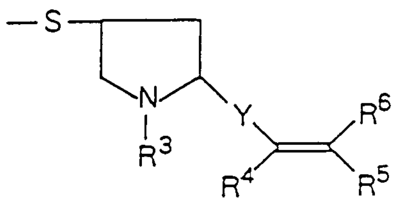

wherein $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula $-X-N(R^7)R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula $-X-N(R^7)R^8$ wherein $R^7$, $R^8$ and X are as defined above, and Y is a lower alkylene group or a single bond, are novel compounds not disclosed any literatures, and that such compounds have strong antibacterial activities against gram positive bacteria such as Staphylococcus aureus and against gram negative bacteria including Pseudomonas aeruginosa and further exhibit excellent stability against DHP-I. The present invention has been accomplished on the basis of this discovery.

The present invention provides a compound of the formula (I):

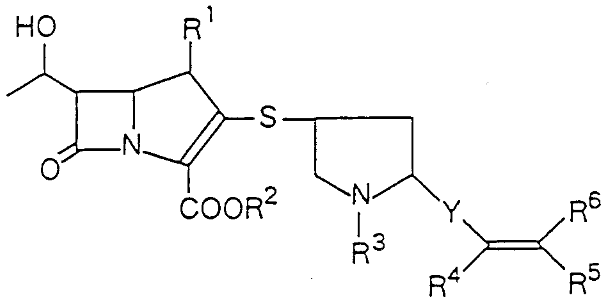

(I)

wherein $R^1$ is a hydrogen atom or a methyl group, $R^2$ is a hydrogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula $-X-N(R^7)R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula $-X-N(R^7)R^8$ wherein $R^7$, $R^8$ and X are as defined above, and Y is a lower alkylene group or a single bond; or a pharmaceutically acceptable salt or ester thereof.

The present invention also provides a process for producing the compound of the formula (I) or a pharmaceutically acceptable salt or ester thereof, which comprises reacting a compound of the formula (II):

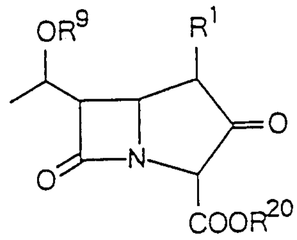

(II)

wherein $R^1$ is as defined above, $R^9$ is a hydrogen atom or a hydroxyl-protecting group, and $R^{20}$ is a hydrogen atom or a carboxyl-protecting group, or a reactive derivative thereof, with a compound of the formula (III):

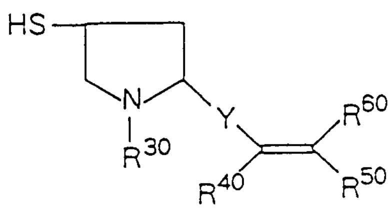

(III)

wherein $R^{30}$ is a hydrogen atom, a lower alkyl group or an imino-protecting group, each of $R^{40}$, $R^{50}$ and $R^{60}$ is a hydrogen atom, a cyano group, a methanesulfonyl group, a lower alkyl group which may have substituents, a carbamoyl group which may have substituents or a group of the formula $-X^1-N(R^{71})R^{81}$ {wherein each of $R^{71}$ and $R^{81}$ which may be the same or different, is a hydrogen atom, a lower alkyl group which may have substituents or an amino-protecting group, or $R^{71}$ and $R^{81}$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to $X^1$ or other nitrogen atom on the heterocyclic ring may form an ammonio group), and $X^1$ is a lower alkylene group which may have substituents}, provided that at least one of $R^{40}$, $R^{50}$ and $R^{60}$ is the group of the formula $-X^1-N(R^{71})R^{81}$ wherein $R^{71}$, $R^{81}$ and $X^1$ are as defined above, and Y is a lower alkylene group or a single bond (provided that the substituents of the lower alkyl group, the heterocyclic group and the lower alkylene group, as well as the carbamoyl group and the imino group on the heterocyclic ring, may be protected, as the case requires), to obtain a compound of the formula (IV):

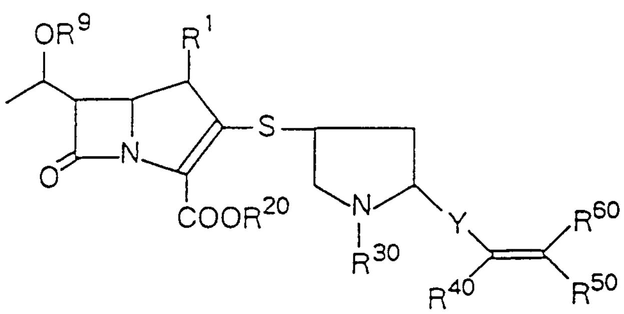

(IV)

wherein $R^1$, $R^9$, $R^{20}$, $R^{30}$, $R^{40}$, $R^{50}$ and $R^{60}$ are as defined above, and if necessary, removing any protecting group of the compound of the formula (IV).

Further, the present invention provides an antibacterial agent comprising an antibacterially effective amount of the compound of the formula (I) or a pharmaceutically acceptable salt or ester thereof, and a pharmaceutically acceptable carrier or diluent.

## BEST MODE FOR CARRYING OUT THE INVENTION

Now, the present invention will be described in detail with reference to the preferred embodiments.

Firstly, the symbols and terms used in this specification will be explained.

The compound of the present invention has a basic structure of the formula:

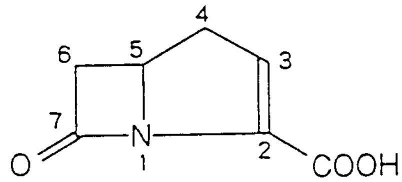

which is systematically referred to as a 7-oxo-1-azabicyclo[3.2.0]hept-2-en-2-carboxylic acid. For the convenience sake, in this specification, this basic structure will be referred to as a 1-carbapen-2-em-3-carboxylic acid by putting the numbers based on a commonly widely used carbapenem of the formula:

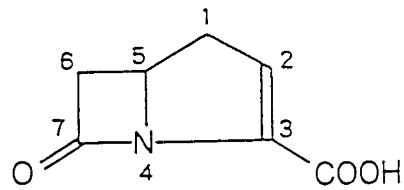

The present invention includes optical isomers based on the asymmetrical carbon atoms at the 1-position, 5-position, 6-position and 8-position of the carbapenem structure and stereoisomers. Among these isomers, preferred is a compound of a (5R,6S,8R) configuration i.e. a compound having a steric configuration of (5R,6S) (5,6-trans) like thienamycin and in which the carbon atom at the 8-position takes a R-configuration, or a compound of a (1R,5S,6S,8R) configuration in a case where a methyl group is present at the 1-position.

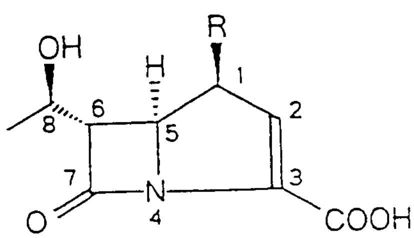

$R = H, -CH_3$

Also with respect to the 2-substituted pyrrolidin-4-ylthio group in the side chain at the 2-position, the present invention includes isomers based on the asymmetrical carbon atoms at the 2-position and 4-position of the pyrrolidine structure and in the side chain at the 2-position. Among these isomers, preferred are compounds of (2'S,4'S) configuration and (2'R,4'R) configuration.

Further, with respect to the side chain at the 2-position of the pyrrolidine structure, there exist isomers based on asymmetrical carbons, and the present invention includes such isomers as well.

The lower alkyl group means a linear or branched alkyl group having from 1 to 6 carbon atoms, such as a methyl group, an ethyl group, a propyl group, an isopropyl group, a butyl group, a sec-butyl group, a tert-butyl group, a pentyl group or a hexyl group. Among them, a methyl group, an ethyl group or a tert-butyl group, is preferred.

The lower alkyl group which may have substituents means the above-mentioned lower alkyl group which is unsubstituted, or a lower alkyl group having at least one substituent at an optional position substitutable on the above-mentioned lower alkyl group.

The substituents of the lower alkyl group may, for example, be a carbamoyl group; a hydroxyl group; an amino group; a trifluoromethyl group; a carbamoyloxy group; a lower alkyl carbamoyl group mono- or di-substituted by the above-mentioned lower alkyl group, such as a methyl carbamoyl group, an ethyl carbamoyl group, a dimethyl carbamoyl group or a diethyl carbamoyl group; a lower alkyl carbamoyloxy group mono- or di-substituted by the above-mentioned lower alkyl group, such as a methyl carbamoyloxy

group, an ethyl carbamoyloxy group, a dimethyl carbamoyloxy group or a diethyl carbamoyloxy group; a lower alkyl amino group mono- or di-substituted by the above-mentioned lower alkyl group, such as an N-methylamino group, an N-ethylamino group, an N,N-dimethylamino group or an N,N-diethylamino group; and a lower alkanoyl amino group such as an N-formylamino group or an N-acetylamino group.

Specific examples of the lower alkyl group which may have substituents, include a carbamoyl lower alkyl group such as a carbamoylmethyl group, a 1-carbamoylethyl group or a 2-carbamoylethyl group; a hydroxy lower alkyl group such as a hydroxymethyl group, a 1-hydroxyethyl group or a 2-hydroxyethyl group; an amino lower alkyl group such as an aminomethyl group, a 1-aminoethyl group or a 2-aminoethyl group; a trihalomethyl group such as a trifluoromethyl group; a carbamoyloxy lower alkyl group such as a carbamoyloxymethyl group, a 1-(carbamoyloxy)ethyl group or a 2-(carbamoyloxy)ethyl group; a (lower alkyl carbamoyl) lower alkyl group having a carbamoyl group mono- or di-substituted by the above-mentioned lower alkyl group, such as a (methylcarbamoyl)methyl group, an (ethylcarbamoyl)methyl group, a (dimethylcarbamoyl)methyl group or a (diethylcarbamoyl)methyl group; a (lower alkyl carbamoyloxy) lower alkyl group having a carbamoyloxy group mono- or di-substituted by the above lower alkyl group, such as a (methylcarbamoyloxy)methyl group, an (ethylcarbamoyloxy)methyl group, a (dimethylcarbamoyloxy)methyl group or a (diethylcarbamoyloxy)methyl group; a trifluoromethyl lower alkyl group such as a trifluoromethylmethyl group, a 1-(trifluoromethyl)ethyl group or a 2-(trifluoromethyl)ethyl group; a (lower alkyl amino) lower alkyl group having an amino group mono- or di-substituted by the above lower alkyl group, such as an N-methylaminomethyl group, an N-ethylaminomethyl group, an N,N-dimethylaminomethyl group or an N,N-diethylamino methyl group; and a (lower alkanoyl amino) lower alkyl group such as an N-formylaminomethyl group or an N-acetylaminomethyl group.

The heterocyclic group may, for example, be a 3- to 8-membered heterocyclic group such as an aziridinyl group, an azetidinyl group, a pyrrolidinyl group, a piperidino group, a piperazinyl group, a morpholino group, a thiomorpholino group, a hexahydro-1H-azepinyl group or an octahydroazocinyl group. Further, the above-mentioned lower alkyl group which may have substituents, may be bonded, or either the nitrogen atom adjacent to X or other nitrogen atom on the heterocyclic ring may form an ammonio group. Such a heterocyclic group may, for example, be an N-methylaziridinio group, an N-methylazetidinio group, an N-methylpyrrolidinio group, an N-methylpiperidinio group, a 1-methylpiperazinio group, a 4,4-dimethylpiperazinio group, an N-methylmorpholinio group, an N-methylthiomorpholinio group, an N-methylhexahydro-1H-azepinio group, or an N-methyloctahydroazocinio group. The heterocyclic group may have at least one substituent at an optional position substitutable on the heterocyclic ring.

The substituents of the heterocyclic ring may, for example, be a carbamoyl group; a cyano group; a methanesulfonyl group; an amino group; a lower alkanoyl group such as a formyl group or an acetyl group; a lower alkyl carbamoyl group mono- or di-substituted by the above-mentioned lower alkyl group, such as a methylcarbamoyl group, an ethylcarbamoyl group, a dimethylcarbamoyl group or a diethylcarbamoyl group; an amino group mono- or di-substituted by the above-mentioned lower alkyl group, such as an N-methylamino group, an N-ethylamino group, an N,N-dimethylamino group or an N,N-diethylamino group; a lower alkanoylamino group such as an N-formylamino group or an N-acetylamino group; and a lower alkyl group which may have the above-mentioned substituents.

The lower alkylene group means a linear or branched alkylene group having from 1 to 6 carbon atoms. For examples, a methylene group, an ethylene group, a propylene group, a 1-methylethylene group, a 2-methylethylene group, a methylmethylene group, a dimethylmethylene group, a butylene group, a 1-methylpropylene group, a 2-methylpropylene group, a 3-methylpropylene group, a 1,1-dimethylethylene group or a 2,2-dimethylethylene group, may be mentioned. Among them, a methylene group, an ethylene group or a propylene group is preferred.

The lower alkylene group which may have substituents, means the above-mentioned lower alkylene group which is unsubstituted or a lower alkylene group having at least one substituent at an optional position substitutable on the above-mentioned lower alkylene group.

The substituents of the lower alkylene group may, for example, be a carbamoyl group; a cyano group; a methanesulfonyl group; an amino group; a lower alkyl carbamoyl group mono- or di-substituted by the above-mentioned lower alkyl group, such as a methylcarbamoyl group, an ethylcarbamoyl group, a dimethylcarbamoyl group or a diethylcarbamoyl group; an amino group mono- or di-substituted by the above-mentioned lower alkyl group, such as an N-methylamino group, an N-ethylamino group, an N,N-dimethylamino group or an N,N-diethylamino group; a lower alkanoyl amino group such as an N-formylamino group or an N-acetylamino group; and the above-mentioned lower alkyl group which may have substituents.

The carbamoyl group which may have substituents, means an unsubstituted carbamoyl group or a lower alkyl carbamoyl group mono- or di-substituted by the above-mentioned lower alkyl group, such as a

methylcarbamoyl group, an ethylcarbamoyl group, a dimethylcarbamoyl group or a diethylcarbamoyl group.

The carboxyl-protecting group may, for example, be a lower alkyl group such as a methyl group, an ethyl group, a propyl group, an isopropyl group or a tert-butyl group; a halogenated lower alkyl group such as a 2,2,2-trichloroethyl group or a 2,2,2-trifluoroethyl group; a lower alkanoyloxyalkyl group such as an acetoxymethyl group, a propionyloxymethyl group, a pivaloyloxymethyl group, a 1-acetoxyethyl group or a 1-propionyloxyethyl group; a lower alkoxycarbonyloxyalkyl group such as a 1-(methoxycarbonyloxy)-ethylgroup, a 1-(ethoxycarbonyloxy)ethyl group or a 1-(isopropoxycarbonyloxy)ethyl group; a lower alkenyl group such as a 2-propenyl group, a 2-chloro-2-propenyl group, a 3-methoxycarbonyl-2-propenyl group, a 2-methyl-2-propenyl group, a 2-butenyl group or a cinnamyl group; an aralkyl group such as a benzyl group, a p-methoxybenzyl group, a 3,4-dimethoxybenzyl group, an o-nitrobenzyl group, a p-nitrobenzyl group, a benzhydryl group or a bis(p-methoxyphenyl)methyl group; a (5-substituted 2-oxo-1,3-dioxol-4-yl)-methyl group such as a (5-methyl-2-oxo-1,3-dioxol-4-yl)methyl group; a lower alkylsilyl group such as a trimethylsilyl group or a tert-butyldimethylsilyl group; an indanyl group, a phthalidyl group or a methoxymethyl group. Particularly preferred are a 2-propenyl group, a p-nitrobenzyl group, a p-methoxybenzyl group, a benzhydryl group and a tert-butyldimethylsilyl group.

The hydroxyl-protecting group may, for example, be a lower alkylsilyl group such as a trimethylsilyl group or a tert-butyldimethylsilyl group; a lower alkoxymethyl group such as a methoxymethyl group or a 2-methoxyethoxymethyl group; a tetrahydropyranyl group; an aralkyl group such as a benzyl group, a p-methoxybenzyl group, a 2,4-dimethoxybenzyl group, an o-nitrobenzyl group, a p-nitrobenzyl group or a trityl group; an acyl group such as a formyl group or an acetyl group; a lower alkoxycarbonyl group such as a tert-butoxycarbonyl group, a 2-iodoethoxycarbonyl group or a 2,2,2-trichloroethoxycarbonyl group; an alkenyloxycarbonyl group such as a 2-propenyloxycarbonyl group, a 2-chloro-2-propenyloxycarbonylgroup, a 3-methoxycarbonyl-2-propenyloxycarbonyl group, a 2-methyl-2-propenyloxycarbonyl group, a 2-butenyloxycarbonyl group or a cinnamyloxycarbonyl group; or an aralkyloxycarbonyl group such as a benzyloxycarbonyl group, a p-methoxybenzyloxycarbonyl group, an o-nitrobenzyloxycarbonyl group or a p-nitrobenzyloxycarbonyl group. Particularly preferred are a 2-propenyloxycarbonyl group, a p-nitrobenzyloxycarbonyl group and a tert-butyldimethylsilyl group.

The amino- or imino-protecting group may, for example, be an aralkylidene group such as a benzylidene group, a p-chlorobenzylidene group, a p-nitrobenzylidene group, a salicylidene group, an $\alpha$-naphthylidene group or a $\beta$-naphthylidene group; an aralkyl group such as a benzyl group, a p-methoxybenzyl group, a 3,4-dimethoxybenzyl group, an o-nitrobenzyl group, a p-nitrobenzyl group, a benzhydryl group, a bis(p-methoxyphenyl)methyl group or a trityl group; a lower alkanoyl group such as a formyl group, an acetyl group, a propionyl group, a butyryl group, an oxalyl group, a succinyl group, or a pivaloyl group; a halogenated lower alkanoyl group such as a chloroacetyl group, a dichloroacetyl group, a trichloroacetyl group or a trifluoroacetyl group; an arylalkanoyl group such as a phenylacetyl group or a phenoxyacetyl group; a lower alkoxycarbonyl group such as a methoxycarbonyl group, an ethoxycarbonyl group, a propoxycarbonyl group or a tert-butoxycarbonyl group; a halogenated lower alkoxycarbonyl group such as a 2-iodoethoxycarbonyl group or a 2,2,2-trichloroethoxycarbonyl group; an alkenyloxycarbonyl group such as a 2-propenyloxycarbonyl group, a 2-chloro-2-propenyloxycarbonyl group, a 3-methoxycarbonyl-2-propenyloxycarbonyl group, a 2-methyl-2-propenyloxycarbonyl group, a 2-butenyloxycarbonyl group or a cinnamyloxycarbonyl group; an aralkyloxycarbonyl group such as a benzyloxycarbonyl group, an o-nitrobenzyloxycarbonyl group, a p-nitrobenzyloxycarbonyl group or a phenethyloxycarbonyl group; or a lower alkylsilyl group such as a trimethylsilyl group or a tert-butyldimethylsilyl group. Particularly preferred are a 2-propenyloxycarbonyl group, a tert-butoxycarbonyl group and a p-nitrobenzyloxycarbonyl group.

Each of $R^4$, $R^5$ and $R^6$ which may be the same or different, is a hydrogen atom, a cyano group, a methanesulfonyl group, a lower alkyl group which may have substituents, a carbamoyl group which may have substituents or a group of the formula $-X-N(R^7)R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula $-X-N(R^7)R^8$ wherein $R^7$, $R^8$ and X are as defined above, the rest being a hydrogen atom, a cyano group, a methanesulfonyl group, a lower alkyl group which may have substituents or a carbamoyl group which may have substituents. Namely, the compound of the present invention is characterized in that at least one of $R^4$, $R^5$ and $R^6$ has a primary, secondary or tertiary amino group, or an ammonio group. Among them, a compound is preferred wherein $R^5$ or $R^6$ is a group of the formula $-(CH_2)_n-$

$N(R^{70})R^{80}$ wherein each of $R^{70}$ and $R^{80}$ which may be the same or different, is a hydrogen atom or a lower alkyl group, or $R^{70}$ and $R^{80}$ form, together with the adjacent nitrogen atom, a 3- to 8-membered heterocyclic group, and n is an integer of from 1 to 4. Further, a compound wherein $R^5$ or $R^6$ is a group of the formula $-CH_2-N(R^{77})R^{88}$ wherein each of $R^{77}$ and $R^{88}$ which may be the same or different, is a hydrogen atom or a lower alkyl group, or $R^{77}$ and $R^{88}$ form, together with the adjacent nitrogen atom, a 4- to 6-membered heterocyclic group, or a compound wherein each of $R^5$ and $R^6$ is a hydrogen atom, is more preferred. Particularly preferred is a compound wherein $R^5$ or $R^6$ is a group of the formula $-CH_2-NH_2$, $-CH_2-NHCH_3$ or $-CH_2-N(CH_3)_2$. The present invention also covers a case where in addition to $R^7$ and $R^8$, the above-mentioned lower alkyl group which may have substituents, is bonded to the nitrogen atom adjacent to X so that the nitrogen atom forms an ammonio group.

$R^2$ is a hydrogen atom or a negative charge. When the side chain at the 2-position of the pyrrolidine ring forms an ammonio group, $R^2$ represents a negative charge forming a pair with the ammonium ion, whereby the compound of the formula (I) forms an intramolecular salt.

Y is a lower alkylene group or a single bond, preferably a methylene group or a single bond. Particularly preferred is a single bond.

Now, the compound of the formula (I) will be described more specifically.

The compound of the formula (I) includes a compound of the formula (I-a):

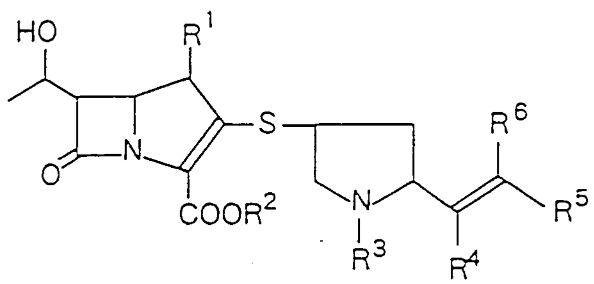

(I-a)

wherein $R^1$ is a hydrogen atom or a methyl group, $R^2$ is a hydrogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula $-X-N(R^7)R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8- membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula $-X-N(R^7)R^8$ wherein $R^7$, $R^8$ and X are as defined above; or a pharmaceutically acceptable salt or ester thereof, and a compound of the formula (I-b):

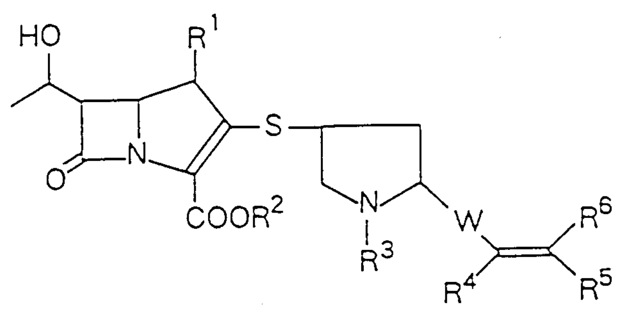

(I-b)

wherein $R^1$ is a hydrogen atom or a methyl group, $R^2$ is a hydogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula $-X-N(R^7)R^8$ {wherein each of $R^7$ and $R^8$ which may be the same or different, is a hydrogen atom or a lower alkyl group which may have substituents, or $R^7$ and $R^8$

form, together with the adjacent nitrogen atom and, if necessary, together with a hetero atom selected from the group consisting of a nitrogen atom, an oxygen atom and a sulfur atom in addition to the adjacent nitrogen atom, a 3- to 8- membered heterocyclic group which may be substituted (provided that either the nitrogen atom adjacent to X or other nitrogen atom on the heterocyclic ring may form an ammonio group), and X is a lower alkylene group which may have substituents, provided that at least one of $R^4$, $R^5$ and $R^6$ is the group of the formula -X-N($R^7$)$R^8$ wherein $R^7$, $R^8$ and X are as defined above, and W is a lower alkylene group; or a pharmaceutically acceptable salt or ester thereof.

The salt of the compound of the formula (I) is a common pharmaceutically acceptable salt and may, for example, be a salt at the carboxyl group at the 3-position of the carbapenem structure, or at the pyrrolidine base at the 2-position of the carbapenem structure or the base on the side chain substituted on the pyrrolidine ring.

The basic addition salt at said carboxyl group includes, for example, an alkali metal salt such as a sodium salt or a potassium salt; an alkaline earth metal salt such as a calcium salt or a magnesium salt; an ammonium salt; an aliphatic amine salt such as a trimethylamine salt, a triethylamine salt, a dicyclohexylamine salt, an ethanolamine salt, a diethanolamine salt, a triethanolamine salt or a procaine salt; an aralkylamine salt such as an N,N'-dibenzylethylenediamine salt; an aromatic heterocyclic amine salt such as a pyridine salt, a picoline salt, a quinoline salt or an isoquinoline salt; a quaternary ammonium salt such as a tetramethylammonium salt, a tetraethylammonium salt, a benzyltrimethylammonium salt, a benzyltriethylammonium salt, a benzyltributylammonium salt, a methyltrioctylammonium salt or a tetrabutylammonium salt; and a basic amino acid salt such as an arginine salt or a lysine salt.

The acid addition salt at the pyrrolidine base or at the base on the side chain substituted on the pyrrolidine ring includes, for example, an inorganic salt such as a hydrochloride, a sulfate, a nitrate, a phosphate, a carbonate, a hydrogencarbonate or a perchlorate; an organic salt such as an acetate, a propionate, a lactate, a maleate, a fumarate, a tartrate, a malate, a succinate or an ascorbate; a sulfonate such as a methanesulfonate, an isethionate, a benzenesulfonate or a p-toluenesulfonate; and an acidic amino acid salt such as an aspartate or a glutamate.

The non-toxic ester of the compound of the formula (I) means a common pharmaceutically acceptable ester at the carboxyl group at the 3-position of the carbapenem structure. For example, it includes an ester with an alkanoyloxymethyl group such as an acetoxymethyl group or a pivaloyloxymethyl group, an ester with an alkoxycarbonyloxyalkyl group such as a 1-(ethoxycarbonyloxy)ethyl group, an ester with a phthalidyl group and an ester with a (5-substituted-2-oxo-1,3-dioxol-4-yl)methyl group such as a (5-methyl-2-oxo-1,3-dioxol-4-yl)methyl group.

Specific examples of the compound of the formula (I) include the following compounds. In the following compounds, the abbreviations have the following meanings:

Ac: acetyl group
Et: ethyl group
iPr: isopropyl group
Me: methyl group
Pr: propyl group

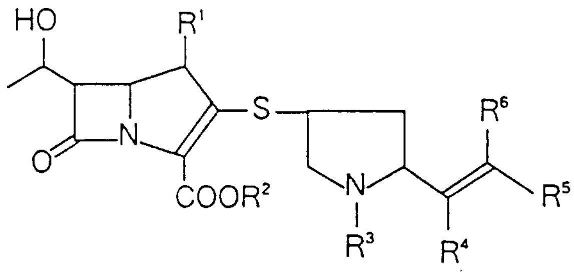

| No. | $R^1$ | $R^2$ | $R^3$ | $R^4$ | $R^5$ | $R^6$ |
|---|---|---|---|---|---|---|
| 1 | Me | H | H | $CH_2NH_2$ | H | H |
| 2 | Me | H | H | $CH_2NHMe$ | H | H |
| 3 | Me | H | H | $CH_2NHEt$ | H | H |
| 4 | Me | H | H | $CH_2NH$*i*Pr | H | H |
| 5 | Me | H | H | $CH_2NMe_2$ | H | H |
| 6 | Me | H | H | $CH_2NEt_2$ | H | H |
| 7 | Me | H | H | $CH_2N$ 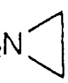 | H | H |
| 8 | Me | H | H | $CH_2N$ 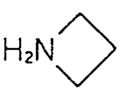 | H | H |
| 9 | Me | H | H | $CH_2N$ 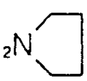 | H | H |
| 10 | Me | H | H | $CH_2N$ 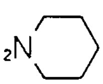 | H | H |
| 11 | Me | H | H | $CH_2N$ 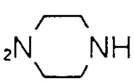 NH | H | H |
| 12 | Me | H | H | $CH_2N$ 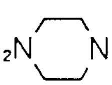 NMe | H | H |
| 13 | Me | H | H | $CH_2N$ 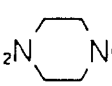 NCHO | H | H |

| No. | $R^1$ | $R^2$ | $R^3$ | $R^4$ | $R^5$ | $R^6$ |
|---|---|---|---|---|---|---|
| 14 | Me | H | H | 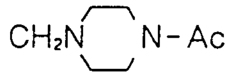 | H | H |
| 15 | Me | H | H | 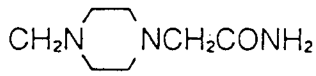 | H | H |
| 16 | Me | H | H | 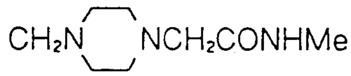 | H | H |
| 17 | Me | H | H | 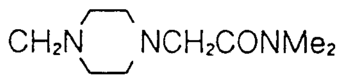 | H | H |
| 18 | Me | H | H | 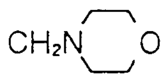 | H | H |
| 19 | Me | H | H | 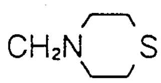 | H | H |
| 20 | Me | Negative charge | H | $CH_2\overset{+}{N}Me_3$ | H | H |
| 21 | Me | Negative charge | H | 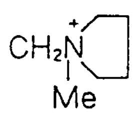 | H | H |
| 22 | Me | Negative charge | H | 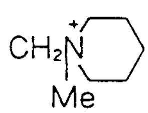 | H | H |
| 23 | Me | Negative charge | H | 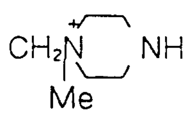 | H | H |
| 24 | Me | Negative charge | H | 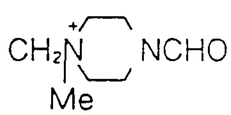 | H | H |
| 25 | Me | Negative charge | H | 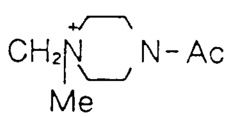 | H | H |

| No. | $R^1$ | $R^2$ | $R^3$ | $R^4$ | $R^5$ | $R^6$ |
|---|---|---|---|---|---|---|
| 26 | Me | Negative charge | H | 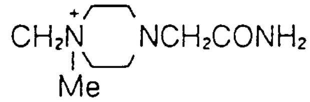 | H | H |
| 27 | Me | Negative charge | H | 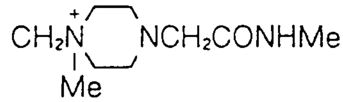 | H | H |
| 28 | Me | Negative charge | H | 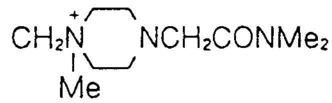 | H | H |
| 29 | Me | Negative charge | H | 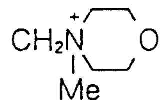 | H | H |
| 30 | Me | Negative charge | H | 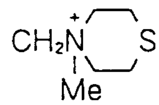 | H | H |
| 31 | Me | Negative charge | H | 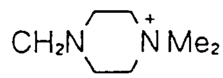 | H | H |
| 32 | Me | Negative charge | H | 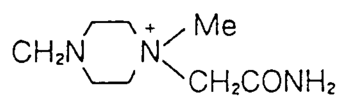 | H | H |
| 33 | Me | Negative charge | H | 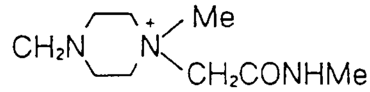 | H | H |
| 34 | Me | Negative charge | H | 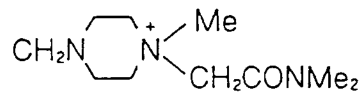 | H | H |
| 35 | Me | Negative charge | H | 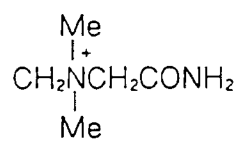 | H | H |
| 36 | Me | Negative charge | H | 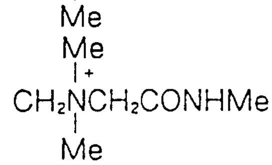 | H | H |
| 37 | Me | Negative charge | H | 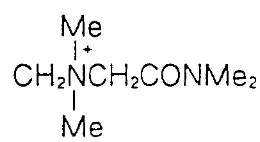 | H | H |

| No. | $R^1$ | $R^2$ | $R^3$ | $R^4$ | $R^5$ | $R^6$ |
|---|---|---|---|---|---|---|
| 38 | Me | Negative charge | H | 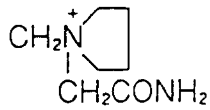 | H | H |
| 39 | Me | Negative charge | H | 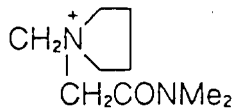 | H | H |
| 40 | Me | Negative charge | H | 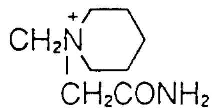 | H | H |
| 41 | Me | Negative charge | H | 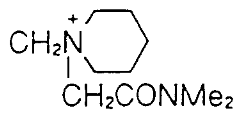 | H | H |
| 42 | Me | Negative charge | H | 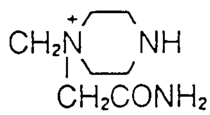 | H | H |
| 43 | Me | Negative charge | H | 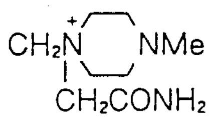 | H | H |
| 44 | Me | Negative charge | H | 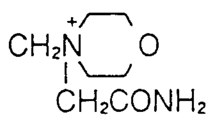 | H | H |
| 45 | Me | Negative charge | H | 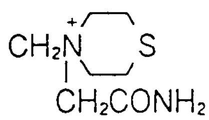 | H | H |

| No. | $R^1$ | $R^2$ | $R^3$ | $R^4$ | $R^5$ | $R^6$ |
|---|---|---|---|---|---|---|
| 46 | Me | H | H | H | $CH_2NH_2$ | H |
| 47 | Me | H | H | H | $CH_2NHMe$ | H |
| 48 | Me | H | H | H | $CH_2NHEt$ | H |
| 49 | Me | H | H | H | $CH_2NH\mathit{i}Pr$ | H |
| 50 | Me | H | H | H | $CH_2NMe_2$ | H |
| 51 | Me | H | H | H | $CH_2NEt_2$ | H |
| 52 | Me | H | H | H | 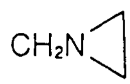 | H |
| 53 | Me | H | H | H | 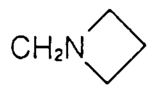 | H |
| 54 | Me | H | H | H | 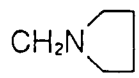 | H |
| 55 | Me | H | H | H | 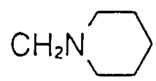 | H |
| 56 | Me | H | H | H | 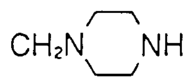 | H |
| 57 | Me | H | H | H | 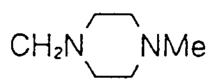 | H |
| 58 | Me | H | H | H | 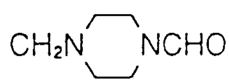 | H |
| 59 | Me | H | H | H | 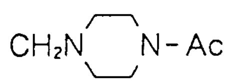 | H |
| 60 | Me | H | H | H | 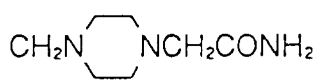 | H |
| 61 | Me | H | H | H | 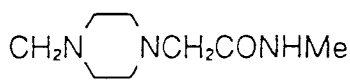 | H |

| No. | $R^1$ | $R^2$ | $R^3$ | $R^4$ | $R^5$ | $R^6$ |
|---|---|---|---|---|---|---|
| 62 | Me | H | H | H | 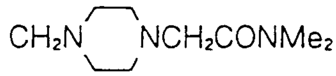 | H |
| 63 | Me | H | H | H | 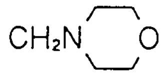 | H |
| 64 | Me | H | H | H | 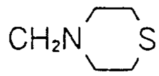 | H |
| 65 | Me | Negative charge | H | H | $CH_2\overset{+}{N}Me_3$ | H |
| 66 | Me | Negative charge | H | H | 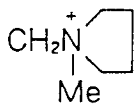 | H |
| 67 | Me | Negative charge | H | H | 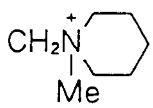 | H |
| 68 | Me | Negative charge | H | H | 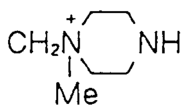 | H |
| 69 | Me | Negative charge | H | H | 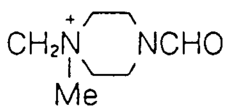 | H |
| 70 | Me | Negative charge | H | H | 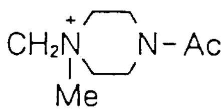 | H |
| 71 | Me | Negative charge | H | H | 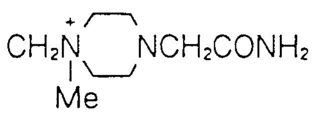 | H |
| 72 | Me | Negative charge | H | H | 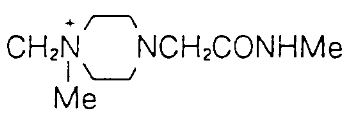 | H |
| 73 | Me | Negative charge | H | H | 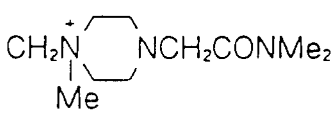 | H |

| No. | $R^1$ | $R^2$ | $R^3$ | $R^4$ | $R^5$ | $R^6$ |
|---|---|---|---|---|---|---|
| 74 | Me | Negative charge | H | H | 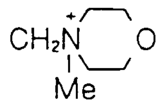 | H |
| 75 | Me | Negative charge | H | H | 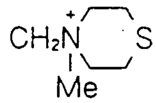 | H |
| 76 | Me | Negative charge | H | H | 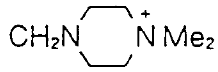 | H |
| 77 | Me | Negative charge | H | H | 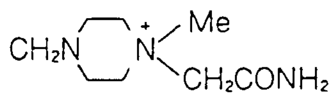 | H |
| 78 | Me | Negative charge | H | H | 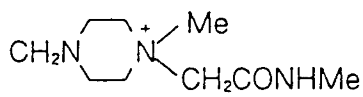 | H |
| 79 | Me | Negative charge | H | H | 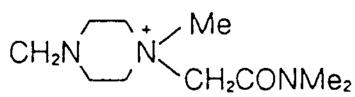 | H |
| 80 | Me | Negative charge | H | H | 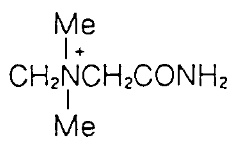 | H |
| 81 | Me | Negative charge | H | H | 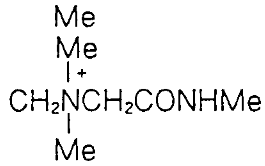 | H |
| 82 | Me | Negative charge | H | H | 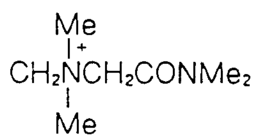 | H |
| 83 | Me | Negative charge | H | H | 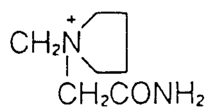 | H |
| 84 | Me | Negative charge | H | H | 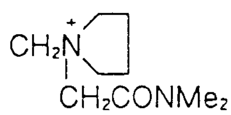 | H |
| 85 | Me | Negative charge | H | H | 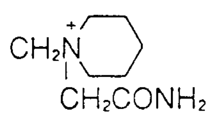 | H |

| No. | $R^1$ | $R^2$ | $R^3$ | $R^4$ | $R^5$ | $R^6$ |
|---|---|---|---|---|---|---|
| 86 | Me | Negative charge | H | H | 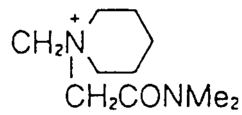 | H |
| 87 | Me | Negative charge | H | H | 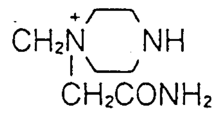 | H |
| 88 | Me | Negative charge | H | H | 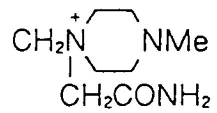 | H |
| 89 | Me | Negative charge | H | H | 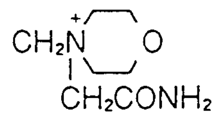 | H |
| 90 | Me | Negative charge | H | H | 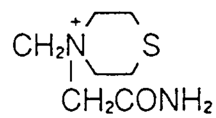 | H |

| No. | $R^1$ | $R^2$ | $R^3$ | $R^4$ | $R^5$ | $R^6$ |
|---|---|---|---|---|---|---|
| 91 | Me | H | H | H | H | $CH_2NH_2$ |
| 92 | Me | H | H | H | H | $CH_2NHMe$ |
| 93 | Me | H | H | H | H | $CH_2NHEt$ |
| 94 | Me | H | H | H | H | $CH_2NH\mathit{i}Pr$ |
| 95 | Me | H | H | H | H | $CH_2NMe_2$ |
| 96 | Me | H | H | H | H | $CH_2NEt_2$ |
| 97 | Me | H | H | H | H | 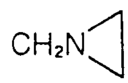 |
| 98 | Me | H | H | H | H | 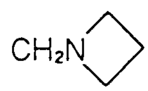 |
| 99 | Me | H | H | H | H | 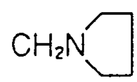 |
| 100 | Me | H | H | H | H | 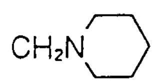 |
| 101 | Me | H | H | H | H | 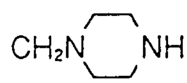 |
| 102 | Me | H | H | H | H | 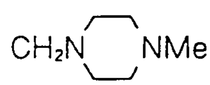 |
| 103 | Me | H | H | H | H | 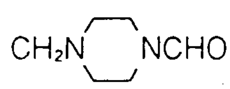 |
| 104 | Me | H | H | H | H | 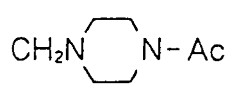 |
| 105 | Me | H | H | H | H | 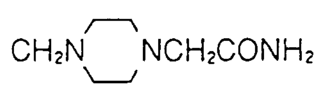 |
| 106 | Me | H | H | H | H | 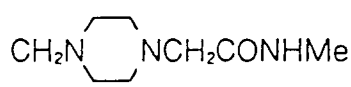 |

| No. | $R^1$ | $R^2$ | $R^3$ | $R^4$ | $R^5$ | $R^6$ |
|---|---|---|---|---|---|---|
| 107 | Me | H | H | H | H | 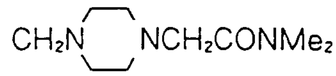 |
| 108 | Me | H | H | H | H | 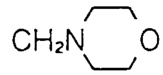 |
| 109 | Me | H | H | H | H | 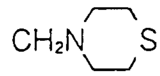 |
| 110 | Me | Negative charge | H | H | H | $CH_2\overset{+}{N}Me_3$ |
| 111 | Me | Negative charge | H | H | H | 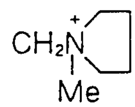 |
| 112 | Me | Negative charge | H | H | H | 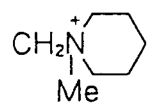 |
| 113 | Me | Negative charge | H | H | H | 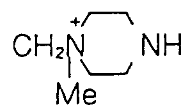 |
| 114 | Me | Negative charge | H | H | H | 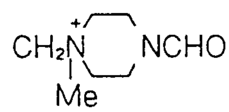 |
| 115 | Me | Negative charge | H | H | H | 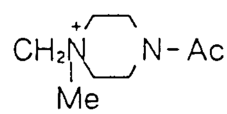 |
| 116 | Me | Negative charge | H | H | H | 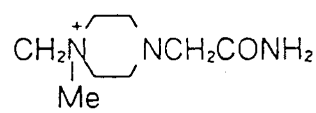 |
| 117 | Me | Negative charge | H | H | H | 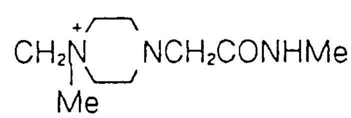 |
| 118 | Me | Negative charge | H | H | H | 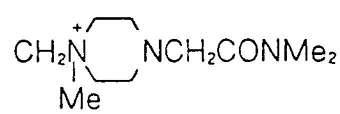 |

| No. | $R^1$ | $R^2$ | $R^3$ | $R^4$ | $R^5$ | $R^6$ |
|---|---|---|---|---|---|---|
| 119 | Me | Negative charge | H | H | H | 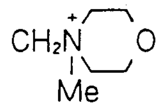 |
| 120 | Me | Negative charge | H | H | H | 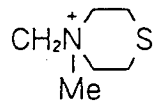 |
| 121 | Me | Negative charge | H | H | H | 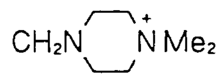 |
| 122 | Me | Negative charge | H | H | H | 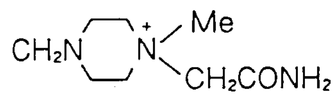 |
| 123 | Me | Negative charge | H | H | H | 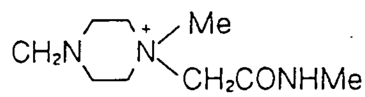 |
| 124 | Me | Negative charge | H | H | H | 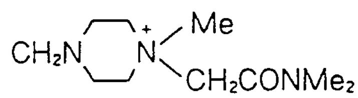 |
| 125 | Me | Negative charge | H | H | H | 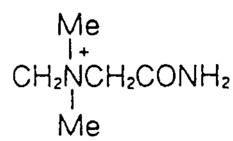 |
| 126 | Me | Negative charge | H | H | H | 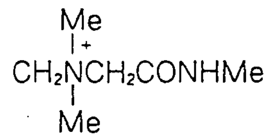 |
| 127 | Me | Negative charge | H | H | H | 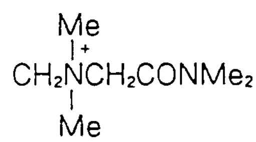 |
| 128 | Me | Negative charge | H | H | H | 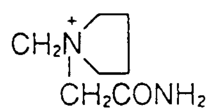 |
| 129 | Me | Negative charge | H | H | H | 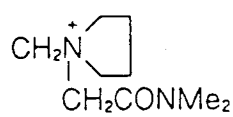 |
| 130 | Me | Negative charge | H | H | H | 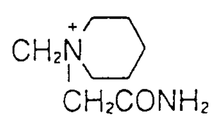 |

| No. | $R^1$ | $R^2$ | $R^3$ | $R^4$ | $R^5$ | $R^6$ |
|---|---|---|---|---|---|---|
| 131 | Me | Negative charge | H | H | H | 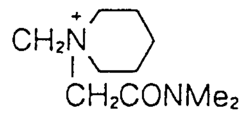 |
| 132 | Me | Negative charge | H | H | H | 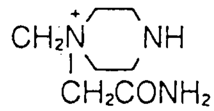 |
| 133 | Me | Negative charge | H | H | H | 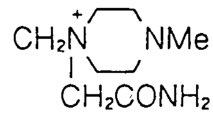 |
| 134 | Me | Negative charge | H | H | H | 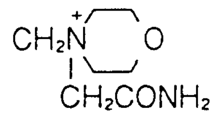 |
| 135 | Me | Negative charge | H | H | H | 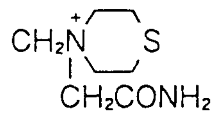 |

| No. | $R^1$ | $R^2$ | $R^3$ | $R^4$ | $R^5$ | $R^6$ |
|---|---|---|---|---|---|---|
| 136 | Me | H | H | $CH_2NH_2$ | $CONH_2$ | H |
| 137 | Me | H | H | $CH_2NHMe$ | $CONH_2$ | H |
| 138 | Me | H | H | $CH_2NMe_2$ | $CONH_2$ | H |
| 139 | Me | H | H | 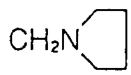 | $CONH_2$ | H |
| 140 | Me | H | H | 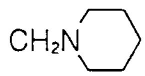 | $CONH_2$ | H |
| 141 | Me | Negative charge | H | $CH_2\overset{+}{N}Me_3$ | $CONH_2$ | H |
| 142 | Me | Negative charge | H | 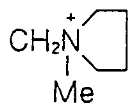 | $CONH_2$ | H |
| 143 | Me | Negative charge | H | 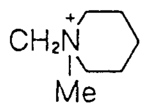 | $CONH_2$ | H |
| 144 | Me | H | H | $CH_2NH_2$ | H | $CONH_2$ |
| 145 | Me | H | H | $CH_2NHMe$ | H | $CONH_2$ |
| 146 | Me | H | H | $CH_2NMe_2$ | H | $CONH_2$ |
| 147 | Me | H | H | 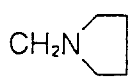 | H | $CONH_2$ |
| 148 | Me | H | H | 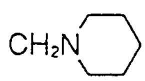 | H | $CONH_2$ |
| 149 | Me | Negative charge | H | $CH_2\overset{+}{N}Me_3$ | H | $CONH_2$ |
| 150 | Me | Negative charge | H | 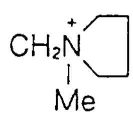 | H | $CONH_2$ |
| 151 | Me | Negative charge | H | 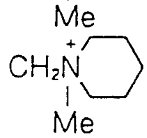 | H | $CONH_2$ |

| No. | $R^1$ | $R^2$ | $R^3$ | $R^4$ | $R^5$ | $R^6$ |
|---|---|---|---|---|---|---|
| 152 | Me | H | H | $CH_2NH_2$ | Me | H |
| 153 | Me | H | H | $CH_2NHMe$ | Me | H |
| 154 | Me | H | H | $CH_2NMe_2$ | Me | H |
| 155 | Me | H | H | 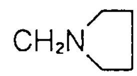 | Me | H |
| 156 | Me | H | H | 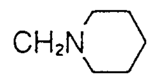 | Me | H |
| 157 | Me | Negative charge | H | $CH_2\overset{+}{N}Me_3$ | Me | H |
| 158 | Me | Negative charge | H | 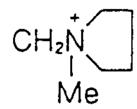 | Me | H |
| 159 | Me | Negative charge | H | 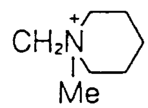 | Me | H |
| 160 | Me | H | H | $CH_2NH_2$ | H | Me |
| 161 | Me | H | H | $CH_2NHMe$ | H | Me |
| 162 | Me | H | H | $CH_2NMe_2$ | H | Me |
| 163 | Me | H | H | 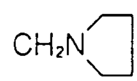 | H | Me |
| 164 | Me | H | H | 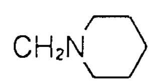 | H | Me |
| 165 | Me | Negative charge | H | $CH_2\overset{+}{N}Me_3$ | H | Me |
| 166 | Me | Negative charge | H | 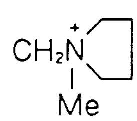 | H | Me |
| 167 | Me | Negative charge | H | 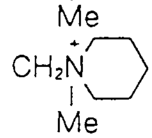 | H | Me |

| No. | $R^1$ | $R^2$ | $R^3$ | $R^4$ | $R^5$ | $R^6$ |
|---|---|---|---|---|---|---|
| 168 | Me | H | H | $CH_2NH_2$ | $CF_3$ | H |
| 169 | Me | H | H | $CH_2NHMe$ | $CF_3$ | H |
| 170 | Me | H | H | $CH_2NMe_2$ | $CF_3$ | H |
| 171 | Me | H | H | 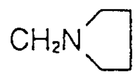 | $CF_3$ | H |
| 172 | Me | H | H | 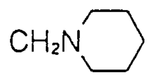 | $CF_3$ | H |
| 173 | Me | Negative charge | H | $CH_2\overset{+}{N}Me_3$ | $CF_3$ | H |
| 174 | Me | Negative charge | H | 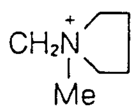 | $CF_3$ | H |
| 175 | Me | Negative charge | H | 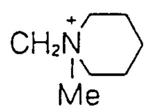 | $CF_3$ | H |
| 176 | Me | H | H | $CH_2NH_2$ | CN | H |
| 177 | Me | H | H | $CH_2NHMe$ | CN | H |
| 178 | Me | H | H | $CH_2NMe_2$ | CN | H |
| 179 | Me | H | H | 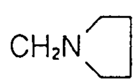 | CN | H |
| 180 | Me | H | H | 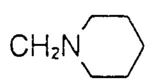 | CN | H |
| 181 | Me | Negative charge | H | $CH_2\overset{+}{N}Me_3$ | CN | H |
| 182 | Me | Negative charge | H | 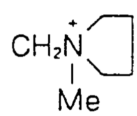 | CN | H |
| 183 | Me | Negative charge | H | 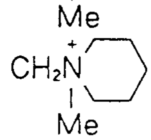 | CN | H |

| No. | $R^1$ | $R^2$ | $R^3$ | $R^4$ | $R^5$ | $R^6$ |
|---|---|---|---|---|---|---|
| 184 | Me | H | H | $CH_2NH_2$ | H | CN |
| 185 | Me | H | H | $CH_2NHMe$ | H | CN |
| 186 | Me | H | H | $CH_2NMe_2$ | H | CN |
| 187 | Me | H | H | 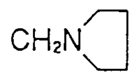 | H | CN |
| 188 | Me | H | H | 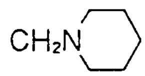 | H | CN |
| 189 | Me | Negative charge | H | $CH_2\overset{+}{N}Me_3$ | H | CN |
| 190 | Me | Negative charge | H | 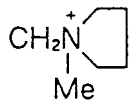 | H | CN |
| 191 | Me | Negative charge | H | 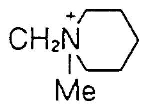 | H | CN |
| 192 | Me | H | H | $CH_2NH_2$ | $SO_2Me$ | H |
| 193 | Me | H | H | $CH_2NHMe$ | $SO_2Me$ | H |
| 194 | Me | H | H | $CH_2NMe_2$ | $SO_2Me$ | H |
| 195 | Me | H | H | 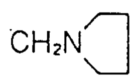 | $SO_2Me$ | H |
| 196 | Me | H | H | 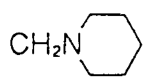 | $SO_2Me$ | H |
| 197 | Me | Negative charge | H | $CH_2\overset{+}{N}Me_3$ | $SO_2Me$ | H |
| 198 | Me | Negative charge | H | 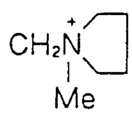 | $SO_2Me$ | H |
| 199 | Me | Negative charge | H | 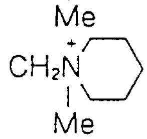 | $SO_2Me$ | H |

| No. | $R^1$ | $R^2$ | $R^3$ | $R^4$ | $R^5$ | $R^6$ |
|---|---|---|---|---|---|---|
| 200 | Me | H | H | $CH_2NH_2$ | H | $SO_2Me$ |
| 201 | Me | H | H | $CH_2NHMe$ | H | $SO_2Me$ |
| 202 | Me | H | H | $CH_2NMe_2$ | H | $SO_2Me$ |
| 203 | Me | H | H | 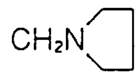 | H | $SO_2Me$ |
| 204 | Me | H | H | 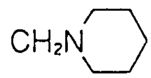 | H | $SO_2Me$ |
| 205 | Me | Negative charge | H | $CH_2\overset{+}{N}Me_3$ | H | $SO_2Me$ |
| 206 | Me | Negative charge | H | 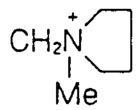 | H | $SO_2Me$ |
| 207 | Me | Negative charge | H | 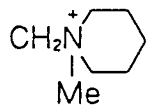 | H | $SO_2Me$ |

| No. | $R^1$ | $R^2$ | $R^3$ | $R^4$ | $R^5$ | $R^6$ |
|---|---|---|---|---|---|---|
| 208 | Me | H | H | H | $CH_2NH_2$ | $CONH_2$ |
| 209 | Me | H | H | H | $CH_2NHMe$ | $CONH_2$ |
| 210 | Me | H | H | H | $CH_2NMe_2$ | $CONH_2$ |
| 211 | Me | H | H | H | 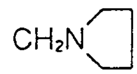 | $CONH_2$ |
| 212 | Me | H | H | H | 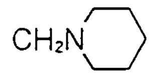 | $CONH_2$ |
| 213 | Me | Negative charge | H | H | $CH_2\overset{+}{N}Me_3$ | $CONH_2$ |
| 214 | Me | Negative charge | H | H | 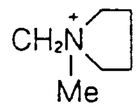 | $CONH_2$ |
| 215 | Me | Negative charge | H | H | 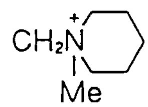 | $CONH_2$ |
| 216 | Me | H | H | $CONH_2$ | $CH_2NH_2$ | H |
| 217 | Me | H | H | $CONH_2$ | $CH_2NHMe$ | H |
| 218 | Me | H | H | $CONH_2$ | $CH_2NMe_2$ | H |
| 219 | Me | H | H | $CONH_2$ | 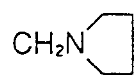 | H |
| 220 | Me | H | H | $CONH_2$ | 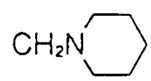 | H |
| 221 | Me | Negative charge | H | $CONH_2$ | $CH_2\overset{+}{N}Me_3$ | H |
| 222 | Me | Negative charge | H | $CONH_2$ | 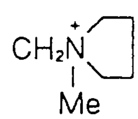 | H |
| 223 | Me | Negative charge | H | $CONH_2$ | 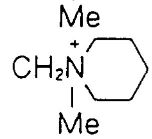 | H |

| No. | $R^1$ | $R^2$ | $R^3$ | $R^4$ | $R^5$ | $R^6$ |
|---|---|---|---|---|---|---|
| 224 | Me | H | H | H | $CH_2NH_2$ | Me |
| 225 | Me | H | H | H | $CH_2NHMe$ | Me |
| 226 | Me | H | H | H | $CH_2NMe_2$ | Me |
| 227 | Me | H | H | H | 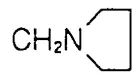 | Me |
| 228 | Me | H | H | H | 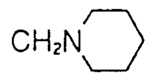 | Me |
| 229 | Me | Negative charge | H | H | $CH_2\overset{+}{N}Me_3$ | Me |
| 230 | Me | Negative charge | H | H | 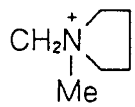 | Me |
| 231 | Me | Negative charge | H | H | 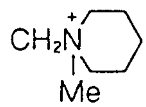 | Me |
| 232 | Me | H | H | Me | $CH_2NH_2$ | H |
| 233 | Me | H | H | Me | $CH_2NHMe$ | H |
| 234 | Me | H | H | Me | $CH_2NMe_2$ | H |
| 235 | Me | H | H | Me | 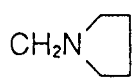 | H |
| 236 | Me | H | H | Me | 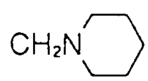 | H |
| 237 | Me | Negative charge | H | Me | $CH_2\overset{+}{N}Me_3$ | H |
| 238 | Me | Negative charge | H | Me | 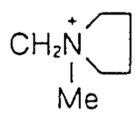 | H |
| 239 | Me | Negative charge | H | Me | 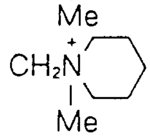 | H |

| No. | $R^1$ | $R^2$ | $R^3$ | $R^4$ | $R^5$ | $R^6$ |
|---|---|---|---|---|---|---|
| 240 | Me | H | H | H | $CH_2NH_2$ | $CF_3$ |
| 241 | Me | H | H | H | $CH_2NHMe$ | $CF_3$ |
| 242 | Me | H | H | H | $CH_2NMe_2$ | $CF_3$ |
| 243 | Me | H | H | H | 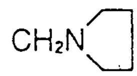 | $CF_3$ |
| 244 | Me | H | H | H | 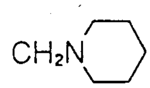 | $CF_3$ |
| 245 | Me | Negative charge | H | H | $CH_2\overset{+}{N}Me_3$ | $CF_3$ |
| 246 | Me | Negative charge | H | H | 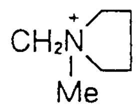 | $CF_3$ |
| 247 | Me | Negative charge | H | H | 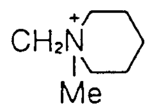 | $CF_3$ |
| 248 | Me | H | H | H | $CH_2NH_2$ | CN |
| 249 | Me | H | H | H | $CH_2NHMe$ | CN |
| 250 | Me | H | H | H | $CH_2NMe_2$ | CN |
| 251 | Me | H | H | H | 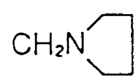 | CN |
| 252 | Me | H | H | H | 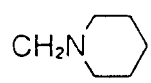 | CN |
| 253 | Me | Negative charge | H | H | $CH_2\overset{+}{N}Me_3$ | CN |
| 254 | Me | Negative charge | H | H | 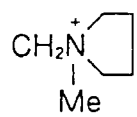 | CN |
| 255 | Me | Negative charge | H | H | 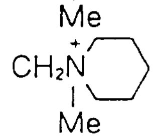 | CN |

| No. | $R^1$ | $R^2$ | $R^3$ | $R^4$ | $R^5$ | $R^6$ |
|---|---|---|---|---|---|---|
| 256 | Me | H | H | CN | $CH_2NH_2$ | H |
| 257 | Me | H | H | CN | $CH_2NHMe$ | H |
| 258 | Me | H | H | CN | $CH_2NMe_2$ | H |
| 259 | Me | H | H | CN | 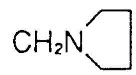 | H |
| 260 | Me | H | H | CN | 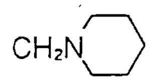 | H |
| 261 | Me | Negative charge | H | CN | $CH_2\overset{+}{N}Me_3$ | H |
| 262 | Me | Negative charge | H | CN | 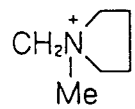 | H |
| 263 | Me | Negative charge | H | CN | 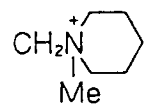 | H |
| 264 | Me | H | H | H | $CH_2NH_2$ | $SO_2Me$ |
| 265 | Me | H | H | H | $CH_2NHMe$ | $SO_2Me$ |
| 266 | Me | H | H | H | $CH_2NMe_2$ | $SO_2Me$ |
| 267 | Me | H | H | H | 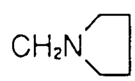 | $SO_2Me$ |
| 268 | Me | H | H | H | 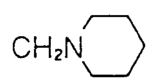 | $SO_2Me$ |
| 269 | Me | Negative charge | H | H | $CH_2\overset{+}{N}Me_3$ | $SO_2Me$ |
| 270 | Me | Negative charge | H | H | 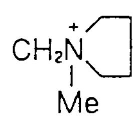 | $SO_2Me$ |
| 271 | Me | Negative charge | H | H | 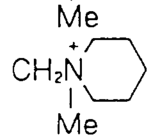 | $SO_2Me$ |

| No. | $R^1$ | $R^2$ | $R^3$ | $R^4$ | $R^5$ | $R^6$ |
|---|---|---|---|---|---|---|
| 272 | Me | H | H | $SO_2Me$ | $CH_2NH_2$ | H |
| 273 | Me | H | H | $SO_2Me$ | $CH_2NHMe$ | H |
| 274 | Me | H | H | $SO_2Me$ | $CH_2NMe_2$ | H |
| 275 | Me | H | H | $SO_2Me$ | 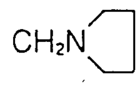 | H |
| 276 | Me | H | H | $SO_2Me$ | 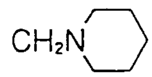 | H |
| 277 | Me | Negative charge | H | $SO_2Me$ | $CH_2\overset{+}{N}Me_3$ | H |
| 278 | Me | Negative charge | H | $SO_2Me$ | 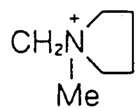 | H |
| 279 | Me | Negative charge | H | $SO_2Me$ | 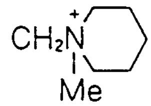 | H |

| No. | $R^1$ | $R^2$ | $R^3$ | $R^4$ | $R^5$ | $R^6$ |
|---|---|---|---|---|---|---|
| 280 | Me | H | H | $CONH_2$ | H | $CH_2NH_2$ |
| 281 | Me | H | H | $CONH_2$ | H | $CH_2NHMe$ |
| 282 | Me | H | H | $CONH_2$ | H | $CH_2NMe_2$ |
| 283 | Me | H | H | $CONH_2$ | H | 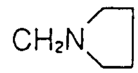 |
| 284 | Me | H | H | $CONH_2$ | H | 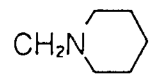 |
| 285 | Me | Negative charge | H | $CONH_2$ | H | $CH_2\overset{+}{N}Me_3$ |
| 286 | Me | Negative charge | H | $CONH_2$ | H | 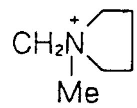 |
| 287 | Me | Negative charge | H | $CONH_2$ | H | 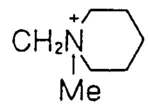 |
| 288 | Me | H | H | H | $CONH_2$ | $CH_2NH_2$ |
| 289 | Me | H | H | H | $CONH_2$ | $CH_2NHMe$ |
| 290 | Me | H | H | H | $CONH_2$ | $CH_2NMe_2$ |
| 291 | Me | H | H | H | $CONH_2$ | 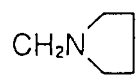 |
| 292 | Me | H | H | H | $CONH_2$ | 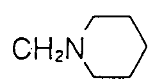 |
| 293 | Me | Negative charge | H | H | $CONH_2$ | $CH_2\overset{+}{N}Me_3$ |
| 294 | Me | Negative charge | H | H | $CONH_2$ | 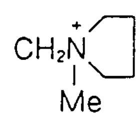 |
| 295 | Me | Negative charge | H | H | $CONH_2$ | 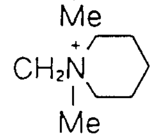 |

| No. | $R^1$ | $R^2$ | $R^3$ | $R^4$ | $R^5$ | $R^6$ |
|---|---|---|---|---|---|---|
| 296 | Me | H | H | Me | H | $CH_2NH_2$ |
| 297 | Me | H | H | Me | H | $CH_2NHMe$ |
| 298 | Me | H | H | Me | H | $CH_2NMe_2$ |
| 299 | Me | H | H | Me | H | 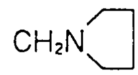 |
| 300 | Me | H | H | Me | H | 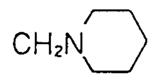 |
| 301 | Me | Negative charge | H | Me | H | $CH_2\overset{+}{N}Me_3$ |
| 302 | Me | Negative charge | H | Me | H | 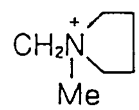 |
| 303 | Me | Negative charge | H | Me | H | 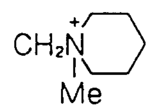 |
| 304 | Me | H | H | H | Me | $CH_2NH_2$ |
| 305 | Me | H | H | H | Me | $CH_2NHMe$ |
| 306 | Me | H | H | H | Me | $CH_2NMe_2$ |
| 307 | Me | H | H | H | Me | 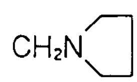 |
| 308 | Me | H | H | H | Me | 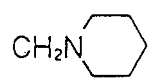 |
| 309 | Me | Negative charge | H | H | Me | $CH_2\overset{+}{N}Me_3$ |
| 310 | Me | Negative charge | H | H | Me | 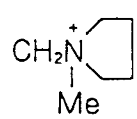 |
| 311 | Me | Negative charge | H | H | Me | 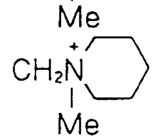 |

| No. | $R^1$ | $R^2$ | $R^3$ | $R^4$ | $R^5$ | $R^6$ |
|---|---|---|---|---|---|---|
| 312 | Me | H | H | $CF_3$ | H | $CH_2NH_2$ |
| 313 | Me | H | H | $CF_3$ | H | $CH_2NHMe$ |
| 314 | Me | H | H | $CF_3$ | H | $CH_2NMe_2$ |
| 315 | Me | H | H | $CF_3$ | H | 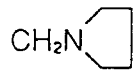 |
| 316 | Me | H | H | $CF_3$ | H | 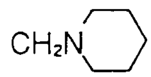 |
| 317 | Me | Negative charge | H | $CF_3$ | H | $CH_2\overset{+}{N}Me_3$ |
| 318 | Me | Negative charge | H | $CF_3$ | H | 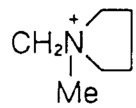 |
| 319 | Me | Negative charge | H | $CF_3$ | H | 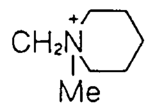 |
| 320 | Me | H | H | CN | H | $CH_2NH_2$ |
| 321 | Me | H | H | CN | H | $CH_2NHMe$ |
| 322 | Me | H | H | CN | H | $CH_2NMe_2$ |
| 323 | Me | H | H | CN | H | 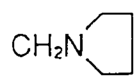 |
| 324 | Me | H | H | CN | H | 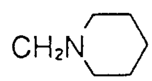 |
| 325 | Me | Negative charge | H | CN | H | $CH_2\overset{+}{N}Me_3$ |
| 326 | Me | Negative charge | H | CN | H | 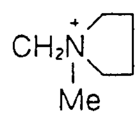 |
| 327 | Me | Negative charge | H | CN | H | 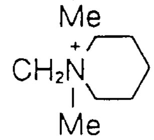 |

| No. | $R^1$ | $R^2$ | $R^3$ | $R^4$ | $R^5$ | $R^6$ |
|---|---|---|---|---|---|---|
| 328 | Me | H | H | H | CN | $CH_2NH_2$ |
| 329 | Me | H | H | H | CN | $CH_2NHMe$ |
| 330 | Me | H | H | H | CN | $CH_2NMe_2$ |
| 331 | Me | H | H | H | CN | 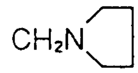 |
| 332 | Me | H | H | H | CN | 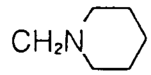 |
| 333 | Me | Negative charge | H | H | CN | $CH_2\overset{+}{N}Me_3$ |
| 334 | Me | Negative charge | H | H | CN | 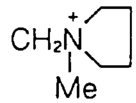 |
| 335 | Me | Negative charge | H | H | CN | 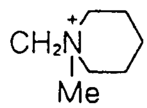 |
| 336 | Me | H | H | $SO_2Me$ | H | $CH_2NH_2$ |
| 337 | Me | H | H | $SO_2Me$ | H | $CH_2NHMe$ |
| 338 | Me | H | H | $SO_2Me$ | H | $CH_2NMe_2$ |
| 339 | Me | H | H | $SO_2Me$ | H | 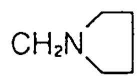 |
| 340 | Me | H | H | $SO_2Me$ | H | 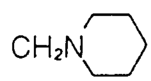 |
| 341 | Me | Negative charge | H | $SO_2Me$ | H | $CH_2\overset{+}{N}Me_3$ |
| 342 | Me | Negative charge | H | $SO_2Me$ | H | 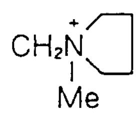 |
| 343 | Me | Negative charge | H | $SO_2Me$ | H | 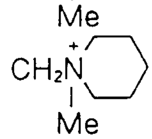 |

| No. | $R^1$ | $R^2$ | $R^3$ | $R^4$ | $R^5$ | $R^6$ |
|---|---|---|---|---|---|---|
| 344 | Me | H | H | H | $SO_2Me$ | $CH_2NH_2$ |
| 345 | Me | H | H | H | $SO_2Me$ | $CH_2NHMe$ |
| 346 | Me | H | H | H | $SO_2Me$ | $CH_2NMe_2$ |
| 347 | Me | H | H | H | $SO_2Me$ | 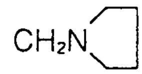 |
| 348 | Me | H | H | H | $SO_2Me$ | 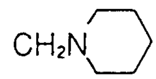 |
| 349 | Me | Negative charge | H | H | $SO_2Me$ | $CH_2\overset{+}{N}Me_3$ |
| 350 | Me | Negative charge | H | H | $SO_2Me$ | 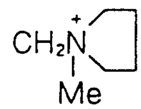 |
| 351 | Me | Negative charge | H | H | $SO_2Me$ | 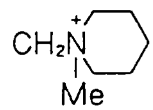 |
| 352 | H | H | H | H | $CH_2NH_2$ | H |
| 353 | H | H | H | H | H | $CH_2NH_2$ |

| No. | $R^1$ | $R^2$ | $R^3$ | $R^4$ | $R^5$ | $R^6$ |
|---|---|---|---|---|---|---|
| 354 | Me | H | H | $CH_2NH_2$ | Et | H |
| 355 | Me | H | H | $CH_2NHMe$ | Et | H |
| 356 | Me | Negative charge | H | $CH_2\overset{+}{N}Me_3$ | Et | H |
| 357 | Me | Negative charge | H | 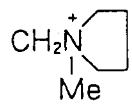 | Et | H |
| 358 | Me | H | H | $CH_2NH_2$ | Pr | H |
| 359 | Me | H | H | $CH_2NHMe$ | Pr | H |
| 360 | Me | Negative charge | H | $CH_2\overset{+}{N}Me_3$ | Pr | H |
| 361 | Me | Negative charge | H | 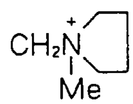 | Pr | H |
| 362 | Me | H | H | $CH_2NH_2$ | iPr | H |
| 363 | Me | H | H | $CH_2NHMe$ | iPr | H |
| 364 | Me | Negative charge | H | $CH_2\overset{+}{N}Me_3$ | iPr | H |
| 365 | Me | Negative charge | H | 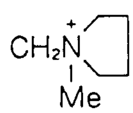 | iPr | H |
| 366 | Me | H | H | $CH_2NH_2$ | H | Et |
| 367 | Me | H | H | $CH_2NHMe$ | H | Et |
| 368 | Me | Negative charge | H | $CH_2\overset{+}{N}Me_3$ | H | Et |
| 369 | Me | Negative charge | H | 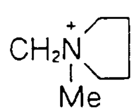 | H | Et |

| No. | $R^1$ | $R^2$ | $R^3$ | $R^4$ | $R^5$ | $R^6$ |
|---|---|---|---|---|---|---|
| 370 | Me | H | H | $CH_2NH_2$ | H | Pr |
| 371 | Me | H | H | $CH_2NHMe$ | H | Pr |
| 372 | Me | Negative charge | H | $CH_2\overset{+}{N}Me_3$ | H | Pr |
| 373 | Me | Negative charge | H | 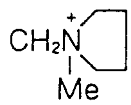 | H | Pr |
| 374 | Me | H | H | $CH_2NH_2$ | H | iPr |
| 375 | Me | H | H | $CH_2NHMe$ | H | iPr |
| 376 | Me | Negative charge | H | $CH_2\overset{+}{N}Me_3$ | H | iPr |
| 377 | Me | Negative charge | H | 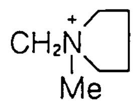 | H | iPr |

| No. | $R^1$ | $R^2$ | $R^3$ | $R^4$ | $R^5$ | $R^6$ |
|---|---|---|---|---|---|---|
| 378 | Me | H | H | H | $CH_2NH_2$ | Et |
| 379 | Me | H | H | H | $CH_2NHMe$ | Et |
| 380 | Me | Negative charge | H | H | $CH_2\overset{+}{N}Me_3$ | Et |
| 381 | Me | Negative charge | H | H | 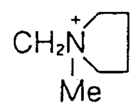 | Et |
| 382 | Me | H | H | H | $CH_2NH_2$ | Pr |
| 383 | Me | H | H | H | $CH_2NHMe$ | Pr |
| 384 | Me | Negative charge | H | H | $CH_2\overset{+}{N}Me_3$ | Pr |
| 385 | Me | Negative charge | H | H | 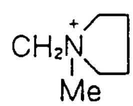 | Pr |
| 386 | Me | H | H | H | $CH_2NH_2$ | iPr |
| 387 | Me | H | H | H | $CH_2NHMe$ | iPr |
| 388 | Me | Negative charge | H | H | $CH_2\overset{+}{N}Me_3$ | iPr |
| 389 | Me | Negative charge | H | H | 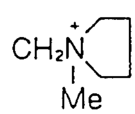 | iPr |
| 390 | Me | H | H | Et | $CH_2NH_2$ | H |
| 391 | Me | H | H | Et | $CH_2NHMe$ | H |
| 392 | Me | Negative charge | H | Et | $CH_2\overset{+}{N}Me_3$ | H |
| 393 | Me | Negative charge | H | Et | 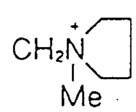 | H |

| No. | $R^1$ | $R^2$ | $R^3$ | $R^4$ | $R^5$ | $R^6$ |
|---|---|---|---|---|---|---|
| 394 | Me | H | H | Pr | $CH_2NH_2$ | H |
| 395 | Me | H | H | Pr | $CH_2NHMe$ | H |
| 396 | Me | Negative charge | H | Pr | $CH_2\overset{+}{N}Me_3$ | H |
| 397 | Me | Negative charge | H | Pr | 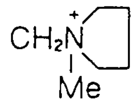 | H |
| 398 | Me | H | H | iPr | $CH_2NH_2$ | H |
| 399 | Me | H | H | iPr | $CH_2NHMe$ | H |
| 400 | Me | Negative charge | H | iPr | $CH_2\overset{+}{N}Me_3$ | H |
| 401 | Me | Negative charge | H | iPr | 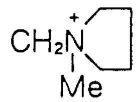 | H |

| No. | $R^1$ | $R^2$ | $R^3$ | $R^4$ | $R^5$ | $R^6$ |
|---|---|---|---|---|---|---|
| 402 | Me | H | H | Et | H | $CH_2NH_2$ |
| 403 | Me | H | H | Et | H | $CH_2NHMe$ |
| 404 | Me | Negative charge | H | Et | H | $CH_2\overset{+}{N}Me_3$ |
| 405 | Me | Negative charge | H | Et | H | 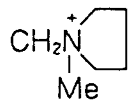 |
| 406 | Me | H | H | Pr | H | $CH_2NH_2$ |
| 407 | Me | H | H | Pr | H | $CH_2NHMe$ |
| 408 | Me | Negative charge | H | Pr | H | $CH_2\overset{+}{N}Me_3$ |
| 409 | Me | Negative charge | H | Pr | H | 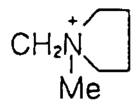 |
| 410 | Me | H | H | iPr | H | $CH_2NH_2$ |
| 411 | Me | H | H | iPr | H | $CH_2NHMe$ |
| 412 | Me | Negative charge | H | iPr | H | $CH_2\overset{+}{N}Me_3$ |
| 413 | Me | Negative charge | H | iPr | H | 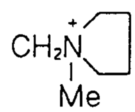 |
| 414 | Me | H | H | H | Et | $CH_2NH_2$ |
| 415 | Me | H | H | H | Et | $CH_2NHMe$ |
| 416 | Me | Negative charge | H | H | Et | $CH_2\overset{+}{N}Me_3$ |
| 417 | Me | Negative charge | H | H | Et | 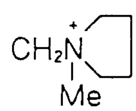 |

| No. | $R^1$ | $R^2$ | $R^3$ | $R^4$ | $R^5$ | $R^6$ |
|---|---|---|---|---|---|---|
| 418 | Me | H | H | H | Pr | $CH_2NH_2$ |
| 419 | Me | H | H | H | Pr | $CH_2NHMe$ |
| 420 | Me | Negative charge | H | H | Pr | $CH_2\overset{+}{N}Me_3$ |
| 421 | Me | Negative charge | H | H | Pr | 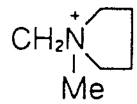 |
| 422 | Me | H | H | H | iPr | $CH_2NH_2$ |
| 423 | Me | H | H | H | iPr | $CH_2NHMe$ |
| 424 | Me | Negative charge | H | H | iPr | $CH_2\overset{+}{N}Me_3$ |
| 425 | Me | Negative charge | H | H | iPr | 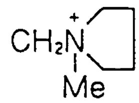 |

| No. | $R^1$ | $R^2$ | $R^3$ | $R^4$ | $R^5$ | $R^6$ |
|---|---|---|---|---|---|---|
| 426 | H | H | H | $CH_2NH_2$ | H | H |
| 427 | H | H | H | H | $CH_2NHMe$ | H |
| 428 | H | H | H | H | H | $CH_2NHMe$ |
| 429 | H | H | H | H | H | $CH_2NMe_2$ |
| 430 | H | H | H | H | H | $CH_2N$ 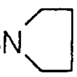 |
| 431 | H | H | H | H | $CH_2NH_2$ | Me |
| 432 | H | H | H | H | $CH_2NHMe$ | Me |
| 433 | H | H | H | H | Me | $CH_2NH_2$ |
| 434 | H | H | H | H | Me | $CH_2NHMe$ |

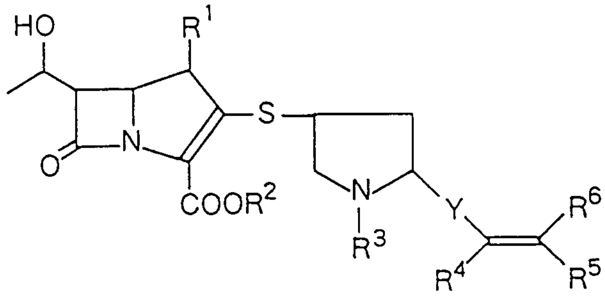

| No. | $R^1$ | $R^2$ | $R^3$ | Y | $R^4$ | $R^5$ | $R^6$ |
|---|---|---|---|---|---|---|---|
| 435 | Me | H | H | $CH_2$ | $CH_2NH_2$ | H | H |
| 436 | Me | H | H | $CH_2$ | $CH_2NHMe$ | H | H |
| 437 | Me | H | H | $CH_2$ | H | $CH_2NH_2$ | H |
| 438 | Me | H | H | $CH_2$ | H | $CH_2NHMe$ | H |
| 439 | Me | H | H | $CH_2$ | H | H | $CH_2NH_2$ |
| 440 | Me | H | H | $CH_2$ | H | H | $CH_2NHMe$ |

Among the above compounds, preferred are compounds identified by compound Nos. 1, 2, 5, 9, 12, 15, 21, 26, 35, 38, 46, 47, 50, 54, 57, 60, 66, 71, 80, 83, 91, 92, 95, 99, 102, 105, 111, 116, 125, 128, 136, 137, 138, 139, 142, 144, 145, 147, 150, 152, 153, 154, 155, 158, 160, 161, 162, 163, 166, 168, 169, 170, 171, 174, 176, 177, 178, 179, 182, 184, 185, 186, 187, 190, 192, 193, 194, 195, 198, 200, 201, 202, 203, 206, 208, 209, 210, 211, 214, 216, 217, 218, 219, 222, 224, 225, 226, 227, 230, 232, 233, 234, 235, 238, 240, 241, 242, 243, 246, 248, 249, 250, 251, 254, 256, 257, 258, 259, 262, 264, 265, 266, 267, 270, 272, 273, 274, 275, 278, 280, 281, 282, 283, 286, 288, 289, 290, 291, 294, 296, 297, 298, 299, 302, 304, 305, 306, 307, 310, 312, 313, 314, 315, 318, 320, 321, 322, 323, 326, 328, 329, 330, 331, 334, 336, 337, 338, 339, 342, 344, 345, 346, 347, 350, 352, 353, 426, 427, 428, 429, 430, 431, 432, 433 and 434.

Among them, particularly preferred are as follows:

1 (1R,5S,6S)-2-[(2S,4S)-2-[[1-(aminomethyl)vinyl]pyrrolidin-4-ylthio]]-6-[(R)-1-hydroxyethyl]-1-methyl-1-carbapen-2-em-3-carboxylic acid,

2 (1R,5S,6S)-6-[(R)-1-hydroxyethyl]-1-methyl-2-[(2S,4S)-2-[1-(N-methylaminomethyl)vinyl]-pyrrolidin-4-ylthio]-1-carbapen-2-em-3-carboxylic acid,

5 (1R,5S,6S)-2-[(2S,4S)-2-[1-(N,N-dimethylaminomethyl)vinyl]pyrrolidin-4-ylthio]-6-[(R)-1-hydroxyethyl]-1-methyl-1-carbapen-2-em-3-carboxylic acid,

9 (1R,5S,6S)-6-[(R)-1-hydroxyethyl]-1-methyl-2-[(2S,4S)-2-[1-(1-pyrrolidinylmethyl)vinyl]pyrrolidin-4-ylthio]-1-carbapen-2-em-3-carboxylic acid,

12 (1R,5S,6S)-6-[(R)-1-hydroxyethyl]-1-methyl-2-[(2S,4S)-2-[1-(4-methyl-1-piperazinylmethyl)vinyl]-pyrrolidin-4-ylthio]-1-carbapen-2-em-3-carboxylic acid,

15 (1R,5S,6S)-2-[(2S,4S)-2-[1-(4-carbamoylmethyl-1-piperazinylmethyl)vinyl]pyrrolidin-4-ylthio]-6-[-(R)-1-hydroxyethyl]-1-methyl-1-carbapen-2-em-3-carboxylic acid,

21 (1R,5S,6S)-6-[(R)-1-hydroxyethyl]-1-methyl-2-[(2S,4S)-2-[1-(N-methylpyrrolidiniomethyl)vinyl]-pyrrolidin-4-ylthio]-1-carbapen-2-em-3-carboxylate,

26 (1R,5S,6S)-2-[(2S,4S)-2-[1-(4-carbamoylmethyl-1-methylpiperaziniomethyl)vinyl]pyrrolidin-4-ylthio]-6-[(R)-1-hydroxyethyl]-1-methyl-1-carbapen-2-em-3-carboxylate,
38 (1R,5S,6S)-2-[(2S,4S)-2-[1-(N-carbamoylmethyl-1-pyrrolidiniomethyl)vinyl]pyrrolidin-4-ylthio]-6-[-(R)-1-hydroxyethyl]-1-methyl-1-carbapen-2-em-3-carboxylate,
46 (1R,5S,6S)-2-[(2S,4S)-2-[(E)-3-amino-1-propenyl]pyrrolidin-4-ylthio]-6-[(R)-1-hydroxyethyl]-1-methyl-1-carbapen-2-em-3-carboxylic acid,
47 (1R,5S,6S)-6-[(R)-1-hydroxyethyl]-1-methyl-2-[(2S,4S)-2-[(E)-3-(N-methylamino)-1-propenyl]-pyrrolidin-4-ylthio]-1-carbapen-2-em-3-carboxylic acid,
50 (1R,5S,6S)-2-[(2S,4S)-2-[(E)-3-(N,N-dimethylamino)-1-propenyl]pyrrolidin-4-ylthio]-6-[(R)-1-hydroxyethyl]-1-methyl-1-carbapen-2-em-3-carboxylic acid,
54 (1R,5S,6S)-6-[(R)-1-hydroxyethyl]-1-methyl-2-[(2S,4S)-2-[(E)-3-(1-pyrrolidinyl)-1-propenyl]-pyrrolidin-4-ylthio]-1-carbapen-2-em-3-carboxylic acid,
57 (1R,5S,6S)-6-[(R)-1-hydroxyethyl]-1-methyl-2-[(2S,4S)-2-[(E)-3-(4-methyl-1-piperazinyl)-1-propenyl]pyrrolidin-4-ylthio]-1-carbapen-2-em-3-carboxylic acid,
60 (1R,5S,6S)-2-[(2S,4S)-2-[(E)-3-(4-carbamoylmethyl-1-piperazinyl)-1-propenyl]pyrrolidin-4-ylthio]-6-[(R)-1-hydroxyethyl]-1-methyl-1-carbapen-2-em-3-carboxylic acid,
66 (1R,5S,6S)-6-[(R)-1-hydroxyethyl]-1-methyl-2-[(2S,4S)-2-[(E)-3-(N-methylpyrrolidinio)-1-propenyl]-pyrrolidin-4-ylthio]-1-carbapen-2-em-3-carboxylate,
71 (1R,5S,6S)-2-[(2S,4S)-2-[(E)-3-(4-carbonylmethyl-1-methylpiperazinio)-1-propenyl]pyrrolidin-4-ylthio]-6-[(R)-1-hydroxyethyl]-1-methyl-1-carbapen-2-em-3-carboxylate,
83 (1R,5S,6S)-2-[(2S,4S)-2-[(E)-3-(N-carbamoylmethyl-1-pyrrolidinio)-1-propenyl]pyrrolidin-4-ylthio]-6-[(R)-1-hydroxyethyl]-1-methyl-1-carbapen-2-em-3-carboxylate,
91 (1R,5S,6S)-2-[(2S,4S)-2-[(Z)-3-amino-1-propenyl]pyrrolidin-4-ylthio]-6-[(R)-1-hydroxyethyl]-1-methyl-1-carbapen-2-em-3-carboxylic acid,
92 (1R,5S,6S)-6-[(R)-1-hydroxyethyl]-1-methyl-2-[(2S,4S)-2-[(Z)-3-(N-methylamino)-1-propenyl]-pyrrolidin-4-ylthio]-1-carbapen-2-em-3-carboxylic acid,
95 (1R,5S,6S)-2-[(2S,4S)-2-[(Z)-3-(N,N-dimethylamino)-1-propenyl]pyrrolidin-4-ylthio]-6-[(R)-1-hydroxyethyl]-1-methyl-1-carbapen-2-em-3-carboxylic acid,
99 (1R,5S,6S)-6-[(R)-1-hydroxyethyl]-1-methyl-2-[(2S,4S)-2-[(Z)-3-(1-pyrrolidinyl)-1-propenyl]-pyrrolidin-4-ylthio]-1-carbapen-2-em-3-carboxylic acid,
102 (1R,5S,6S)-6-[(R)-1-hydroxyethyl]-1-methyl-2-[(2S,4S)-2-[(Z)-3-(4-methyl-1-piperazinyl)-1-propenyl]pyrrolidin-4-ylthio]-1-carbapen-2-em-3-carboxylic acid,
105 (1R,5S,6S)-2-[(2S,4S)-2-[(Z)-3-(4-carbamoylmethyl-1-piperazinyl)-1-propenyl]pyrrolidin-4-ylthio]-6-[(R)-1-hydroxyethyl]-1-methyl-1-carbapen-2-em-3-carboxylic acid,
111 (1R,5S,6S)-6-[(R)-1-hydroxyethyl]-1-methyl-2-[(2S,4S)-2-[(Z)-3-(N-methylpyrrolidinio)-1-propenyl]-pyrrolidin-4-ylthio]-1-carbapen-2-em-3-carboxylate,
116 (1R,5S,6S)-2-[(2S,4S)-2-[(Z)-3-(4-carbamoylmethyl-1-methylpiperazinio)-1-propenyl]pyrrolidin-4-ylthio]-6-[(R)-1-hydroxyethyl]-1-methyl-1-carbapen-2-em-3-carboxylate,
128 (1R,5S,6S)-2-[(2S,4S)-2-[(Z)-3-(N-carbamoylmethyl-1-pyrrolidinio)-1-propenyl]pyrrolidin-4- ylthio]-6-[(R)-1-hydroxyethyl]-1-methyl-1-carbapen-2-em-3-carboxylate,
224 (1R,5S,6S)-2-(2S,4S)-2-[(E)-3-amino-2-methyl-1-propenyl]pyrrolidin-4-ylthio]-6-[(R)-1-hydroxyethyl]-1-methyl-1-carbapen-2-em-3-carboxylic acid,
225 (1R,5S,6S)-6-[(R)-1-hydroxyethyl]-2-[(2S,4S)-2-[(E)-3-(N-methylamino)-2-methyl-1-propenyl]-pyrrolidin-4-ylthio]-1-methyl-1-carbapen-2-em-3-carboxylic acid,
304 (1R,5S,6S)-2-[(2S,4S)-2-[(Z)-3-amino-2-methyl-1-propenyl]pyrrolidin-4-ylthio]-6-[(R)-1-hydroxyethyl]-1-methyl-1-carbapen-2-em-3-carboxylic acid,
305 (1R,5S,6S)-6-[(R)-1-hydroxyethyl]-2-1(2S,4S)-2-[(Z)-3-(N-methylamino)-2-methyl-1-propenyl]-pyrrolidin-4-ylthio]-1-methyl-1-carbapen-2-em-3-carboxylic acid,
352 (5R,6S)-2-[(2S,4S)-2-[(E)-3-amino-1-propenyl]pyrrolidin-4-ylthio]-6-[(R)-1-hydroxyethyl]-1-carbapen-2-em-3-carboxylic acid,
353 (5R,6S)-2-[(2S,4S)-2-[(Z)-3-amino-1-propenyl]pyrrolidin-4-ylthio]-6-[(R)-1-hydroxyethyl]-1-carbapen-2-em-3-carboxylic acid,
426 (5R,6S)-2-[(2S,4S)-2-[[1-(aminomethyl)vinyl]pyrrolidin-4-ylthio]]-6-[(R)-1-hydroxyethyl]-1-carbapen-2-em-3-carboxylic acid,
427 (5R,6S)-6-[(R)-1-hydroxyethyl]-2-[(2S,4S)-2-[(E)-3-(N-methylamino)-1-propenyl]pyrrolidin-4-ylthio]-1-carbapen-2-em-3-carboxylic acid,
428 (5R,6S)-6-[(R)-1-hydroxyethyl]-2-[(2S,4S)-2-[(Z)-3-(N-methylamino)-1-propenyl]pyrrolidin-4-ylthio]-1- carbapen-2-em-3-carboxylic acid,

429 (5R,6S)-2-[(2S,4S)-2-[(Z)-3-(N,N-dimethylamino)-1-propenyl]pyrrolidin-4-ylthio]-6-[(R)-1-hydroxyethyl]-1-carbapen-2-em-3-carboxylic acid,

430 (5R,6S)-6-[(R)-1-hydroxyethyl]-2-[(2S,4S)-2-[(Z)-3-(1-pyrrolidinyl)-1-propenyl]pyrrolidin-4-ylthio]-1-carbapen-2-em-3-carboxylic acid,

431 (5R,6S)-2-[(2S,4S)-2-[(E)-3-amino-2-methyl-1-propenyl]pyrrolidin-4-ylthio]-6-[(R)-1-hydroxyethyl]-1-carbapen-2-em-3-carboxylic acid,

432 (5R,6S)-6-[(R)-1-hydroxyethyl]-2-[(2S,4S)-2-[(E)-3-(N-methylamino)-2-methyl-1-propenyl]-pyrrolidin-4-ylthio]-1-carbapen-2-em-3-carboxylic acid,

433 (5R,6S)-2-[(2S,4S)-2-[(Z)-3-amino-2-methyl-1-propenyl]pyrrolidin-4-ylthio]-6-[(R)-1-hydroxyethyl]-1-carbapen-2-em-3-carboxylic acid and

434 (5R,6S)-6-[(R)-1-hydroxyethyl]-2-[(2S,4S)-2-[(Z)-3-(N-methylamino)-2-methyl-1-propenyl]-pyrrolidin-4-ylthio]-1-carbapen-2-em-3-carboxylic acid.

Especially preferred are compounds No. 91 i.e. (1R,5S,6S)-2-[(2S,4S)-2-[(Z)-3-amino-1-propenyl]-pyrrolidin-4-ylthio]-6-[(R)-1-hydroxyethyl]-1-methyl-1-carbapen-2-em-3-carboxylic acid, No. 304 i.e. (1R,5S,6S)-2-[(2S,4S)-2-[(Z)-3-amino-2-methyl-1-propenyl]pyrrolidin-4-ylthio]-6-[(R)-1-hydroxyethyl]-1-methyl-1-carbapen-2-em-3-carboxylic acid and No. 305 i.e. (1R,5S,6S)-6-[(R)-1-hydroxyethyl]-2-[(2S,4S)-2-[-(Z)-3-(N-methylamino)-2-methyl-1-propenyl]pyrrolidin-4-ylthio]-1-methyl-1-carbapen-2-em-3-carboxylic acid.

Now, the process for producing the compound of the present invention will be described.

An activating reagent is reacted to a compound of the formula:

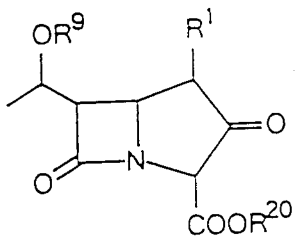

(II)

wherein $R^1$ is a hydrogen atom or a methyl group, $R^9$ is a hydrogen atom or a hydroxyl-protecting group, and $R^{20}$ is a hydrogen atom or a carboxyl-protecting group, in an inert organic solvent in the presence of a base to form a reactive derivative of the formula (II'):

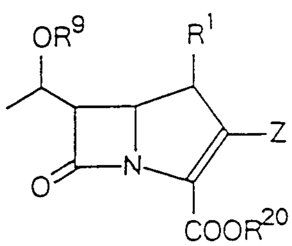

(II')

wherein $R^1$, $R^9$ and $R^{20}$ are as defined above, and Z is a leaving group.

The inert organic solvent to be used for the reaction may, for example, be diethyl ether, tetrahydrofuran, dioxane, benzene, toluene, chlorobenzene, methylene chloride, chloroform, carbon tetrachloride, dichloroethane, trichloroethylene, acetone, ethyl acetate, acetonitrile, N,N-dimethylformamide, hexamethylphosphoric triamide or a mixture of such solvents. Particularly preferred are acetonitrile and benzene.

The base to be used for the reaction may, for example, be a tertiary aliphatic amine such as trimethylamine, triethylamine, N,N-diisopropylethylamine, N-methylmorpholine, N-methylpyrrolidine, N-methylpiperidine, N,N-dimethylaniline, 1,8-diazabicyclo[5.4.0]undec-7-ene (DBU) or 1,5-diazabicyclo[4.3.0]-non-5-ene (DBN); or an aromatic amine such as pyridine, 4-dimethylaminopyridine, picoline, lutidine, quinoline or isoquinoline. Particularly preferred are N,N-diisopropylethylamine and triethylamine.

The activating reagent to be used for the reaction may, for example, be an acid anhydride such as trifluoroacetic anhydride, methanesulfonic anhydride, trifluoromethanesulfonic anhydride or p-toluenesulfonic anhydride; or an acid chloride such as methanesulfonyl chloride, p-toluenesulfonyl chloride or diphenyl chlorophosphate. Particularly preferred is diphenyl chlorophosphate.

In the formula (II'), Z is a leaving group such as a trifluoroacetoxy group, a methanesulfonyloxy group, a trifluoromethanesulfonyloxy group, a p-toluenesulfonyloxy group or a diphenoxyphosphoryloxy group. Particularly preferred is a diphenoxyphosphoryloxy group.

For the reaction, from 1 to 3 mols, preferably from 1 to 1.5 mols, of the base and from 1 to 1.2 mols of the activating reagent are used per mol of the compound of the formula (II).

The reaction is conducted usually within a temperature range of from -40 to 50 ° C, preferably from -20 to 20 ° C, and usually completed quantitatively in from 0.5 to 3 hours.

After completion of the reaction, the reaction product is treated in accordance with a usual method to obtain the reactive derivative (II') of the compound of the formula (II) quantitatively.

The reaction of the reactive derivative of the formula (II') with a compound of the formula:

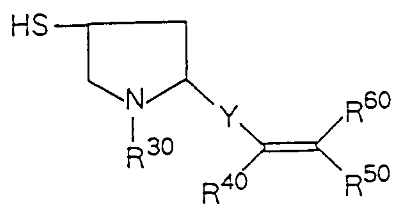

(III)

wherein $R^{30}$ is a hydrogen atom, a lower alkyl group or an imino-protecting group, each of $R^{40}$, $R^{50}$ and $R^{60}$ is a hydrogen atom, a cyano group, a methanesulfonyl group, a lower alkyl group which may have substituents, a carbamoyl group which may have substituents or a group of the formula $-X^1-N(R^{71})R^{81}$ {wherein each of $R^{71}$ and $R^{81}$ which may be the same or different, is a hydrogen atom, a lower alkyl group which may have substituents or an amino-protecting group, or $R^{71}$ and $R^{81}$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to $X^1$ or other nitrogen atom on the heterocyclic ring may form an ammonio group), and $X^1$ is a lower alkylene group which may have substituents}, provided that at least one of $R^{40}$, $R^{50}$ and $R^{60}$ is the group of the formula $-X^1-N(R^{71})R^{81}$ wherein $R^{71}$, $R^{81}$ and $X^1$ are as defined above, and Y is a lower alkylene group or a single bond (provided that the substituents of the lower alkyl group, the heterocyclic group and the lower alkylene group, as well as the carbamoyl group and the imino group on the heterocyclic ring, may be protected, as the case requires), is conducted using the above mentioned inert organic solvent and base to form a compound of the formula:

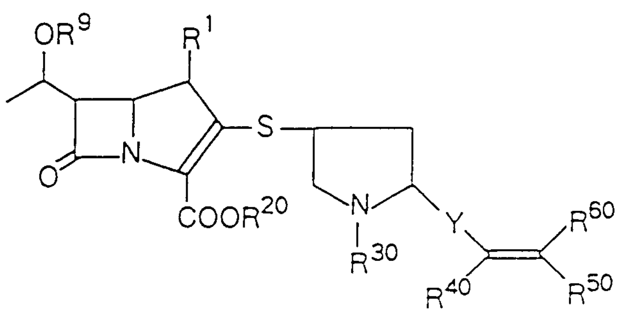

(IV)

wherein $R^1$, $R^9$, $R^{20}$, $R^{30}$, $R^{40}$, $R^{50}$, $R^{60}$ and Y are as defined above.

The reaction is conducted using from 1 to 2 mols, preferably from 1 to 1.5 mols, of the base and from 1 to 1.2 mols of the compound of the formula (III), per mol of the reactive derivative of the formula (II'). The reaction is conducted usually within a temperature range of from -40 to 50 ° C, preferably from -20 to 20 ° C, and the reaction is completed usually in from 0.5 to 3 hours.

Further, the compound of the formula (IV) can be prepared in one step from the compound of the formula (II). Namely, without isolating the reactive derivative of the formula (II') prepared from the compound of the formula (II), the compound of the formula (III) is reacted thereto in the same reaction system to prepare the compound of the formula (IV) efficiently. To conduct the production in one step, from 2 to 4 mols, preferably from 2.5 to 3.5 mols, of the base is employed per mol of the compound of the formula (II).

After completion of the reaction, usual treatment is conducted to obtain a crude product of the formula (IV), which may be subjected to a reaction for removing a protecting group without purification. However, it is preferred to purify the crude product (IV) by crystallization or by column chromatography by means of e.g. silica gel.

From the compound of the formula (IV) thus obtained, a compound of the formula (I) can be obtained, if necessary, by conducting a reaction for removing a protecting group for a hydroxyl group, an amino or imino group and a carboxyl group.

For the removal of the protecting groups, the method varies depending upon the type of the protecting groups. However, the removal can be conducted in accordance with conventional methods, for example, by solvolysis, by chemical reduction or by hydrogenation.

For example, when in the above formula (IV), the protecting group for the hydroxyl group and/or for the amino or imino group is an aralkyloxycarbonyl group such as a benzyloxycarbonyl group or a p-nitrobenzyloxycarbonyl group, and the protecting group for the carboxyl group is an aralkyl group such as a benzyl group, a p-nitrobenzyl group or a benzhydryl group, such protecting groups can be removed by catalytic hydrogenation by means of a platinum catalyst such as platinum oxide, platinum wire or platinum black, or a palladium catalyst such as palladium black, palladium oxide, palladium-carbon or palladium hydroxide-carbon.

As a solvent to be used for such a catalytic hydrogenation reaction, methanol, ethanol, tetrahydrofuran, dioxane, acetic acid or a solvent mixture of such an organic solvent with water or with a buffer solution of e.g. a phosphate, may be used.

The reaction can be completed in from 0.5 to 4 hours at a temperature within a range of from 0 to 50 °C under hydrogen gas stream of from 1 to 4 atm.

When in the above formula (IV), the protecting group for the hydroxyl group and/or the amino or imino group is an allyloxycarbonyl group, and the protecting group for the carboxyl group is an allyl group, such protecting groups can be removed by reacting an organo-soluble palladium complex catalyst in an inert organic solvent containing an allyl group-capturing agent (method by W. McCombie et al., J. Org. Chem., vol. 47, p. 587-590 (1982) and method by F. Guibé, the same literature, vol. 52, p. 4,984-4,993 (1987)).

The solvent useful for the reaction includes, for example, water, acetone, diethyl ether, tetrahydrofuran, dioxane, ethyl acetate, acetonitrile, methylene chloride, chloroform and a solvent mixture thereof.

The palladium compound complex useful for this reaction includes, for example, palladium-carbon, palladium hydroxide-carbon, palladium(II) chloride, palladium(II) acetate, tetrakis(triphenylphosphine)-palladium (O), tetrakis(triphenoxyphosphine)palladium (O), tetrakis(triethoxyphosphine)palladium (O), bis-[ethylenebis(diphenylphosphine)]palladium (O), tetrakis[tri(2-furyl)phosphine]palladium (O), bis-(triphenylphosphine)palladium(II) chloride and bis(triphenylphosphine)palladium(II) acetate.

The allyl group-capturing agent may, for example, be dimedone, formic acid, acetic acid, ammonium formate, sodium formate, sodium 2-ethylhexanoate, potassium 2-ethylhexanoate, pyrrolidine, piperidine and tributyltin hydride.

The reaction is conducted usually within a temperature range of from -10 to 50 °C, preferably from 0 to 30 °C using from 0.01 to 0.5 mol of the catalyst and from 1 to 6 mols of the nucleophilic agent relative to 1 mol of the compound of the formula (IV), and the reaction is completed usually in from 0.5 to 3 hours.

Further, when in the above formula (IV), the protecting group for the hydroxyl group and/or the amino or imino group is an o-nitrobenzyloxycarbonyl group, and the protecting group for the carboxyl group is an o-nitrobenzyl group, such protecting groups can be removed by a photo reaction (method by Amit et al., J. Org. Chem., vol. 39, p. 192-196 (1974)).

After completion of the reactions for removing the protecting groups, the compound of the formula (I) can be isolated by usual treatment such as column chromatography using silica gel or adsorptive resin, freeze-drying or crystallization.

Further, when the protecting group for the carboxyl group at the 3-position of the compound of the formula (IV) is a lower alkanoyloxyalky group such as an acetoxymethyl group or a pivaloyloxymethyl group, a methoxymethyl group, an indanyl group or a phthalidyl group, such an ester will be physiologically hydrolyzed in vivo. Therefore, such a compound can directly be administered to a human being or to an animal without preliminarily removing the protecting group.

The compound of the formula (I) can be converted to a pharmaceutically acceptable salt or ester by a conventional method.

The starting material of the formula (II) can be prepared, for example, by a method by Salzmann et al. when $R^1$ is a hydrogen atom (J. Am. Chem. Soc., vol. 102, p.6161-6163 (1981)) or by a method by Shih et al. when $R^1$ is a methyl group (Heterocycles, vol. 21, p.29-40 (1984)).

The starting material of the formula (III) can be synthesized by the following method.

The hydroxyl group of the compound 1 is activated by a usual method, and a thioacetate such as potassium thioacetate is reacted thereto to convert it to an acetylthio derivative 3, followed by alkali or acid hydrolysis to obtain a thiol derivative of the formula (III).

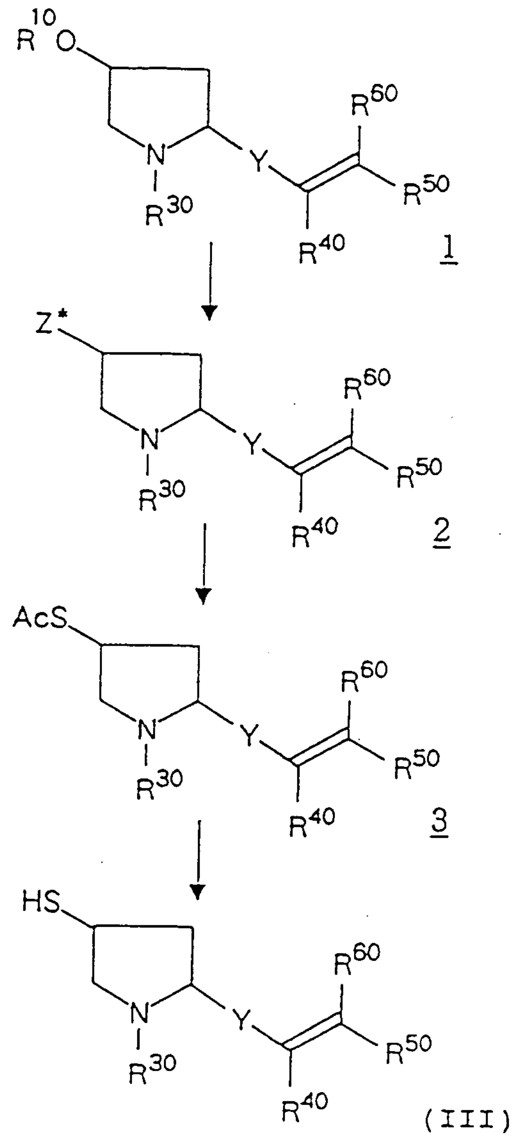

1

2

3

(III)

In the above formulas, $R^{10}$ is a hydrogen atom or a hydroxyl-protecting group, Z* is a leaving group selected from the group consisting of a chlorine atom, a bromine atom, an iodine atom, a trifluoroacetoxy group, a methanesulfonyloxy group, a trifluoromethanesulfonyloxy group and a p-toluenesulfonyloxy group, Ac is an acetyl group, and $R^{30}$, $R^{40}$, $R^{50}$, $R^{60}$ and Y are as defined above.

A group of compounds having the formula 1 can be prepared in accordance with the methods described in the Reference Examples.

The compounds of the present invention exhibit strong antibacterial activities against various gram positive bacteria and gram negative bacteria.

To demonstrate the usefulness of the compounds of the present invention, the in vitro antibacterial activities against bacteria were measured by the following agar plate dilution method (standard method by Japan Chemotherapy Society, Chemotherapy, vol. 29, p. 76-79 (1981)). One platinum loopful of each test microorganism incubated overnight in Mueller Hinton broth, was inoculated to Mueller Hinton agar (inoculum size: $10^6$ CFU/mℓ). Such culture media contained antibacterial agents in various concentrations. After incubation at 37°C for 16 hours, the minimum inhibitory concentrations (MIC: μg/mℓ) were measured.

The results of the antibacterial activities of the compounds of the present invention are shown in Table 1.

Table 1

Minimum Inhibitory Concentration(MIC: μg/mℓ)

| Test microorganism | Example 2 | Example 7 | Example 9 | BO-2171 | Imipenem |
|---|---|---|---|---|---|
| *S. aureus* 209P NIHJ JC1 | <0.006 | <0.006 | <0.006 | 0.025 | <0.006 |
| *S. aureus* MB 4970 | 0.012 | <0.006 | <0.006 | 0.025 | 0.012 |
| *S. aureus* BB 5939* | 1.56 | 1.56 | 1.56 | 6.25 | 6.25 |
| *P. aeruginosa* MB 5002 | 0.78 | 0.78 | 0.78 | 6.25 | 1.56 |
| *P. aeruginosa* MB 5178 | 1.56 | 1.56 | 3.13 | 12.5 | 25 |

*β-lactamase producing microorganism

The antibacterial activities of the compounds of the present invention described in the Examples, as representative examples of the compound of the present invention, were measured by a disc diffusion test by the method of Bauer et al. (Amer. J. Clin. Pathol., vol. 45, p. 493 (1966)). Thienamycin or imipenem was used as the internal standard.

MIC of each test compound was calculated from the diameter of the inhibition ring formed by the disc containing the test compound by using the calculation formula reported by Humphrey and Lightbown (J. Gen. Microbiol., vol. 7, p. 129 (1952)). For each microorganism, a geometrical average of MIC was obtained, and the activity ratio to thienamycin was calculated.

The antibacterial activities are represented by the ratio to thienamycin ( = 1.0), whereby the larger the numerical value, the higher the activities.

The DHP-I susceptibility was quantitatively analyzed by the method by Kropp et al., Antimicrob. Agents

Chemother., vol. 22, p. 62-70 (1982), whereby the smaller the numerical value representing the ratio to imipenem (=1.0), the higher the stability. The antibacterial potency and the DHP-I susceptibility of the compounds of the present invention were compared with imipenem and BO-2171. The results are shown in Table 2.

Table 2

Relative antibacterial potency to thienamycin and DHP-I susceptibility

| Test microorganism | Example 2 | Example 7 | Example 9 | BO-2171 | Imipenem |
|---|---|---|---|---|---|
| Meth-R <u>S. aureus</u> | 12.9 | 13.5 | 19.7 | 7.68 | 2.57 |
| THM-R <u>P.aeruginosa</u> | 15.2 | 31.3 | 13.4 | 3.28 | 2.0 |
| DHP-I susceptibility | <0.05 | <0.05 | 0.10 | 0.3 | 1.0 |

The compounds of the present invention are characterized in that at least one of $R^4$, $R^5$ and $R^6$ has a primary, secondary or tertiary amino group or an ammonio group, and they have excellent antibacterial activities against various gram positive bacteria and gram negative bacteria and are useful as antibacterial agents for the treatment and prevention of the human infectious diseases caused by such bacteria. Typical pathogens sensitive to the antibacterial agents of the present invention include, for example, species of genus Staphylococcus, genus Enterococcus, genus Escherichia, genus Enterobacter, genus Klebsiella,

genus Serratia, genus Proteus and genus Pseudomonas. The compounds of the present invention exhibit excellent antibacterial activities particularly against Methicillin resistant Staphylococcus aureus and against thienamycin resistant Pseudomonas aeruginosa.

The compounds of the present invention are very stable against DHP-I although the stability varies depending upon the individual compounds, and they are excellent also in the physicochemical stability and in the solubility in water.

The compounds of the present invention may be used in the form of drug formulations suitable for non-oral administration, oral administration or external administration, by mixing them with carriers of solid or liquid excipients known in this field. The main administration route is non-oral (intravenous or intramuscular injection) administration by injection or local administration. Drug formulations include liquid formulations such as injection solutions, syrups or emulsions, solid formulations such as tablets, capsules or granules, and external application formulations such as ointments or suppositories. These formulations may contain additives such as a base, an assisting agent, a stabilizer, a wetting agent, an emulsifier, an absorption-promoting agent, a surfactant, etc. which are commonly employed, as the case requires.

The additives include, for example, distilled water for injection, Ringer's solution, glucose, sucrose syrup, gelatin, edible oil, cacao butter, ethylene glycol, sucrose, corn starch, magnesium stearate and talc.

The dose varies depending upon the condition of the patient, the weight, the age, the sex, the type of formulation, the number of administration times, etc. Usually, however, a preferred daily dose of the active ingredient to an adult is from about 5 to 50 mg/kg, and a preferred daily dose to a child is within a range of from about 5 to 25 mg/kg, which is preferably administered once a day or in a few times a day.

The compound of the present invention may be administered in combination with a DHP-I inhibiting agent such as cilastatin [sodium (Z)-7-(L-amino-2-carboxyethylthio)-2-(2,2-dimethylcyclopropanecarboxamide)-2-heptenoate] (Japanese Unexamined Patent Publication No. 81518/1981; European Patent No. 28,778; J. Med. Chem., vol. 30, p. 1074 (1987)).

Now, the present invention will be described in further detail with reference to Examples and Reference Examples. However, it should be understood that the present invention is by no means restricted by such specific Examples.

In the thin layer chromatography in the Examples and Reference Examples, silica gel $60F_{245}$ (Merck) was used as the plate, and an ultraviolet detector was used as a detecting device. As the silica gel for the column, Wakogel™ C-300 (Wako Junyaku) was used, and as the silica gel for reversed phase column, LC-SORB™ SP-B-ODS (Chemco) or YMC.GEL™ ODS-AQ 120-S50 (Yamamura Chemical Laboratories) was used. As the high pressure liquid chromatograph, JASCO 800 series (Nippon Bunko) was used. When the NMR spectrum was measured using a dimethyl sulfoxide-$d_6$ or chloroform-d solution, tetramethylsilane (TMS) was used as the internal standard, and when measured using a deuterium oxide solution, 2,2-dimethyl-2-silapentane-5-sulfonate (DSS) was used as the internal standard, and the measurement was conducted by means of XL-200 (200 MHz;Varian) model spectrometer. All $\delta$ values are shown by ppm.

The meanings of the abbreviations used for the NMR measurement are as follows:

s: singlet
d: doublet
t: triplet
q: quartet
ABq: AB-type quartet
dd: double doublet
m: multiplet
br: broad
J: coupling constant
Hz: hertz
$CDCl_3$: chloroform-d
$D_2O$: deuterium oxide

The meanings of the abbreviations used in the reaction formulas are as follows:

Ac: acetyl group
All: allyl group
Alloc: allyloxycarbonyl group
Boc: tert-butoxycarbonyl group
Ms: methanesulfonyl group
TBS: tert-butyldimethylsilyl group
Tr: trityl group

EXAMPLE 1

(1R,5S,6S)-2-[(2S,4S)-2-[(E)-3-amino-1-propenyl]pyrrolidin-4-ylthio]-6-[(R)-1-hydroxyethyl]-1-methyl-1-carbapen-2-em-3-carboxylic acid

1)

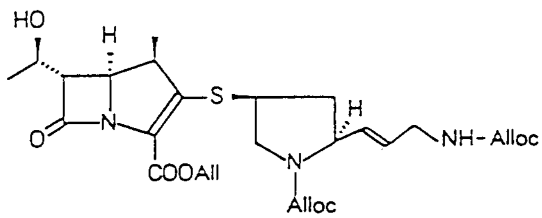

Allyl (1R,5S,6S)-2-diphenoxyphosphoryloxy-6-[(R)-1-hydroxyethyl]-1-methyl-1-carbapen-2-em-3-carboxylate (516 mg, 1.03 mmol) and (2S,4S)-N-allyloxycarbonyl-2-[(E)-3-allyloxycarbonylamino-1-propenyl]-4-mercaptopyrrolidine (360 mg, 1.05 mmol, compound of Reference Example 1) were dissolved in acetonitrile (20 ml). Under a nitrogen stream, N,N-diisopropylethylamine (0.19 ml, 1.09 mmol) was dropwise added thereto at -10 °C, and then the mixture was stirred overnight at 5 °C. The reaction solution was concentrated under reduced pressure, and the residue was subjected to silica gel column chromatography (Wakogel™ C-300, ethyl acetate) to obtain allyl (1R,5S,6S)-2-[(2S,4S)-N-allyloxycarbonyl-2-[(E)-3-allyloxycarbonylamino-1-propenyl]pyrrolidin-4-ylthio]-6-[(R)-1-hydroxyethyl]-1-methyl-1-carbapen-2-em-3-carboxylate (296 mg, yield: 49.8%).

IR(KBr)cm$^{-1}$: 1770, 1700, 1540, 1400

NMR($CDCl_3$) $\delta$:

1.25(3H,d,J=8Hz),1.34(3H,d,J=7Hz),1.80(1H,m), 2.35-2.68(3H,m),3.13-3.45(4H,m),5.15-5.55(6H,m), 5.64-(2H,m),5.80-6.10(3H,m)

2)

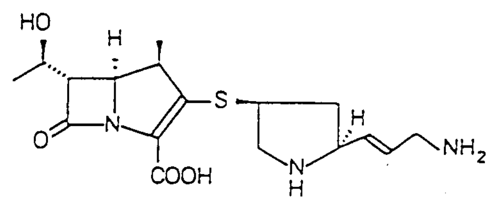

To a methylene chloride solution (11 ml) of the compound (295 mg, 0.51 mmol) obtained by the above reaction, water (46 $\mu$l), bis(triphenylphosphine)palladium(II) chloride (18 mg, 0.025 mmol) and tributyltin hydride (0.54 ml, 2.0 mmol) were added in a nitrogen stream under cooling with ice. This solution was stirred at the same temperature for 20 minutes and at room temperature for 20 minutes. The reaction mixture was extracted with water (70 ml). The aqueous layer was washed twice with chloroform, and then insoluble matters were filtered off. The filtrate was concentrated to a volume of about 15 ml under reduced pressure and then subjected to reversed phase column chromatography (YMC-GEL™ ODS-AQ 120-250, 50 ml, a 20% methanol aqueous solution). The fraction containing the desired product was concentrated and freeze-dried to obtain the above-identified compound (58 mg, yield: 30.8%).

IR(KBr)cm$^{-1}$: 1750, 1580, 1390

NMR($D_2O$) $\delta$:

1.20(3H,d,J=7Hz),1.29(3H,d,J=6Hz),1.47(1H,m), 2.54(1H,m),3.02(1H,dd,J=12,3Hz),3.20-3.47(3H,m), 3.59-(2H,d,J=6Hz),3.78(2H,m),4.22(2H,m), 5.81(1H,dt,J=16,6Hz),5.98(1H,dd,J=16,7Hz)

HPLC:

| | |
|---|---|
| Column: | YMC™-Pack ODS-AQ, 5$\mu$, 4.6$\phi$ × 150 mm |
| Eluent: | 0.01 M Phosphate buffer (pH 6.5)-Methanol (80:20) |
| Flow rate: | 1.0 ml/min |

| | |
|---|---|
| Temperature: | 40 ° C |
| Detector: | 290 nm |
| Retention time: | 3.14 min |

EXAMPLE 2

(1R,5S,6S)-2-[(2S,4S)-2-[(Z)-3-amino-1-propenyl]pyrrolidin-4-ylthio]-6-[(R)-1-hydroxyethyl]-1-methyl-1-carbapen-2-em-3-carboxylic acid

1)

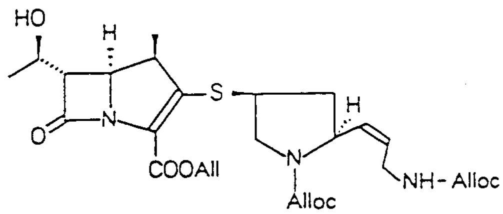

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (850 mg, 1.70 mmol) and (2S,4S)-N-allyloxycarbonyl-2-[(Z)-3-allyloxycarbonylamino-1-propenyl]-4-mercaptopyrrolidine (460 mg, 1.42 mmol, compound of Reference Example 2) to obtain allyl (1R,5S,6S)-2-[(2S,4S)-N-allyloxycarbonyl-2-[(Z)-3-allyloxycarbonylamino-1-propenyl]pyrrolidin-4-ylthio]-6-[(R)-1-hydroxyethyl]-1-methyl-1-carbapen-2-em-3-carboxylate (399 mg, yield: 40.7%).
IR(KBr)$cm^{-1}$: 1780, 1700, 1410
NMR($CDCl_3$) δ:
1.28(3H,d,J = 8Hz),1.38(3H,d,J = 7Hz),2.62(1H,m), 3.22-3.45(3H,m),4.26(2H,m),5.82-6.10(3H,m)

2)

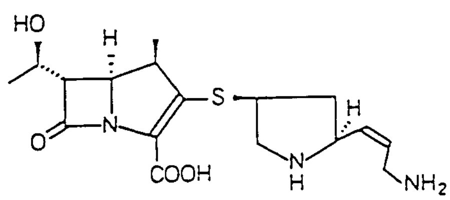

The same procedure as in Example 1-2 was carried out by using the compound (382 mg, 0.66 mmol) obtained by the above reaction, to obtain the above-idendified compound (78 mg, yield: 32.0%).
IR(KBr)$cm^{-1}$: 1750, 1580, 1390
NMR($D_2O$) δ:
1.21(3H,d,J = 8Hz),1.29(3H,d,J = 7Hz),1.44(1H,m), 2.76(1H,m),3.04(1H,dd,J = 12,4Hz),3.20-3.48(3H,m), 3.70-(2H,d,J = 8Hz),4.23(2H,m),5.68(1H,dt,J = 10,8Hz), 5.87(1H,dd,J = 10,9Hz)
HPLC (the same condition as in Example 1)
Retention time: 2.92 min

EXAMPLE 3

(1R,5S,6S)-6-[(R)-1-hydroxyethyl]-1-methyl-2-[(2S,4S)-2-[(Z)-3-(N-methylamino)-1-propenyl]pyrrolidin-4-ylthio]-1-carbapen-2-em-3-carboxylic acid

1)

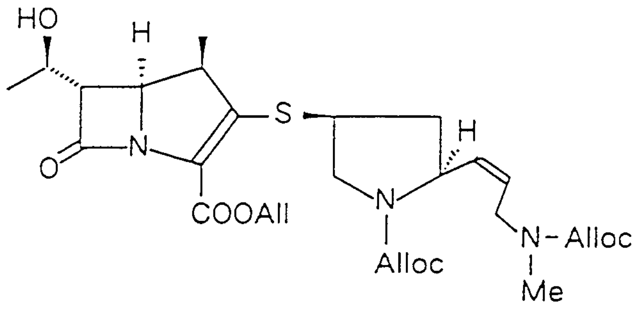

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (760 mg, 1.52 mmol) and (2S,4S)-N-allyloxycarbonyl-2-[(Z)-3-(N-allyloxycarbonyl-N-methylamino)-1-propenyl]-4-mercaptopyrrolidine (416 mg, 1.26 mmol, compound of Reference Example 3) to obtain allyl (1R,5S,6S)-2-[(2S,4S)-N-allyloxycarbonyl-2-[(Z)-3-(N-allyloxycarbonyl-N-methylamino)-1-propenyl]pyrrolidin-4-ylthio]-6-[(R)-1-hydroxyethyl]-1-methyl-1-carbapen-2-em-3-carboxylate (502 mg, yield: 56.9%).
IR(KBr)$cm^{-1}$: 1780, 1700, 1400, 1210
NMR($CDCl_3$) δ:
1.28(3H,d,J = 8Hz),1.37(3H,d,J = 7Hz),1.70(1H,m), 4.52-4.92(6H,m),5.16-5.66(8H,m),5.80-6.10(3H,m)

2)

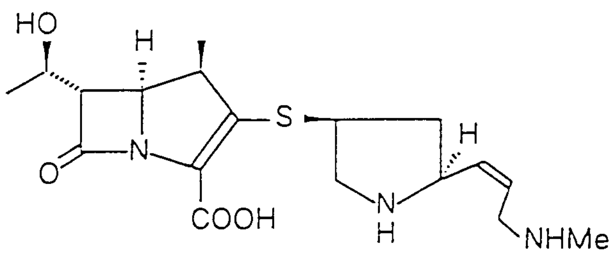

The same procedure as in Example 1-2 was carried out by using the compound (500 mg, 0.85 mol) obtained by the above reaction, to obtain the above-identified compound (84 mg, yield: 30.0%).
IR(KBr)$cm^{-1}$: 1750, 1590, 1390
NMR($D_2O$) δ:
1.18(3H,d,J = 8Hz),1.26(3H,d,J = 7Hz),1.44(1H,m), 2.55(1H,m),2.67(3H,s),3.04(1H,dd,J = 12,4Hz), 3.22-3.46-(3H,m),3.73(2H,d,J = 8Hz),3.82(1H,m), 4.05(1H,q,J = 8Hz),4.20(2H,m),5.67(1H,m), 5.94(1H,t,J = 10Hz)

EXAMPLE 4

(1R,5S,6S)-2-[(2S,4S)-2-[(Z)-3-(N,N-dimethylamino)-1-propenyl]pyrrolidin-4-ylthio]-6-[(R)-1-hydroxyethyl]-1-methyl-1-carbapen-2-em-3-carboxylic acid

1)

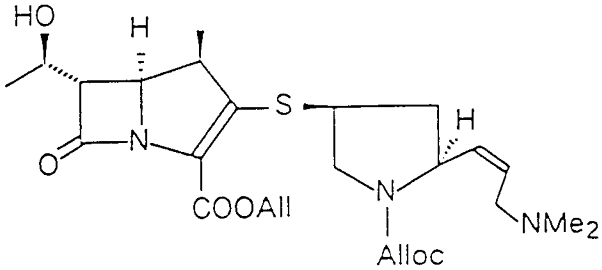

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (1.0 g, 20 mmol)

and (2S,4S)-N-allyloxycarbonyl-2-[(Z)-3-(N,N-dimethylamino)-1-propenyl]-4-mercaptopyrrolidine trifluoroacetate (640 mg, 1.68 mmol, compound of Reference example 4) to obtain allyl (1R,5S,6S)-2-[-(2S,4S)-N-allyloxycarbonyl-2-[(Z)-3-(N,N-dimethylamino)-1-propenyl]pyrrolidin-4-ylthio]-6-[(R)-1-hydroxyethyl]-1-methyl-1-carbapen-2-em-3-carboxylate (150 mg, yield: 14.5 %).
IR(KBr)cm$^{-1}$: 1770, 1700, 1400, 1200
NMR($CDCl_3$) δ:
1.24 (3H,d,J = 8Hz),1.33(3H,d,J = 7Hz),1.68(1H,m), 2.07-2.66(7H,m),4.46-4.90(5H,m),5.12-5.52(4H,m), 5.62-(2H,m),5.94(2H,m)

2)

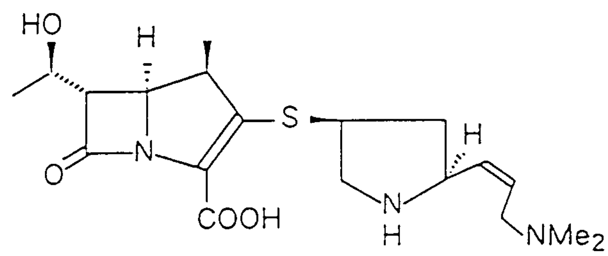

The same procedure as in Example 1-2 was carried out by using the compound (150 mg, 0.29 mmol) obtained by the above reaction, to obtain the above-identified compound (38 mg, yield: 33.1%).
IR(KBr)cm$^{-1}$: 1760, 1600, 1390
NMR($D_2O$) δ:
1.20(3H,d,J = 8Hz),1.28(3H,d,J = 7Hz),1.53(1H,m), 2.60(1H,m),2.79(6H,s),3.10(1H,dd,J = 12,4Hz), 3.28-3.48-(3H,m),3.75(2H,d,J = 8Hz),3.87(1H,m), 4.21(3H,m)

EXAMPLE 5

(1R,5S,6S)-6-[(R)-1-hydroxyethyl]-1-methyl-2-[(2S,4S)-2-[(Z)-3-(1-pyrrolidinyl)-1-propenyl]pyrrolidin-4-ylthio]-1-carbapen-2-em-3-carboxylic acid

1)

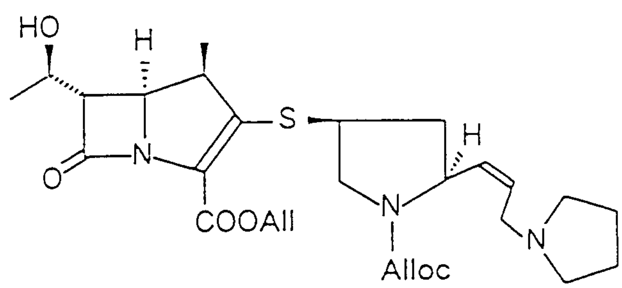

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (900 mg, 1.8 mmol) and (2S,4S)-N-allyloxycarbonyl-4-mercapto-2-[(Z)-3-(1-pyrrolidinyl)-1-propenyl]pyrrolidine trifluoroacetate (513 mg, 1.26 mmol, compound of Reference Example 5) to obtain allyl (1R,5S,6S)-2-[-(2S,4S)-N-allyloxycarbonyl-2-[3-(1-pyrrolidinyl)-1-propenyl]pyrrolidin-4-ylthio]-6-[(R)-1-hydroxyethyl]-1-methyl-1-carbapen-2-em-3-carboxylate (253 mg, yield: 25.8%).
IR(KBr)cm$^{-1}$: 1770, 1700, 1400, 1200
NMR($CDCl_3$) δ:
1.24(3H,d,J = 8Hz),1.32(3H,d,J = 7Hz),1.66-2.00(5H,m), 4.44-4.90(5H,m),5.10-5.55(4H,m),5.65(2H,m),5.94-(2H,m)

2)

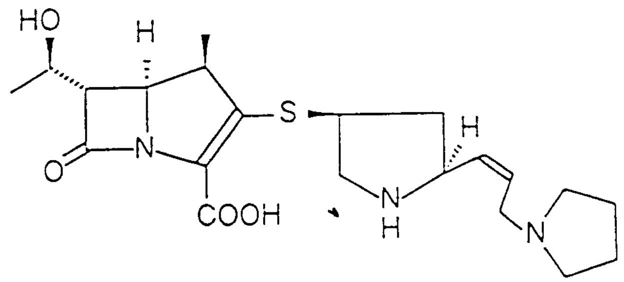

The same procedure as in Example 1-2 was carried out by using the compound (253 mg, 0.466 mmol) obtained by the above reaction, to obtain the above-identified compound (50 mg, yield: 25.5%).
IR(KBr)cm$^{-1}$: 1760, 1600, 1390
NMR($D_2O$) δ:
1.18(3H,d,J = 8Hz),1.26(3H,d,J = 7Hz),1.42(1H,m), 2.02(4H,m),2.54(1H,m),3.00(1H,dd,J = 12,4Hz), 3.14-3.48-(7H,m),3.82(3H,m),4.00(1H,q,J = 8Hz), 4.18(2H,m),5.68(1H,m),5.94(1H,t,J = 10Hz)

EXAMPLE 6

(1R,5S,6S)-6-[(R)-1-hydroxyethyl]-1-methyl-2-[(2S,4S)-2-[(E)-3-(N-methylamino)-1-propenyl]pyrrolidin-4-ylthio]-1-carbapen-2-em-3-carboxylic acid hydrochloride

1)

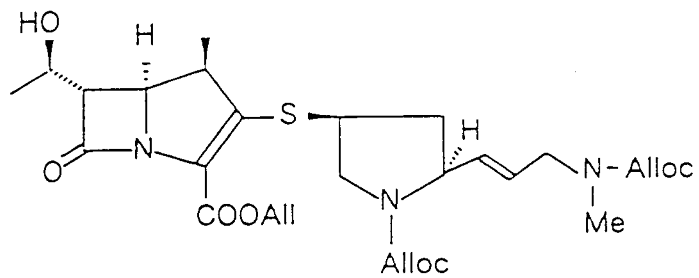

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (1.5 g, 3.0 mmol) and (2S,4S)-N-allyloxycarbonyl-2-[(E)-3-(N-allyloxycarbonyl-N-methylamino)-1-propenyl]-4-mercaptopyrrolidine (850 mg, 2.51 mmol), compound of Reference Example 6) to obtain allyl (1R,5S,6S)-2-[(2S,4S)-N-allyloxycarbonyl-2-[(E)-3-(N-allyloxycarbonyl-N-methylamino)-1-propenyl]pyrrolidin-4-ylthio]-6-[(R)-1-hydroxyethyl]-1-methyl-1-carbapen-2-em-3-carboxylate (627 mg, yield: 35.4%).
IR(KBr)cm$^{-1}$: 1770, 1700, 1400, 1200
NMR($CDCl_3$) δ:
1.26(3H,d,J = 8Hz),1.36(3H,d,J = 7Hz),1.76(1H,m), 2.60(1H,m),2.86(3H,s),4.50-4.95(6H,m), 5.15-5.55(6H,m)-,5.60(2H,m),5.95(2H,m)

2)

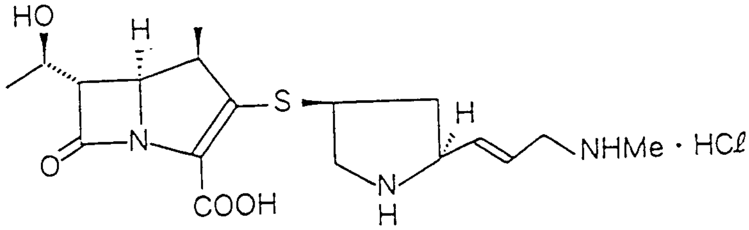

To a methylene chloride solution (22.5 ml) of the compound (592 mg, 1.0 mmol) obtained by the above reaction, water (91 μl), bis(triphenylphosphine) palladium(II) chloride (35.2 mg, 0.05 mmol) and tributyltin hydride (1.03 ml, 3.83 mmol) were added in a nitrogen stream under cooling with ice. This solution was

stirred at the same temperature for 20 minutes and at room temperature for 20 minutes. The reaction mixture was extracted with water (150 ml). The aqueous layer was washed twice with chloroform, and insoluble matters were filtered off. The filtrate was concentrated under reduced pressure to a volume of 30 ml and then subjected to reversed phase column chromatography (YMC-GEL™ ODS-AQ 120-S50, 50 ml, a 20% methanol aqueous solution). The fraction containing the desired product was collected and adjusted to pH 6.5 with 1N hydrochloric acid, and then it was concentrated and freeze-dried to obtain the above-identified compound (190 mg, yield: 45.3%).
IR(KBr)cm$^{-1}$: 1750, 1700, 1390
NMR($D_2O$) δ:
1.22(3H,d,J = 8Hz),1.30(3H,d,J = 7Hz),1.89(1H,m), 2.74(3H,s),2.82(1H,m),3.28-3.52(3H,m), 3.64-3.80(3H,m)-,4.08(1H,m),4.18-4.44(3H,m), 6.04(1H,dt,J = 15,6Hz),6.19(1H,dd,J = 15,8Hz)

EXAMPLE 7

(1R,5S,6S)-2-[(2S,4S)-2-[(Z)-3-amino-2-methyl-1-propenyl]pyrrolidin-4-ylthio]-6-[(R)-1-hydroxyethyl]-1-methyl-1-carbapen-2-em-3-carboxylic acid hydrochloride

1)

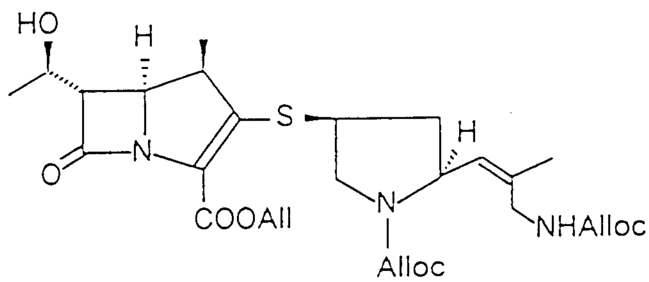

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (600 mg, 1.20 mmol) and (2S,4S)-N-allyloxycarbonyl-2-[(Z)-3-allyloxycarbonylamino-2-methyl-1-propenyl]-4-mercaptopyrrolidine (326 mg, 0.96 mmol, compound of Reference Example 7) to obtain allyl (1R,5S,6S)-2-[(2S,4S)-N-allyloxycarbonyl-2-[(Z)-3-allyloxycarbonylamino-2-methyl-1-propenyl]pyrrolidin-4-ylthio)-6-[(R)-1-hydroxyethyl]-1-methyl-1-carbapen-2-em-3-carboxylate (260 mg, yield: 36%).
IR(KBr)cm$^{-1}$: 1770, 1700, 1410
NMR($CDCl_3$) δ:
1.27(3H,d,J = 8Hz),1.35(3H,d,J = 7Hz),1.56-1.82(4H,m), 2.60(1H,m),3.16-3.47(3H,m),3.60(1H,m), 4.44-4.90-(7H,m),5.10-5.54(7H,m),5.88-6.10(3H,m)

2)

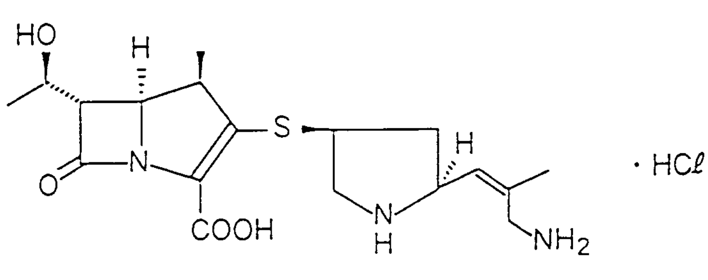

The same procedure as in Example 6-2 was carried out by using the compound (255 mg, 0.42 mmol) obtained by the above reaction, to obtain the above-identified compound (60 mg, yield: 33.8%).
IR(KBr)cm$^{-1}$: 1760, 1700, 1390
NMR($D_2O$) δ:
1.22(3H,d,J = 8Hz),1.29(3H,d,J = 7Hz),1.80(1H,m), 1.92(3H,s),2.78(1H,m),3.27-3.52(3H,m), 3.58-3.86(3H,m)-,4.05(1H,m),4.25(2H,m),4.56(1H,m), 5.76(1H,d,J = 8Hz)

EXAMPLE 8

(1R,5S,6S)-2-[(2S,4S)-2-[(E)-3-amino-2-methyl-1-propenyl]pyrrolidin-4-ylthio]-6-[(R)-1-hydroxyethyl]-1-methyl-1-carbapen-2-em-3-carboxylic acid hydrochloride

1)

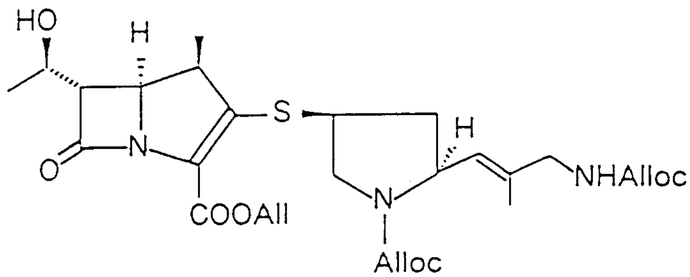

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (890 mg, 1.78 mmol) and (2S,4S)-N-allyloxycarbonyl-2-[(E)-3-allyloxycarbonylamino-2-methyl-1-propenyl]-4-mercaptopyrrolidine (463 mg, 1.32 mmol, compoud of Reference Example 8) to obtain allyl (1R,5S,6S)-2-[(2S,4S)-N-allyloxycarbonyl-2-[(E)-3-allyloxycarbonylamino-2-methyl-1-propenyl]pyrrolidin-4-ylthio]-6-[(R)-1-hydroxyethyl]-1-methyl-1-carbapen-2-em-3-carboxylate (366 mg, yield: 34.1%).
IR(KBr)$cm^{-1}$: 1770, 1710, 1540, 1410
NMR($CDCl_3$) $\delta$:
1.27(3H,d,J = 8Hz),1.35(3H,d,J = 7Hz),1.52-1.82(4H,m), 2.58(1H,m),3.20-3.45(3H,m),3.62(1H,m),3.74(2H,m), 4.45-4.98(7H,m),5.12-5.55(7H,m),5.78-6.10(3H,m)

2)

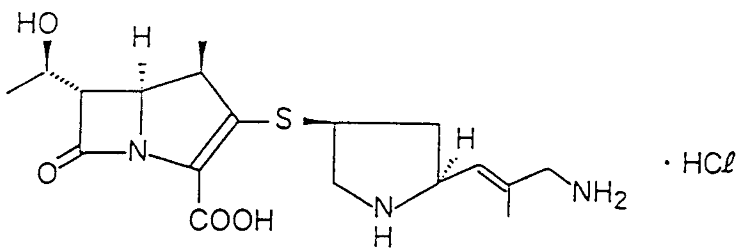

The same procedure as in Example 6-2 was carried out by using the compound (360 mg, 0.60 mmol) obtained by the above reaction, to obtain the above-identified compound (73 mg, yield: 29.2%).
IR(KBr)$cm^{-1}$: 1750, 1600, 1390
NMR($D_2O$) $\delta$:
1.24(3H,d,J = 8Hz),1.30(3H,d,J = 7Hz),1.70-1.93(4H,m), 2.82(1H,m),3.30-3.54(3H,m),3.58-3.80(3H,m), 4.06-(1H,m),4.27(2H,m),5.70(1H,d,J = 8Hz)

EXMAPLE 9

(1R,5S,6S)-6-[(R)-1-hydroxyethyl]-2-[(2S,4S)-2-[(Z)-3-(N-methylamino)-2-methyl-1-propenyl]pyrrolidin-4-ylthio]-1-methyl-1-carbapen-2-em-3-carboxylic acid hydrochloride

1)

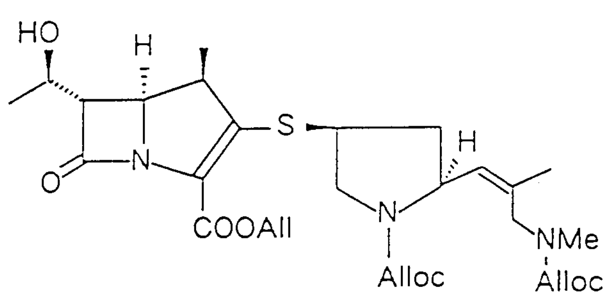

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (550 mg, 1.10 mmol) and (2S,4S)-N-allyloxycarbonyl-2-[(Z)-3-(N-allyloxycarbonyl-N-methylamino)-2-methyl-1-propenyl]-4-mercaptopyrrolidine (308 mg, 0.87 mmol, compound of Reference Example 9) to obtain allyl (1R,5S,6S)-2-[-(2S,4S)-N-allyloxycarbonyl-2-[(Z)-3-(N-allyloxycarbonyl-N-methylamino)-2-methyl-1-propenyl]pyrrolidin-4-ylthio]-6-[(R)-1-hydroxyethyl]-1-methyl-1-carbapen-2-em-3-carboxylate (194 mg, yield: 29.3%).
IR(KBr)$cm^{-1}$: 1780, 1700, 1400, 1280, 1140
NMR($CDCl_3$) $\delta$:
1.25(3H,d,J = 8Hz),1.34(3H,d,J = 7Hz),1.52-1.80(4H,m), 2.56(1H,m),2.84(3H,s),3.14-3.46(3H,m),3.58(1H,m), 4.40-4.90(7H,m),5.10-5.53(7H,m),5.72-6.12(3H,m)

2)

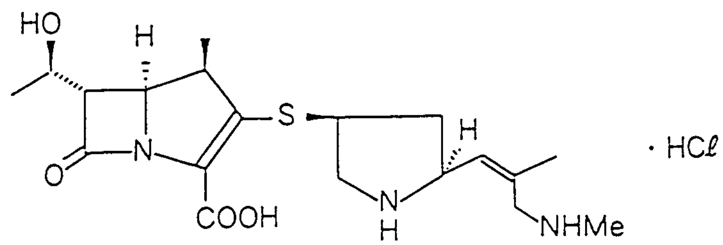

The same procedure as in Example 6-2 was carried out by using the compound (192 mg, 0.32 mmol) obtained by the above reaction, to obtain the above-identified compound (55 mg, yield: 39.9%).

EXMAPLE 10

<u>(1R,5S,6S)-6-[(R)-1-hydroxyethyl]-2-[(2S,4S)-2-[(E)-3-(N-methylamino)-2-methyl-1-propenyl]pyrrolidin-4-ylthio]-1-methyl-1-carbapen-2-em-3-carboxylic acid hydrochloride</u>

1)

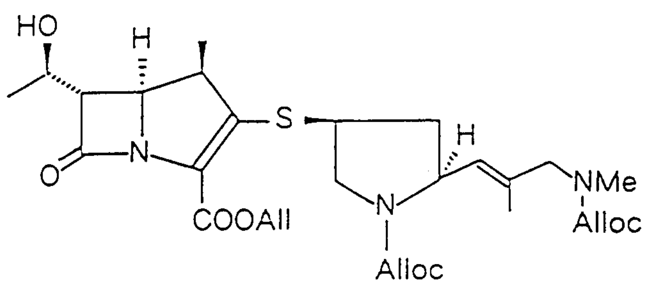

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (500 mg, 1.0 mmol) and (2S,4S)-N-allyloxycarbonyl-2-[(E)-3-(N-allyloxycarbonyl-N-methylamino)-2-methyl-1-propenyl]-4-mercaptopyrrolidine (290 mg, 0.84 mmol, compound of Reference Example 10) to obtain allyl (1R,5S,6S)-2-[(2S,4S)-N-allyloxycarbonyl-2-[(E)-3-(N-allyloxycarbonyl-N-methylamino)-2-methyl-1-propenyl]pyrrolidin-4-ylthio]-6-[(R)-1-hydroxyethyl]-1-methyl-1-carbapen-2-em-3-carboxylate (304 mg, yield: 50.5%).
IR(KBr)$cm^{-1}$: 1780, 1700, 1400
NMR($CDCl_3$) $\delta$:
1.26(3H,d,J = 8Hz),1.34(3H,d,J = 7Hz),1.53-1.80(4H,m), 2.60(1H,m),2.80(3H,s),3.18-3.46(3H,m),3.60(1H,m), 3.85(2H,m),4.44-4.90(7H,m),5.08-5.53(7H,m), 5.72-6.10(3H,m)

2)

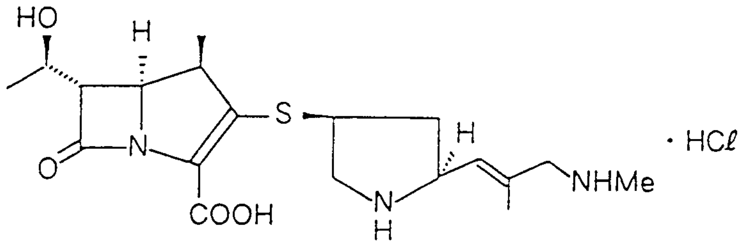

The same procedure as in Example 6-2 was carried out by using the compound (302 mg, 0.50 mmol) obtained by the above reaction, to obtain the above-identified compound (78 mg, yield: 36.0%).
IR(KBr)cm$^{-1}$: 1750, 1690, 1390
NMR($CDCl_3$) δ:
1.25(3H,d,J = 8Hz),1.32(3H,d,J = 7Hz),1.74-1.96(4H,m), 2.86(1H,m),3.34-3.46(3H,m),3.64-3.82(3H,m), 4.10-(1H,m),4.29(2H,m),4.64(1H,m),5.83(1H,d,J = 8Hz)

EXAMPLE 11

(1R,5S,6S)-2-[(2S,4S)-2-[[1-(aminomethyl)vinyl]pyrrolidin-4-ylthio]]-6-[(R)-1-hydroxyethyl]-1-methyl-1-carbapen-2-em-3-carboxylic acid

1)

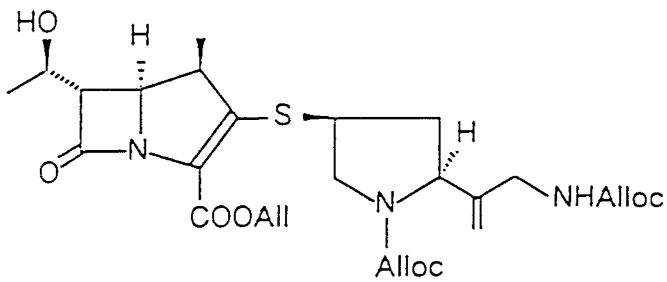

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (1.10 g, 2.2 mmol) and (2S,4S)-N-allyloxycarbonyl-2-[1-[(N-allyloxycarbonylamino)methyl]vinyl]-4-mercaptopyrrolidine (530 mg, 1.62 mmol, compound of Reference Example 11) to obtain allyl (1R,5S,6S)-2-[(2S,4S)-N-allyloxycarbonyl-2-[1-[(N-allyloxycarbonylamino)methyl]vinyl]pyrrolidin-4-ylthio]-6-[(R)-1-hydroxyethyl]-1-methyl-1-carbapen-2-em-3-carboxylate (643 mg, yield: 68%).
NMR($CDCl_3$) δ:
1.26(3H,d,J = 8Hz),1.35(3H,d,J = 7Hz),4.49-4.93(6H,m), 4.97-5.53(8H,m),5.75-6.11(3H,m)

2)

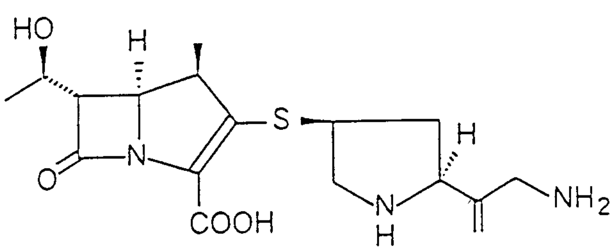

The same procedure as in Example 6-2 was carried out by using the compound (332 mg, 0.58 mmol) obtained by the above reaction, to obtain the above-identified compound (17 mg, yield: 8%).
IR(KBr)cm$^{-1}$: 1750
NMR($D_2O$) δ:

1.21(3H,d,J = 8Hz),1.26(3H,d,J = 7Hz),5.50(1H,br s), 5.63(1H,br s)

EXAMPLE 12

(1R,5S,6S)-6-[(R)-1-hydroxyethyl]-1-methyl-2-[(2S,4S)-2-[1-[(N-methylamino)methyl]vinyl]pyrrolidin-4-ylthio]-1-carbapen-2-em-3-carboxylic acid hydrochloride

1)

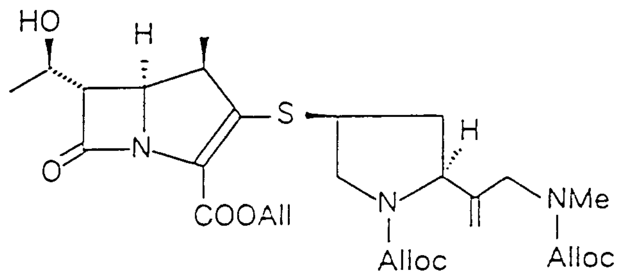

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (4.3 g, 8.6 mmol) and (2S,4S)-N-allyloxycarbonyl-2-[1-[(N-allyloxycarbonyl-N-methylamino)methyl)vinyl]-4-mercaptopyrrolidine (2.87 g, 8.4 mmol, compoud of Reference Example 12) to obtain allyl (1R,5S,6S)-2-[(2S,4S)-N-allyloxycarbonyl-2-[1-[(N-allyloxycarbonyl-N-methylamino)methyl]vinyl]pyrrolidin-4-ylthio]-6-[(R)-1-hydroxyethyl]-1-methyl-1-carbapen-2-em-3-carboxylate (3.53 g, yield: 69%).
NMR($CDCl_3$) $\delta$:
1.24(3H,d,J = 7.3Hz),1.35(3H,d,J = 6.3Hz),1.73(3H,m), 2.12(1H,m),2.47(1H,m),2.86(3H,br s),3.20-3.40(3H,m), 4.50-4.98(6H,m),5.12-5.48(4H,m),5.80-6.01(2H,m)

2)

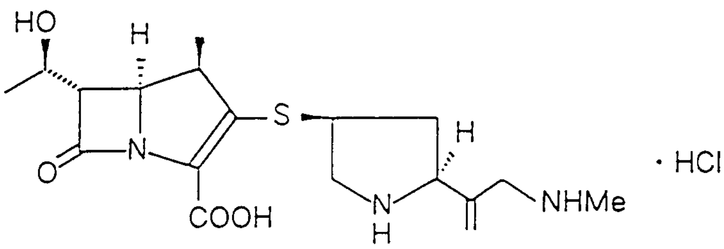

The same procedure as in Example 6-2 was carried out by using the compound (1.39 g, 2.36 mmol) obtained by the above reaction, to obtain the above-identified compound (267 mg, yield: 27%).
IR(KBr)$cm^{-1}$: 1750
NMR($D_2O$) $\delta$:
1.06(3H,d,J = 7.3Hz),1.13(3H,d,J = 6.3Hz),1.63(1H,m), 2.61(3H,s),3.09-3.48(4H,m),3.50-3.75(2H,m), 3.82-(1H,m),3.94(1H,m),4.09(2H,m),5.32(1H,s), 5.47(1H,s)

EXAMPLE 13

(1R,5S,6S)-6-[(R)-1-hydroxyethyl]-1-methyl-2-[(2S,4S)-2-[2-[(N-methylamino)methyl]allyl]pyrrolidin-4-ylthio]-1-carbapen-2-em-3-carboxylic acid hydrochloride

1)

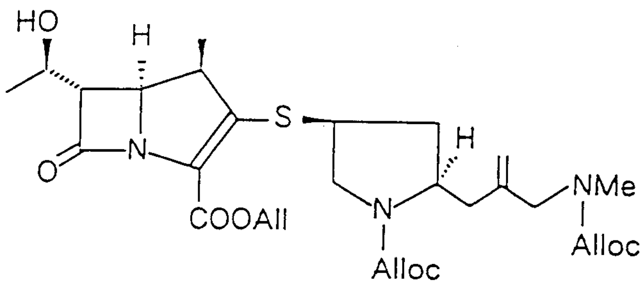

The same procedure as in Example 1-1 was carried out by using allyl (1R,5S,6S)-2-diphenoxyphosphoryloxy-6-[(R)-1-hydroxyethyl]-1-methyl-1-carbapen-2-em-3-carboxylate (1.71 g, 3.43 mmol) and (2S,4S)-N-allyloxycarbonyl-2-[[2-(N-allyloxycarbonyl-N-methylamino)methyl]allyl)-4-mercaptopyrrolidine (1.22 g, 3.43 mmol, compound of Reference Example 13) to obtain allyl (1R,5S,6S)-2-[(2S,4S)-N-allyloxycarbonyl-2-[2-(N-allyloxycarbonyl-N-methylamino)methyl]allylpyrrolidin-4-ylthio]-6-[(R)-1-hydroxyethyl]-1-methyl-1-carbapen-2-em-3-carboxylate (1.36 g, yield: 65%).
NMR($CDCl_3$) δ:
1.24(3H,d,J = 7.3Hz),1.35(3H,d,J = 6.3Hz),1.73(3H,m), 2.86(3H,br s),3.20-3.41(3H,m),4.50-4.98(6H,m), 5.12-5.50(4H,m),5.80-6.01(2H,m)

2)

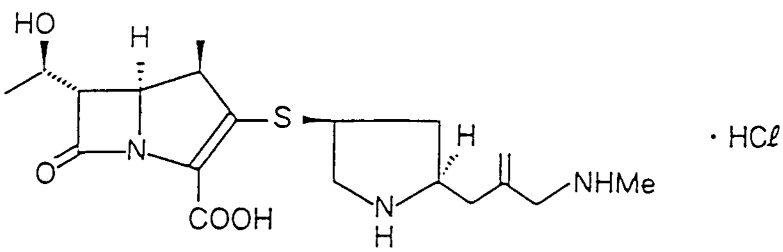

The same procedure as in Example 6-2 was carried out by using the compound (1.36 g, 2.25 mmol) obtained by the above reaction, to obtain the above-identified compound (213 mg, yield: 21%).
IR(KBr)$cm^{-1}$: 1750
NMR($D_2O$) δ:
1.17(3H,d,J = 7.3Hz),1.24(3H,d,J = 8.4Hz),1.70(1H,m), 2.63(2H,m),2.70(3H,s),3.25-3.60(3H,m), 3.60-3.80-(4H,m),4.20(2H,m),5.32(1H,s),5.36(1H,s)

REFERENCE EXAMPLE 1

(2S,4S)-N-allyloxycarbonyl-2-[(E)-3-allyloxycarbonylamino-1-propenyl]-4-mercaptopyrrolidine

1)

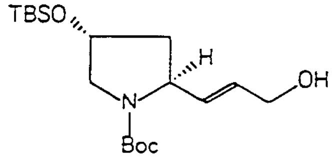

To a methylene chloride solution (70 ml) of ethyl 3-[(2S,4R)-N-tert-butoxycarbonyl-4-tert-butyldimethylsiloxypyrrolidin-2-yl]acrylate (3.55 g, 8.9 mmol, compound of Reference Example 1-4 of Japanese Patent Application No. 87628/1991), a toluene solution (19.2 ml) of 1.0 M diisobutylaluminum

hydride was dropwise added in a nitrogen stream at -78°C, and the mixture was stirred at the same temperature for two hours. To the reaction solution, acetic acid (2.55 ml, 44.5 mmol) was added. Then, the mixture was extracted with methylene chloride (100 ml). The organic layer was washed sequentially with a 1N aqueous sodium hydroxide solution, water and a saturated sodium chloride aqueous solution and dried over anhydrous sodium sulfate and then concentrated under reduced pressure. The residue was subjected to silica gel column chromatography [Wakogel™ C-300, hexane-ethyl acetate (3:1)] to obtain (2S,4R)-N-tert-butoxycarbonyl-4-tert-butyldimethylsiloxy-2-[(E)-3-hydroxy-1-propenyl]pyrrolidine (918 mg, yield: 28.9%).

NMR($CDCl_3$) δ:

0.04(6H,s),0.86(9H,s),1.42(9H,s),1.78(1H,m), 2.01(1H,m),3.40(2H,m),4.12(2H,d,J=5Hz), 4.34(2H,m),5.58-(1H,dd,J=16,6Hz),5.74(1H,dt,J=16,5Hz)

2)

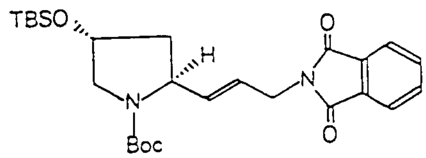

To a tetrahydrofuran solution (40 ml) of the compound (918 mg, 2.57 mmol) obtained by the above reaction, triphenylphosphine (1.01 g, 3.85 mmol) and phthalimide (662 mg, 4.50 mmol) were added. Then, diethyl azodicarboxylate (0.607 ml, 3.85 mmol) was dropwise added thereto in a nitrogen stream under cooling with ice, and the mixture was stirred at the same temperature for 2.5 hours. The reaction solution was extracted with ethyl acetate (70 ml). The organic layer was washed with water and a saturated sodium chloride aqueous solution, dried over anhydrous sodium sulfate and then concentrated under reduced pressure. The residue was subjected to silica gel column chromatography [Wakogel™ C-300, hexane-ethyl acetate (10:1)] to obtain (2S,4R)-N-tert-butoxycarbonyl-4-tert-butyldimethylsiloxy-2-[(E)-3-phthalimido-1-propenyl]pyrrolidine (961 mg, yield: 76.9%).

NMR($CDCl_3$) δ:

0.03(6H,s),0.85(9H,s),1.37(9H,br s),1.78(1H,m), 1.98(1H,m),3.42(2H,m),4.29(4H,m),5.64(2H,m), 7.74(2H,m)-,7.88(2H,m)

3)

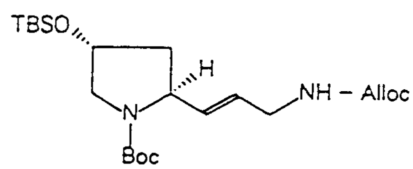

To an ethanol solution (15 ml) of the compound (960 mg, 1.98 mmol) obtained by the above reaction, hydrazine monohydrate (0.288 ml, 5.94 mmol) was added at room temperature, and the mixture was stirred overnight at the same temperature. Insoluble matters were filtered off from the reaction mixture. Then, the filtrate was concentrated under reduced pressure, and the residue was extracted with ethyl acetate (70 ml). The organic layer was washed sequentially with 3N aqueous ammonia, water and a saturated sodium chloride aqueous solution, then dried over anhydrous sodium sulfate and concentrated under reduced pressure. The residue was dissolved in tetrahydrofuran (15 ml), and triethylamine (0.35 ml, 2.52 mmol) and allyl chloroformate (0.24 ml, 2.26 mmol) were added thereto under cooling with ice, and the mixture was stirred at the same temperature for one hour. The reaction mixture was extracted with ethyl acetate (50 ml). The organic layer was washed with water and a saturated sodium chloride aqueous solution, dried over anhydrous sodium sulfate and then concentrated under reduced pressure. The residue was subjected to silica gel column chromatography [Wakogel™ C-300, hexane-ethyl acetate (5:1)] to obtain (2S,4R)-2-[(E)-3-allyloxycarbonylamino-1-propenyl]-N-allyloxycarbonyl-4-tert-butyldimethylsiloxypyrrolidine (680 mg, yield: 75.5%).

NMR($CDCl_3$) δ:
0.06(6H,s),0.87(9H,s),1.44(9H,s),1.77(1H,m), 2.02(1H,m),3.42(2H,m),3.82(2H,m),4.33(2H,m), 4.58(2H,m),5.17-5.39(2H,m),5.57(2H,m),5.95(1H,m)

4)

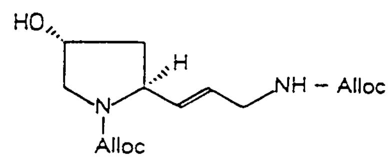

A methanol solution (10 ml) of 7.5N hydrogen chloride was added to the compound (680 mg, 1.49 mmol) obtained by the above reaction, and the mixture was stirred overnight at room temperature. The reaction solution was concentrated under reduced pressure, and the residue was dissolved in a mixed solution of dioxane (10 ml) and water (5 ml). While adjusting the pH to 9 with a 1N sodium hydroxide aqueous solution under cooling with ice, allyl chloroformate (0.24 ml, 2.26 mmol) was dropwise added thereto. The reaction solution was stirred at the same temperature for further 30 minutes and then concentrated under reduced pressure. The residue was extracted with ethyl acetate (70 ml). The organic layer was washed with water and a saturated sodium chloride aqueous solution, dried over anhydrous sodium sulfate and then concentrated under reduced pressure. The residue was subjected to silica gel column chromatography (Wakogel™ C-300, 1.5% methanol-chloroform) to obtain (2S,4R)-N-allyloxycarbonyl-2-[(E)-3-allyloxycarbonylamino-1-propenyl]-4-hydroxypyrrolidine (440 mg, yield: 90.5%).
NMR($CDCl_3$) δ:
1.84(1H,m),2.17(1H,m),3.55(2H,m),3.79(2H,m), 4.32-4.64(6H,m),5.12-5.38(4H,m),5.57(2H,m), 5.90-6.04-(2H,m)

5)

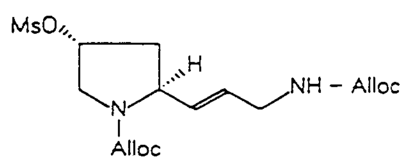

To a tetrahydrofuran solution (10 ml) of the compound (440 mg, 1.35 mmol) obtained by the above reaction, triethylamine (0.207 ml, 1.49 mmol) and methanesulfonyl chloride (0.112 ml, 1.45 mmol) were dropwise added in a nitrogen stream under cooling with ice. The reaction mixture was stirred at the same temperature for one hour and then extracted with ethyl acetate (70 ml). The organic layer was washed with water and a saturated sodium chloride aqueous solution, dried over anhydrous sodium sulfate and concentrated under reduced pressure to obtain (2S,4R)-N-allyloxycarbonyl-2-[(E)-3-allyloxycarbonylamino-1-propenyl]-4-methanesulfonyloxypyrrolidine (550 mg, yield: 100%).
NMR($CDCl_3$) δ:
2.05(1H,m),2.48(1H,m),3.07(3H,s),3.66(1H,dd, J=12,4Hz),3.82(2H,m),3.96(1H,br d,J=12Hz), 4.44-4.72-(5H,m),4.98(1H,m),5.16-5.42(4H,m), 5.62(2H,m),5.84-6.06(2H,m)

6)

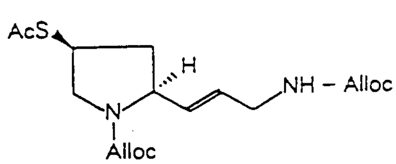

To an N,N-dimethylformamide solution (20 ml) of the compound (550mg, 1.36 mmol) obtained by the

above reaction, potassium thioacetate (315 mg, 2.76 mmol) and sodium iodide (228 mg, 1.52 mmol) were added. The mixture was stirred overnight in a nitrogen stream at a temperature of from 60 to 70 ° C. The reaction mixture was extracted with ethyl acetate (70 ml). The organic layer was washed three times with water and once with a saturated sodium chloride aqueous solution, dried over anhydrous sodium sulfate and then concentrated under reduced pressure. The residue was subjected to silica gel column chromatography [Wakogel™ C-300, hexane-ethyl acetate (3:1)] to obtain (2S,4S)-N-allyloxycarbonyl-2-[(E)-3-allyloxycarbon ylamino-1-propenyl]-4-acetylthiopyrrolidine (397 mg, yield: 74.8%).
NMR($CDCl_3$) δ:
1.76(1H,m),2.35(3H,s),2.48(1H,m),3.26(1H,m), 3.82(2H,m) ,3.88-4.14(2H,m),4.42(1H,m), 4.66(4H,m),5.16-5.40-(4H,m),5.63(2H,m),5.80-6.04(2H,m)

7)

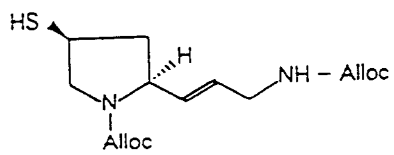

To a methanol solution (12 ml) of the compound (397 mg, 1.03 mmol) obtained by the above reaction, a 1N sodium hydroxide aqueous solution (1.09 ml) was dropwise added in a nitrogen stream under cooling with ice, and the mixture was stirred at the same temperature for 15 minutes. To the reaction solution, 1N hydrochloric acid (1.1 ml) was added, and the mixture was concentrated under reduced pressure. The residue was extracted with ethyl acetate (70 ml). The organic layer was washed with water and a saturated sodium chloride aqueous solution, dried over anhydrous sodium sulfate and then concentrated under reduced pressure to obtain the above-identified compound (360 mg, yield: 100%).
NMR($CDCl_3$) δ:
1.56-1.78(2H,m),2.58(1H,m),3.23(2H,m),3.82(2H,m), 4.06(1H,m),4.35(1H,m),4.60(4H,m),5.17-5.42(4H,m), 5.66-(2H,m),5.82-6.06(2H,m)

REFERENCE EXAMPLE 2

(2S,4S)-N-allyloxycarbonyl-2-[(Z)-3-(allyloxycarbonylamino)-1-propenyl]-4-mercaptopyrrolidine

1)

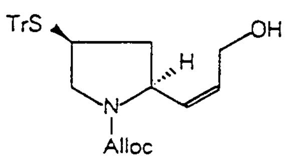

To a toluene solution (80 ml) of methyl (Z)-3-[(2S,4S)-N-allyloxycarbonyl-4-tritylthiopyrrolidin-2-yl]-acrylate (4.0 g, 7.83 mmol, compound of Reference Example 3-2 of Japanese Patent Application No. 205984/1990), a toluene solution (19.6 ml) of diisobutylaluminum hydride was dropwise added in a nitrogen stream at -78 ° C, and the mixture was stirred at the same temperature for one hour. Then, temperature was raised to -10 ° C over a period of 1.5 hours. To the reaction solution, acetic acid (3.5 ml, 61.1 mmol) was dropwise added. Then, the mixture was extracted with ethyl acetate (150 ml). The organic layer was washed sequentially with a 1N sodium hydroxide aqueous solution, water and a saturated sodium chloride aqueous solution, dried over anhydrous sodium sulfate and concentrated under reduced pressure. The residue was subjected to silica gel column chromatography [Wakogel™ C-300, hexane-ethyl acetate (3:1)] to obtain (2S,4S)-N-allyloxycarbonyl-2-[(Z)-3-hydroxy-1-propenyl]-4-tritylthiopyrrolidine (1.90 g, yield: 50.1%).
NMR($CDCl_3$) δ:

1.53(1H,m),2.17(1H,m),2.64-3.00(3H,m), 3.68-3.98(2H,m),4.24-4.67(4H,m),5.11-5.40(3H,m), 5.73-5.97(2H,m)-,7.16-7.54(15H,m)

2)

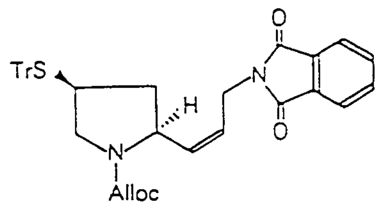

The same procedure as in Reference Example 1-2 was carried out by using the compound (1.90 g, 3.93 mmol) obtained by the above reaction, to obtain (2S,4S)-N-allyloxycarbonyl-2-[(Z)-3-phthalimido-1-propenyl]-4-tritylthiopyrrolidine (2.39 g, yield: 99.4%).
NMR($CDCl_3$) $\delta$:
1.53(1H,m),2.26(1H,m),2.87(2H,m),5.00-5.66(4H,m), 5.84(1H,m),7.16-7.56(15H,m),7.75(2H,m),7.87(2H,m)

3)

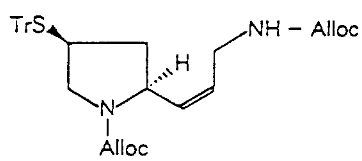

The same procedure as in Reference Example 1-3 was carried out by using the compound (2.39 g, 3.90 mmol) obtained by the above reaction, to obtain (2S,4S)-N-allyloxycarbonyl-2-[(Z)-3-(allyloxycarbonylamino)-1-propenyl]-4-tritylthiopyrrolidine (1.93 g, yield: 87.3%).
NMR($CDCl_3$) $\delta$:
1.54(1H,m),2.14(1H,m),2.64-3.02(3H,m), 3.64-4.02(3H,m),4.22-4.63(5H,m),5.10-6.02(8H,m), 7.16-7.56(15H,m)

4)

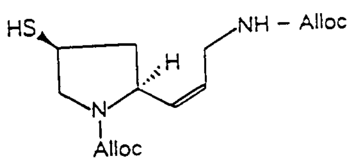

To a methylene chloride solution (2.5 ml) of the compound (902 mg, 1.59 mmol) obtained by the above reaction, trifluoroacetic acid (2.5 ml) and triethylsilane (0.268 ml, 1.68 mmol) were added in a nitrogen stream under cooling with ice, and the mixture was stirred at the same temperature for one hour. The reaction solution was concentrated under reduced pressure, and the residue was extracted with ethyl acetate (70 ml). The organic layer was washed twice with 1M phosphate buffer (pH 5.5) and once with a saturated sodium chloride aqueous solution, dried over anhydrous sodium sulfate and then concentrated under reduced pressure. The residue was subjected to silica gel column chromatography [Wakogel™ C-300, hexane-ethyl acetate (4:1)] to obtain the above-identified compound (460 mg, yield: 89%).
NMR($CDCl_3$) $\delta$:
1.50-1.78(2H,m),2.62(1H,m),3.10-3.42(2H,m), 3.74-4.20(4H,m),4.58(4H,m),5.16-6.08(8H,m)

REFERENCE EXAMPLE 3

(2S,4S)-N-allyloxycarbonyl-2-[(Z)-3-(N-allyloxycarbonyl-N-methylamino)-1-propenyl]-4-mercaptopyrrolidine

1)

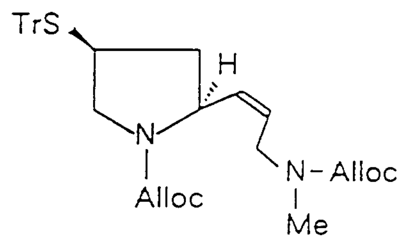

To an N,N-dimethylformamide solution (15 ml) of (2S,4S)-N-allyloxycarbonyl-2-[(Z)-3-(allyloxycarbonylamino)-1-propenyl]-4-tritylthiopyrrolidine (1.02 g, 1.8 mmol), 60% sodium hydride (110 mg, 2.75 mmol) was added in a nitrogen stream under cooling with ice, and the mixture was stirred at the same temperature for 40 minutes. Then, methyl iodide (0.45 ml, 7.2 mmol) was dropwise added thereto, and the mixture was stirred overnight at room temperature. The reaction mixture was extracted with ethyl acetate (70 ml). The organic layer was washed three times with water and once with a saturated sodium chloride aqueous solution, dried over anhydrous magnesium sulfate and then concentrated under reduced pressure. The residue was subjected to silica gel column chromatography [Wakogel™ C-300, hexane-ethyl acetate (3:1)] to obtain (2S,4S)-N-allyloxycarbonyl-2-[(Z)-3-(N-allyloxycarbonyl-N-methylamino)-1-propenyl]-4-tritylthiopyrrolidine (791 mg, yield: 75.4%).

NMR($CDCl_3$) $\delta$:
1.50(1H,m),2.64-3.04(6H,m),3.66-4.64(8H,m), 5.10-5.50(6H,m),5.90(2H,m),7.16-7.60(15H,m)

2)

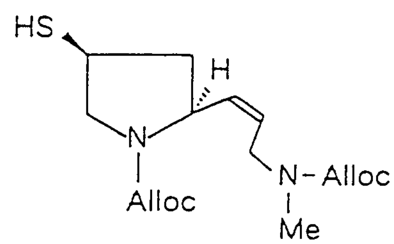

The same procedure as in Reference Example 2-4 was carried out by using the compound (791 mg, 1.36 mmol) obtained by the above reaction, to obtain the above-identified compound (416 mg, yield: 90.0%).

NMR($CDCl_3$) $\delta$:
1.62(1H,m),2.60(1H,m),2.93(3H,s),3.13-3.38(2H,m), 3.73-4.40(4H,m),4.54-4.76(4H,m),5.14-5.42(4H,m), 5.56-(2H,m),5.92(2H,m)

REFERENCE EXAMPLE 4

(2S,4S)-N-allyloxycarbonyl-2-[(Z)-3-(N,N-dimethylamino)-1-propenyl]-4-mercaptopyrrolidine trifluoroacetate

1)

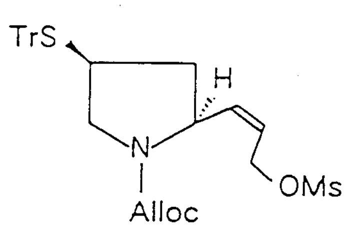

The same procedure as in Reference Example 1-5 was carried out by using (2S,4S)-N-allyloxycarbonyl-2-[(Z)-3-hydroxy-1-propenyl]-4-tritylthiopyrrolidine (3.6 g, 7.45 mmol) to obtain (2S,4S)-N-allyloxycarbonyl-2-[(Z)-3-methanesulfonyloxy-1-propenyl]-4-tritylthiopyrrolidine (4.07 g, yield: 97.3%).

NMR($CDCl_3$) $\delta$:
1.48(1H,m),2.68-3.06(6H,m),4.20-4.58(4H,m), 4.62-4.98(2H,m),5.12-5.22(2H,m),5.60(2H,m), 5.92(1H,m),7.16-7.54(15H,m)

2)

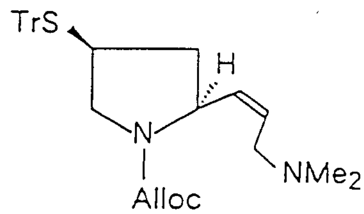

To an N,N-dimethylformamide solution (15 ml) of the compound (2.03 g, 3.62 mmol) obtained by the above reaction, dimethylamine hydrochloride (890 mg, 10.9 mmol) and sodium hydrogencarbonate (1.06 g, 12.6 mmol) were added, and the mixture was stirred at room temperature for 5 hours. The reaction mixture was concentrated under reduced pressure, and the residue was extracted with ethyl acetate (70 ml). The organic layer was washed sequentially with water and a saturated sodium chloride aqueous solution, dried over anhydrous magnesium sulfate and then concentrated under reduced pressure. The residue was subjected to silica gel column chromatography [Wakogel™ C-300, 3% methanol-chloroform] to obtain (2S,4S)-N-allyloxycarbonyl-2-[(Z)-3-(N,N-dimethylamino)-1-propenyl]-4-tritylthiopyrrolidine (1.24 g, yield: 67.2%).

NMR($CDCl_3$) $\delta$:
1.48(1H,m),1,80-2.40(6H,m),2.64-3.15(4H,m), 4.22-4.62(4H,m),5.06-5.32(2H,m),5.35-5.66(2H,m), 5.86(1H,m)-,7.18-7.57(15H,m)

3)

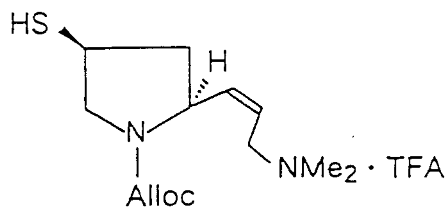

To a methylene chloride solution (3.5 ml) of the compound (1.23 g, 2.41 mmol) obtained by the above reaction, trifluoroacetic acid (3.5 ml) and triethylsilane (0.41 ml, 2.57 mmol) were added in a nitrogen stream under cooling with ice, and the mixture was stirred at the same temperature for 30 minutes. The reaction mixture was concentrated under reduced pressure, and the residue was subjected to silica gel column chromatography [Wakogel™ C-300, 3% methanol-chloroform] to obtain the above-identified compound (640 mg, yield: 69.5%).

NMR($CDCL_3$) $\delta$:
1.62(1H,m),2.62(1H,m),2.84(6H,m),3.12-3.42(3H,m), 3.74-4.22(3H,m),4.42-4.66(2H,m),5.16-5.40(2H,m), 5.55-6.10(3H,m)

REFERENCE EXAMPLE 5

(2S,4S)-N-allyloxycarbonyl-2-[(Z)-3-(1-pyrrolidinyl)-1-propenyl]-4-mercaptopyrrolidine trifluoroacetate

1)

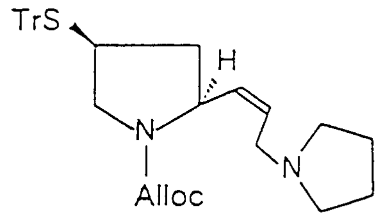

Pyrrolidine (0.91 ml, 10.9 mmol) was added to an N,N-dimethylformamide solution (15 ml) of (2S,4S)-N-allyloxycarbonyl-2-[(Z)-3-methanesulfonyloxy-1-propenyl]-4-tritylthiopyrrolidine (2.03 g, 3.62 mmol), and the mixture was stirred at room temperature for 5 hours. The reaction mixture was concentrated under reduced pressure, and the residue was extracted with ethyl acetate (70 ml). The organic layer was washed sequentially with water and a saturated sodium chloride aqueous solution, dried over anhydrous magnesium sulfate and concentrated under reduced pressure. The residue was subjected to silica gel column chromatography [Wakogel™ C-300, 3% methanol-chloroform] to obtain (2S,4S)-N-allyloxycarbonyl-2-[(Z)-3-(1-pyrrolidinyl)-1-propenyl]-4-tritylthiopyrrolidine (1.34 g, yield: 69.1%).
NMR($CDCl_3$) $\delta$:
1.46(1H,m),1.56-2.10(5H,m),2.18-2.60(4H,m), 2.65-3.40(5H,m),4.20-4.60(3H,m),5.06-5.42(3H,m), 5.58(1H,m)-,5.84(1H,m),7.16-7.60(15H,m)

2)

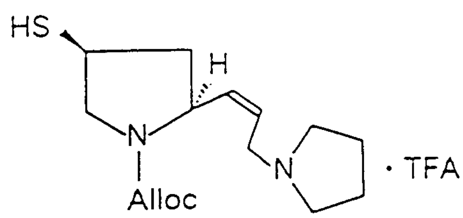

The same procedure as in Reference Example 4-3 was carried out by using the compound (1.34 g, 2.5 mmol) obtained by the above reaction, to obtain the above-identified compound (513 mg, yield: 50.3%).

REFERENCE EXAMPLE 6

(2S,4S)-N-allyloxycarbonyl-2-[(E)-3-(N-allyloxycarbonyl-N-methylamino)-1-propenyl]-4-mercaptopyrrolidine

1)

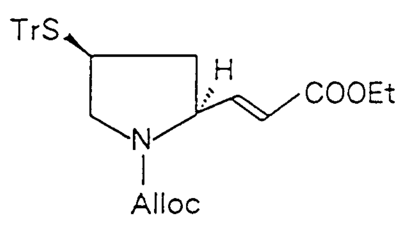

To a methylene chloride solution (150 ml) of oxalyl chloride (7.05 ml, 80.8 mmol), a methylene chloride solution (25 ml) of dimethyl sulfoxide (8.8 ml, 124 mmol) was dropwise added in a nitrogen stream at -78°C, and the reaction solution was stirred at the same temperature for 30 minutes. To this mixture, a methylene chloride solution (130 ml) of (2S,4S)-N-allyloxycarbonyl-2-hydroxymethyl-4-tritylthiopyrrolidine (27.1 g, 59.0 mmol, compound of Reference Example 3-1 of Japanese Patent Application No. 205984/1990) was dropwise added. This reaction mixture was stirred at the same temperature for 30 minutes, and then triethylamine (27.1 ml, 195 mmol) was added thereto. The mixture was further stirred for 30 minutes. This

reaction mixture was poured into methylene chloride (200 ml). The organic layer was washed sequentially with dilute hydrochloric acid, water and a saturated sodium chloride aqueous solution, then dried over anhydrous magnesium sulfate and concentrated under reduced pressure to obtain an oily residue of (2S,4S)-N-allyloxycarbonyl-2-formyl-4-tritylthiopyrrolidine.

Ethyl diethylphosphonoacetate (14.2 g, 63.3 ml) was added to a tetrahydrofuran suspension (260 ml) of 60% sodium hydride in a nitrogen stream under cooling with ice, and the mixture was stirred at the same temperature for 30 minutes. To this reaction solution, the tetrahydrofuran solution (100 ml) of the (2S,4S)-N-allyloxycarbonyl-2-formyl-4-tritylthiopyrrolidine obtained by the above reaction, was dropwise added, and the mixture was stirred at the same temperature for 30 minutes. The reaction solution was extracted with ethyl acetate (350 ml). The organic layer was washed sequentially with water and a saturated sodium chloride aqueous solution, dried over anhydrous magnesium sulfate and then concentrated under reduced pressure. The residue was subjected to silica gel column chromatography [Wakogel™ C-300, hexane-ethyl acetate (4:1)] to obtain ethyl 3-[(2S,4S)-N-allyloxycarbonyl-4-tritylthiopyrrolidin-2-yl]acrylate (28.31 g, yield: 96.1%).

NMR($CDCl_3$) $\delta$:

1.26 (3H,t,J = 8Hz),1.80-2.20(1H,m),2.66-3.50(3H,m), 4.18(3H,m),4.48(2H,m),5.05-5.34(2H,m), 5.64-6.00-(2H,m),6.76(1H,dd,J = 15,7Hz),7.14-7.58(15H,m)

2)

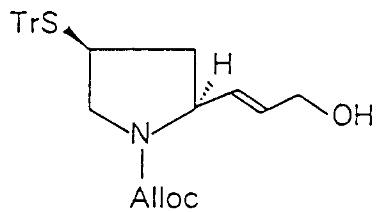

The same procedure as in Reference Example 2-1 was carried out by using the compound (28.31 g, 55.4 mmol) obtained by the above reaction, to obtain (2S,4S)-N-allyloxycarbonyl-2-[(E)-3-hydroxy-1-propenyl]-4-tritylthiopyrrolidine (3.4 g, yield: 12.7%).

NMR($CDCl_3$) $\delta$:

1.56(1H,m),2.10(1H,m),2.58-3.10(3H,m), 3.96-4.16(2H,m),4.34-4.60(2H,m),5.08-5.32(2H,m), 5.48-5.98(3H,m)-,7.14-7.56(15H,m)

3)

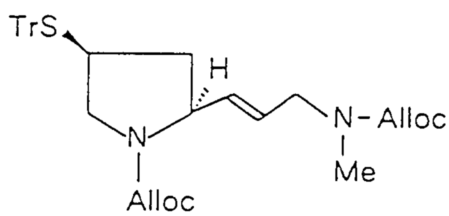

To a tetrahydrofuran solution (40 ml) of the compound (3.0 g, 6.2 mmol) obtained by the above reaction, methane-sulfonyl chloride (0.514 ml, 6,64 mmol) and triethylamine (0.95 ml, 0.683 mmol) were added in a nitrogen stream under cooling with ice, and the mixture was stirred at the same temperature for 30 minutes. Then, a 40% methylamine solution in methanol (2.5 g) was added thereto, and the mixture was stirred at room temperature for 4 hours. The reaction mixture was concentrated under reduced pressure and then extracted with ethyl acetate (100 ml). The organic layer was washed sequentially with water and a saturated sodium chloride aqueous solution, dried over anhydrous magnesium sulfate and then concentrated under reduced pressure. The residue was dissolved in a tetrahydrofuran solution (60 ml), and triethylamine (0.95 ml, 6.83 mmol) and allyl chloroformate (0.66 ml, 6.22 mmol) were added under cooling with ice. The mixture was stirred at the same temperature for one hour. The reaction mixture was extracted with ethyl acetate (100 ml). The organic layer was washed sequentially with water and a saturated sodium chloride aqueous solution and then concentrated under reduced pressure. The residue was subjected to silica gel column chromatography [Wakogel™ C-300, hexane-ethyl acetate (3:1)] to obtain (2S,4S)-N-allyloxycarbonyl-2-[(E)-3-(N-allyloxycarbonyl-N-methylamino)-1-propenyl]-4-tritylthiopyrrolidine (1.73 g, yield: 47.9%).

NMR($CDCl_3$) δ:

1.55(1H,m),2.08(1H,m),2.58-3.44(6H,m),3.84(2H,m), 4.08(1H,m),4.35-4.62(4H,m),5.06-5.38(4H,m), 5.48-(2H,m),5.88(2H,m),7.15-7.58(15H,m)

4)

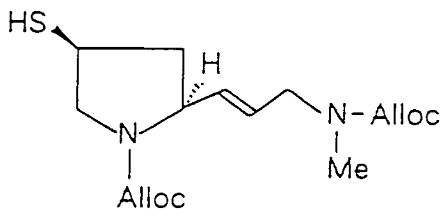

The same procedure as in Reference Example 2-4 was carried out by using the compound (1.73 g, 2.97 mmol) obtained by the above reaction, to obtain the above-identified compound (850 mg, yield: 84.6%).

NMR($CDCl_3$) δ:

1.70(1H,m),2.58(1H,m),2.86(3H,s),3.10-3.38(2H,m), 3.88(2H,m),4.06(1H,m),4.34(1H,m),4.45-4.66(4H,m), 5.10-5.40(4H,m),5.60(2H,m),5.95(2H,m)

REFERENCE EXAMPLE 7

(2S,4S)-N-allyloxycarbonyl-2-[(Z)-3-(allyloxycarbonylamino)-2-methyl-1-propenyl]-4-mercaptopyrrolidine

1)

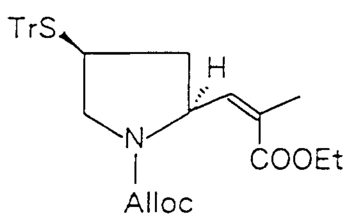

The same procedure as in Reference Example 6-1 was carried out by using (2S,4S)-N-allyloxycarbonyl-2-hydroxymethyl-4-tritylthiopyrrolidine (8.19 g, 17.8 mmol) and ethyl 2-diethylphosphonopropionate (4.76 g, 20.0 mmol) to obtain ethyl 3-[(2S,4S)-N-allyloxycarbonyl-4-tritylthiopyrrolidin-2-yl]-2-methylacrylate (8.58 g, yield: 91.6%).

2)

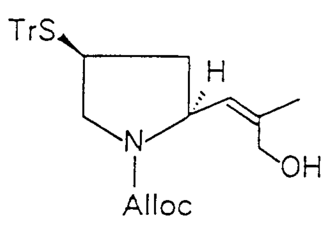

The same procedure as in Reference Example 2-1 was carried out by using the compound (8.58 g, 15.9 mmol) obtained by the above reaction, to obtain (2S,4S)-N-allyloxycarbonyl-2-[(Z)-3-hydroxy-2-methyl-1-propenyl]-4-tritylthiopyrrolidine (1.90 g, yield: 24%, less polar compound) and E-isomer (2.26 g, yield: 28.6%, polar compound).

Z-isomer (less polar compound)

NMR($CDCl_3$) δ:
1.50(1H,m),1.80(3H,s),2.17(1H,m),2.62-3.02(3H,m), 3.62(1H,m),4.08(1H,m),4.32-4.62(4H,m), 5.02-(1H,d,J=10Hz),5.12-5.34(2H,m),5.88(1H,m), 7.15-7.60(15H,m)

E-isomer (polar compound)

NMR($CDCl_3$) δ:
1.45(1H,m),1.65(3H,br s),2.02(1H,m),2.60-3.34(3H,m), 3.97(2H,br s),4.15-4.58(3H,m),5.05-5.32(3H,m), 5.83-(1H,m),7.14-7.54(15H,m)

3)

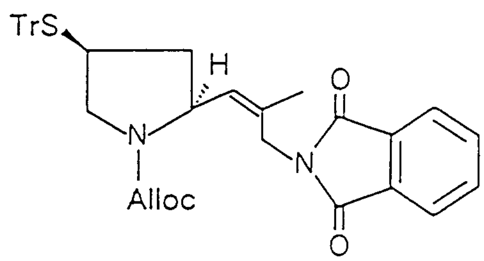

The same procedure as in Reference Example 2-2 was carried out by using the Z-isomer (1.88 g, 3.78 mmol) obtained by the above reaction, to obtain (2S,4S)-N-allyloxycarbonyl-2-[(Z)-2-methyl-3-phthalimido-1-propenyl]-4-tritylthiopyrrolidine (2.089 g, yield: 88.2%).
NMR($CDCl_3$) δ:
1.40-1.76(4H,m),2.70-4.30(3H,m),4.04-4.86(5H,m), 5.10-5.36(3H,m),5.87(1H,m),7.18-7.60(15H,m), 7.76(2H,m)-,7.90(2H,m)

4)

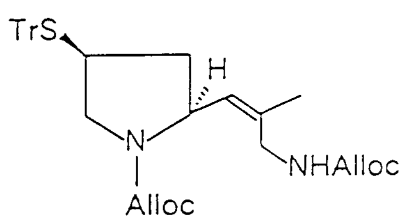

The same procedure as in Reference Example 2-3 was carried out by using the compound (2.08 g, 3.32 mmol) obtained by the above reaction, to obtain (2S,4S)-N-allyloxycarbonyl-2-[(Z)-3-allyloxycarbonylamino-2-methyl-1-propenyl]-4-tritylthiopyrrolidine (1.26 g, yield: 65.3%).
NMR($CDCl_3$) δ:
1.50(1H,m),1.72(3H,s),2.10(1H,m),2.60-3.06(3H,m), 3.80(2H,m),4.20-4.68(7H,m),4.96-5.40(6H,m), 5.92(2H,m)-,7.15-7.58(15H,m)

5)

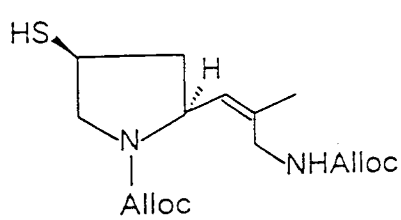

The same procedure as in Reference Example 2-4 was carried out by using the compound (640 mg, 1.1 mmol) obtained by the above reaction, to obtain the above-identified compound (326 mg, yield: 85.9%).
NMR($CDCl_3$) $\delta$:
1.60(1H,m),1.78(3H,s),2.58(1H,m),3.08-3.36(2H,m), 3.88(2H,m),4.02(1H,m),4.44-4.82(5H,m), 5.10-5.44(5H,m),5.80-6.16(3H,m)

REFERENCE EXAMPLE 8

(2S,4S)-N-allyloxycarbonyl-2-[(E)-3-(allyloxycarbonylamino)-2-methyl-1-propenyl]-4-mercaptopyrrolidine

1)

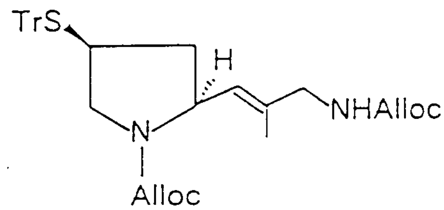

The same procedures as in Reference Example 2-2 and 2-3 were carried out by using (2S,4S)-N-allyloxycarbonyl-2-[(E)-3-hydroxy-2-methyl-1-propenyl]-4-tritylthiopyrrolidine (2.24 g, 4.5 mmol) to obtain (2S,4S)-N-allyloxycarbonyl-2-[(E)-3-allyloxycarbonylamino-2-methyl-1-propenyl]-4-tritylthiopyrrolidine (1.48 g, yield: 56.7%).
NMR($CDCl_3$) $\delta$:
1.46(1H,m),1.64(3H,s),2.02(1H,m),2.62-3.40(3H,m), 3.68(2H,m),4.12-4.72(6H,m),5.08-5.40(5H,m), 5.88(2H,m),7.14-7.52(15H,m)

2)

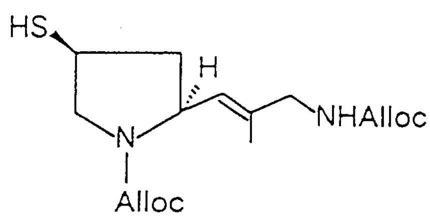

The same procedure as in Reference Example 2-4 was carried out by using the compound (860 mg, 1.45 mmol) obtained by the above reaction, to obtain the above-identified compound (463 mg, yield: 90.8%).
NMR($CDCl_3$) $\delta$:
1.47-1.82(4H,m),2.57(1H,m),3.10-3.36(2H,m), 3.72(2H,m),4.02(1H,m),4.40-4.66(5H,m), 5.12-5.40(5H,m),5.78-6.04(2H,m)

REFERENCE EXAMPLE 9

(2S,4S)-N-allyloxycarbonyl-2-[(Z)-3-(N-allyloxycarbonyl-N-methylamino)-2-methyl-1-propenyl]-4-mercaptopyrrolidine

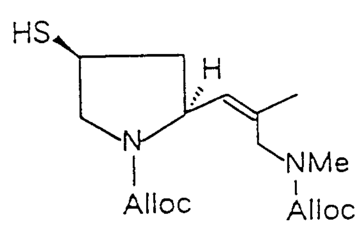

The same procedures as in Reference Example 3-1 and 3-2 were carried out by using (2S,4S)-N-allyloxycarbonyl-2-[(Z)-3-(allyloxycarbonylamino)-2-methyl-1-propenyl]-4-tritylthiopyrrolidine (600 mg, 1.03 mmol) to obtain the above-identified compound (308 mg, yield: 84.6%).
NMR($CDCl_3$) $\delta$:
1.48-1.75(4H,m),2.76(1H,m),2.88(3H,br s), 3.22(2H,m),3.76-4.32(4H,m),4.44-4.70(4H,m), 5.15-5.42(5H,m)-,5.92(2H,m)

REFERENCE EXAMPLE 10

(2S,4S)-N-allyloxycarbonyl-2-[(E)-3-(N-allyloxycarbonyl-N-methylamino)-2-methyl-1-propenyl]-4-mercaptopyrrolidine

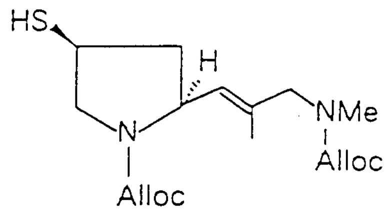

The same procedures as in Reference Example 3-1 and 3-2 were carried out by using (2S,4S)-N-allyloxycarbonyl-2-[(E)-3-(allyloxycarbonylamino)-2-methyl-1-propenyl]-4-tritylthiopyrrolidine (600 mg, 1.03 mmol) to obtain the above-identified compound (295 mg, yield: 81.0%).
NMR($CDCl_3$) $\delta$:
1.46-1.75(4H,m),2.60(1H,m),2.82(3H,s),3.25(2H,m), 3.86(2H,m),4.04(1H,m),4.46-4.68(5H,m), 5.14-5.42(5H,m)-,5.92(2H,m)

REFERENCE EXAMPLE 11

(2S,4S)-N-allyloxycarbonyl-2-[1-[(N-allyloxycarbonylamino)methyl]vinyl]-4-mercaptopyrrolidine

1)

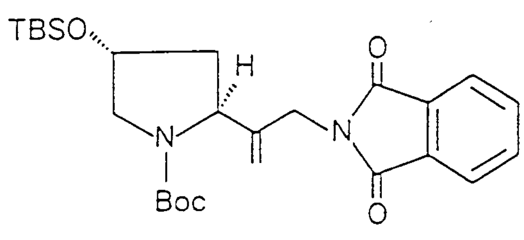

To a tetrahydrofuran solution (25 ml) of (2S,4R)-N-tert-butoxycarbonyl-4-tert-butyldimethylsiloxy-2-[1-(hydroxymethyl)vinyl]pyrrolidine (2.45 g, 6.9 mmol, compound of Reference Example 5-4 of Japanese Patent Application No. 263270/1991), phthalimide (1.77 g, 12.0 mmol), triphenylphosphine (2.75 g, 10.5 mmol) and diethyl azodicarboxylate (1.65 ml, 10.5 mmol) were added at 0 ° C, and the mixture was stirred at the same temperature for two hours. The reaction solution was concentrated under reduced pressure, and the residue was dissolved in a solvent mixture of ethyl acetate (15 ml) and heptane (15 ml) and left to stand overnight. Precipitated solid was filtered and washed with heptane-ethyl acetate (2:1). The filtrate and the washing solution were put together and distilled under reduced pressure. The residue was subjected to silica gel column chromatography [Wakogel™ C-300, heptane-ethyl acetate (4:1)] to obtain (2S,4R)-N-tert-butoxycarbonyl-4-tert-butyldimethylsiloxy-2-[1-(phthalimidomethyl)vinyl]pyrrolidine (2.85 g, yield: 84%).
NMR($CDCl_3$) $\delta$:
0.05(6H,s),0.87(9H,s),1.39(9H,s),2.07(2H,m), 3.51(2H,br s),4.10-4.27(2H,m),4.37(2H,m), 4.83(1H,br s),4.99-(1H,br s),7.65-7.90(4H,m)

2)

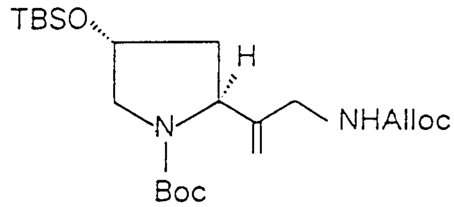

Hydrazine monohydrate (0.85 ml, 17.6 mmol) was added to an ethanol solution (40 ml) of (2S,4R)-N-tert-butoxycarbonyl-4-tert-butyldimethylsiloxy-2-[1-(phthalimidomethyl)vinyl]pyrrolidine (2.85 g, 5.86 mmol) at room temperature, and the mixture was stirred overnight at the same temperature. The reaction mixture was filtered and washed with ethanol. The filtrate and the washing solution were put together and distilled under reduced pressure. To the residue, ethyl acetate and 8% aqueous ammonia were added and subjected to liquid separation. Then, the organic layer was dried over anhydrous sodium sulfate and distilled under reduced pressure to obtain 2.11 g of an oily substance. To a dry methylene chloride solution (30 ml) of this oily substance, allyl chloroformate (1.92 ml, 18 mmol) and triethylamine (3.34 ml, 24 mmol) were added at 0°C, and the mixture was stirred overnight at the same temperature. The reaction solution was concentrated under reduced pressure, and the residue was subjected to silica gel column chromatography [Wakogel™ C-300, heptane-ethyl acetate (4:1)] to obtain (2S,4R)-2-[1-[(N-allyloxycarbonylamino)methyl]-vinyl]-N-tert-butoxycarbonyl-4-tert-butyldimethylsiloxypyrrolidine (2.15 g, yield: 82%).

NMR($CDCl_3$) δ:

0.04(6H,s),0.86(9H,s),1.42(9H,s),1.86(1H,m), 2.06(1H,m),3.46(2H,m),3.76(2H,m),4.32(1H,m), 4.42(1H,m),4.60-(2H,br d),5.00(2H,br s), 5.12-5.38(3H,m),5.92(1H,m)

3)

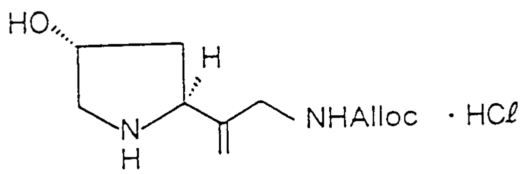

A 3N hydrogen chloride solution in methanol was added to (2S,4R)-2-[1-[(N-allyloxycarbonylamino)-methyl]vinyl]-N-tert-butoxycarbonyl-4-tert-butyldimethylsiloxypyrrolidine (2.15 g, 4.88 mmol) at 0°C, and the mixture was stirred at room temperature for 3 hours. The reaction solution was distilled under reduced pressure to obtain crude (2S,4R)-2-[1-[(N-allyloxycarbonylamino)methyl]vinyl]-4-hydroxypyrrolidine monohydrochloride (1.14 g).

NMR($D_2O$) δ:

2.03-2.35(2H,m),3.29(1H,br d),3.60(1H,m), 3.83(2H,br s),4.36(1H,m),4.55(2H,br d), 4.69(2H,br s),5.15-5.23-(4H,m),5.93(1H,m)

4)

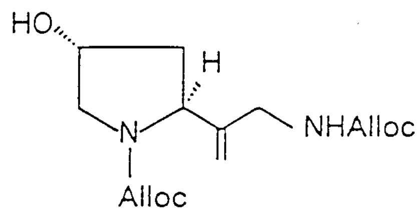

(2S,4R)-2-[1-[(N-allyloxycarbonylamino)methyl]vinyl]-4-hydroxypyrrolidine monohydrochloride (1.14 g, 4.4 mmol) was added to dioxane (15 ml) and water (10 ml). To this mixture, allyl chloroformate (0.63 ml, 6

mmol) was dropwise added while adjusting the pH to a level of from 8 to 9 with a 3N sodium hydroxide aqueous solution at 0 ° C. After the dropwise addition, the mixture was stirred at the same temperature for 10 minutes. The reaction mixture was distilled under reduced pressure, extracted with ethyl acetate (80 ml), washed with a saturated sodium hydrogencarbonate solution (40 ml) and a saturated sodium chloride aqueous solution (30 ml), dried over anhydrous sodium sulfate and then distilled off under reduced pressure. The residue thereby obtained was subjected to silica gel column chromatography [Wakogel™ C-300, heptane-ethyl acetate (1:1)-ethyl acetate] to obtain (2S,4R)-N-allyloxycarbonyl-2-[1-[(N-allyloxycarbonylamino)methyl]vinyl]-4-hydroxypyrrolidine (1.1 g, yield: 80%).
NMR($CDCl_3$) $\delta$:
2.00(1H,m),2.20(1H,m),3.60-4.00(4H,m), 4.60(4H,d,J = 6Hz),5.00-5.64(4H,m),5.94(2H,m)

5)

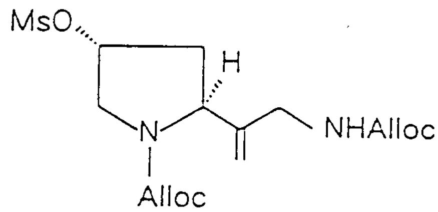

To a methylene chloride solution (10 ml) of (2S,4R)-N-allyloxycarbonyl-2-[1-[(N-allyloxycarbonylamino)-methyl]vinyl]-4-hydroxypyrrolidine (892 mg, 2.87 mmol), methanesulfonyl chloride (0.29 ml, 3.73 mmol) and triethylamine (0.68 ml, 4.88 mmol) were added at 0 ° C, and the mixture was stirred at the same temperature for 15 minutes. To the reaction solution, chloroform (40 ml) was added, and the mixture was washed with dilute hydrochloric acid, a saturated sodium hydrogencarbonate aqueous solution and a saturated sodium chloride aqueous solution, dried over anhydrous sodium sulfate and then distilled under reduced pressure. The residue thereby obtained, was subjected to silica gel column chromatography (Wakogel™ C-300, ethyl acetate) to obtain (2S,4R)-N-allyloxycarbonyl-2-[1-[(N-allyloxycarbonylamino)methyl]vinyl]-4-methanesulfonyloxypyrrolidine (943 mg, yield: 84%).
NMR($CDCl_3$) $\delta$:
2.14(1H,m),2.56(1H,m),3.06(3H,s),3.62-3.94(3H,m), 4.04(1H,m),4.40(4H,br s),5.10(2H,br s), 5.16-5.40(5H,m)-,5.92(2H,m)

6)

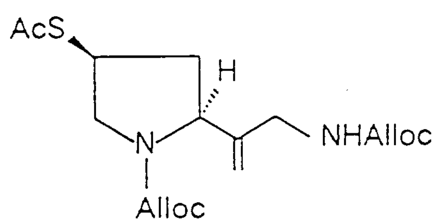

To a dimethylformamide solution (15 ml) of (2S,4R)-N-allyloxycarbonyl-2-[1-[(N-allyloxycarbonylamino)-methyl]vinyl]-4-methanesulfonyloxypyrrolidine (943 mg, 2.43 mmol), potassium thioacetate (560 mg, 4.9 mmol) and potassium iodide (475 mg, 3.16 mmol) were added under a nitrogen stream, and the mixture was stirred at 70 ° C for 3.5 hours. To the reaction mixture, ethyl acetate (100 ml) was added, and the mixture was washed with water (40 ml) and a saturated sodium chloride aqueous solution (40 ml), dried over anhydrous sodium sulfate and then distilled under reduced pressure. The residue thereby obtained, was subjected to silica gel column chromatography [Wakogel™ C-300, heptane-ethyl acetate (2:1)] to obtain (2S,4S)-4-acetylthio-N-allyloxycarbonyl-2-[1-[(N-allyloxycarbonylamino)methyl]vinyl]pyrrolidine (831 mg, yield: 92%).
NMR($CDCl_3$) $\delta$:
1.86(1H,m),2.14(3H,s),2.64(1H,m),3.28(1H,m), 3.70-4.04(4H,m),4.20(1H,m),4.42(1H,t,J = 8Hz), 4.58-(4H,d,J = 7Hz),5.09(2H,br s),5.22-5.42(4H,m), 5.94(2H,m)

7)

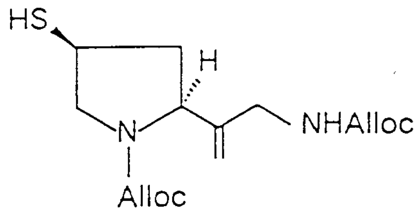

A 1N sodium hydroxide aqueous solution (2.0 ml) was added to a methanol solution (6 ml) of (2S,4S)-4-acetylthio-N-allyloxycarbonyl-2-[1-[(N-allyloxycarbonylamino)methyl]vinyl]pyrrolidine (622 mg, 1.68 mmol) at 0°C, and the mixture was stirred at the same temperature for 15 minutes. To this reaction mixture, 1N hydrochloric acid (2.1 ml) was added, and the pH was adjusted to 4. Then, the mixture was distilled under reduced pressure. To the residue, ethyl acetate (50 ml) and water (20 ml) were added. The organic layer was washed with a saturated sodium chloride aqueous solution, dried over anhydrous sodium sulfate and then distilled under reduced pressure to obtain crude (2S,4S)-N-allyloxycarbonyl-2-[1-[(N-allyloxycarbonylamino)methyl]vinyl]-4-mercaptopyrrolidine (530 mg, yield: 96%).

REFERENCE EXAMPLE 12

(2S,4S)-N-allyloxycarbonyl-2-[1-[(N-allyloxycarbonyl-N-methylamino)methyl]vinyl]-2-mercaptopyrrolidine

1)

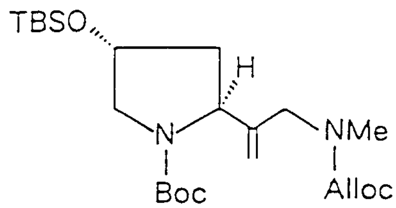

60% sodium hydride (980 mg, 25 mmol) was added to a DMF solution (80 ml) of (2S,4R)-2-[1-[(N-allyloxycarbonylamino)methyl]vinyl]-N-tert-butoxycarbonyl-4-tert-butyldimethylsiloxypyrrolidine (5.42 g, 12.3 mmol) at 0°C, and the mixture was stirred at the same temperature for 25 minutes. Then, methyl iodide (1.56 ml, 25 mmol) was added thereto, and the mixture was stirred overnight at room temperature. To the reaction mixture, water (100 ml) was added, and the mixture was extracted with ethyl acetate (450 ml). The organic layer was washed with water and a saturated sodium chloride aqueous solution, dried over anhydrous sodium sulfate and concentrated under reduced pressure to obtain crude (25,4R)-2-[1-[(N-allyloxycarbonyl-N-methylamino)methyl]vinyl]-N-tert-butoxycarbonyl-4-tert-butyldimethylsiloxypyrrolidine (6.0 g).

NMR($CDCl_3$) $\delta$:

0.04(6H,s),0.85(9H,s),1.41(9H,s),2.88(3H,s), 4.58(2H,br s),4.80(1H,br s),4.96(1H,br s), 5.10-5.32 (2H,m),5.90-(1H,m)

2)

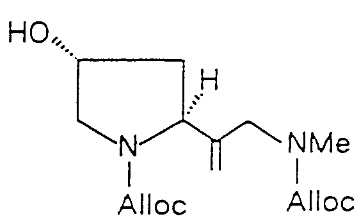

A 4.8N hydrogen chloride solution in methanol (40 ml) was added to the compound (6.0 g) obtained by the above reaction, and the mixture was stirred overnight at room temperature. The reaction solution was concentrated under reduced pressure. To the residue, chloroform (20 ml × 2) was added, and the mixture was concentrated. To the residue, water (30 ml) and dioxane (60 ml) were added, and allyl chlorocarbonate (1.35 ml, 12.9 mmol) was dropwise added thereto, while adjusting the pH to a level of from 9 to 10 with 1N sodium hydroxide under cooling with ice. The mixture was stirred at the same temperature for 20 minutes. The reaction solution was extracted with ethyl acetate (250 ml). The organic layer was washed with water (80 ml × 2) and with a saturated sodium chloride aqueous solution (80 ml × 2), dried over anhydrous sodium sulfate and then concentrated under reduced pressure. The residue was subjected to silica gel column chromatography [Wakogel™ C-300, heptane-ethyl acetate (2:1)→ethyl acetate] to obtain (2S,4R)-N-allyloxycarbonyl-2-[1-[(N-allyloxycarbonyl-N-methylamino)methyl]vinyl]-4-hydroxypyrrolidine (3.09 g, yield: 80.9%).

NMR($CDCl_3$) δ:

1.94(1H,m),2.21(1H,m),2.91(3H,br s),4.43-4.60(6H,m), 4.87(1H,br s),5.01(1H,s),5.10-5.40(4H,m),5.92(2H,m)

3)

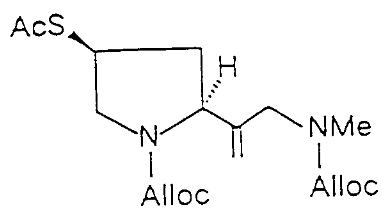

To a methylene chloride solution (40 ml) of the compound (3.09 g, 9.5 mmol) obtained by the above reaction, triethylamine (1.86 ml, 13.3 mmol) and methanesulfonyl chloride (0.88 ml, 11.4 mmol) were added under cooling with ice, and the mixture was stirred at the same temperature for 15 minutes. To the reaction solution, chloroform (100 ml) was added, and the mixture was washed with dilute hydrochloric acid, a saturated sodium hydrogencarbonate aqueous solution and a saturated sodium chloride aqueous solution, dried over anhydrous sodium sulfate and concentrated under reduced pressure. The residue was subjected to silica gel column chromatography [Wakogel™ C-300, heptane-ethyl acetate (1:2)] to obtain 3.0 g of an oily substance. Potassium thioacetate (2.53 g, 22.2 mmol) was added to a DMF solution (30 ml) of the obtained oily substance (3.0 g), and the mixture was stirred under a nitrogen stream at 70°C for 4 hours. To the reaction mixture, ethyl acetate (250 ml) was added, and the mixture was washed with water and a saturated sodium chloride aqueous solution and then dried over anhydrous sodium sulfate. The organic layer was concentrated under reduced pressure. The residue thereby obtained, was subjected to silica gel column chromatography [Wakogel™ C-300, heptane-ethyl acetate (2:1→1:1)] to obtain (2S,4S)-4-acetylthio-N-allyloxycarbonyl-2-[1-[(N-allyloxycarbonyl-N-methylamino)methyl]vinyl]pyrrolidine (3.3 g, yield: 90%).

NMR($CDCl_3$) δ:

1.91(1H,m),2.31(3H,s),2.62(1H,m),2.88(3H,br s), 3.26(1H,m),3.63(1H,m),3.89(1H,m),4.17(1H,m), 4.34(1H,m)-,4.58(4H,m),4.93(1H,br s),5.06(1H,br s), 5.15-5.36(4H,m),5.88(2H,m)

4)

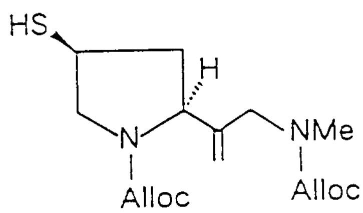

1N sodium hydroxide (8.7 ml) was dropwise added to a methanol solution (30 ml) of the compound (3.3 g, 8.6 mmol) obtained by the above reaction, at 0°C, and the mixture was stirred for 10 minutes. Then, 1N hydrochloric acid (9.0 ml) was dropwise added thereto at the same temperature. The reaction mixture was concentrated and then extracted with ethyl acetate. The organic layer was washed with a saturated sodium chloride aqueous solution, dried over anhydrous sodium sulfate and concentrated under reduced pressure

to obtain a crude product of the above-identified compound (2.87 g).

REFERENCE EXAMPLE 13

(2S,4S)-N-allyloxycarbonyl-2-[2-(N-allyloxycarbonyl-N-methylamino)allyl]-4-mercaptopyrrolidine

1)

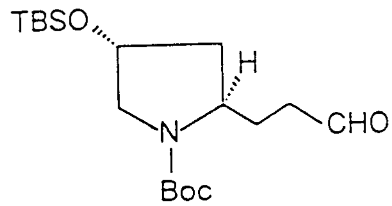

Oxalyl chloride (1.58 ml, 18.5 mmol) was dropwise added to a methylene chloride solution (40 ml) of dimethyl sulfoxide (2.5 ml, 35 mmol) at -70 ° C, and the mixture was stirred at the same temperature for 30 minutes. Then, a methylene chloride solution (10 ml) of (2S,4R)-4-tert-butyldimethylsiloxy-N-tert-butoxycarbonyl-2-(2-hydroxyethyl)pyrrolidine (3.7 g, 10.3 mmol) was dropwise added thereto, and the mixture was stirred at the same temperature for 30 minutes. Then, triethylamine (10 ml, 72 mmol) was dropwise added thereto, and the temperature was raised to room temperature. Water was added to the reaction solution, and the organic layer was washed with dilute hydrochloric acid, a saturated sodium hydrogencarbonate aqueous solution and a saturated sodium chloride aqueous solution, dried over anhydrous sodium sulfate and concentrated under reduced pressure. The obtained oily substance was subjected to silica gel column chromatography [Wakogel™ C-300, heptane-ethyl acetate (4:1)] to obtain (2S,4R)-4-tert-butyldimethylsiloxy-N-tert-butoxycarbonyl-2-(2-formylethyl)pyrrolidine (3.14 g, yield: 85%).
NMR($CDCl_3$) δ:
0.04(6H,s),0.85(9H,s),1.44(9H,s),1.66(1H,m), 1.92(1H,m),2.00(2H,m),2.42(2H,m),3.22-3.60(2H,m), 3.95(1H,m)-,4.31(1H,m),9.74(1H,s)

2)

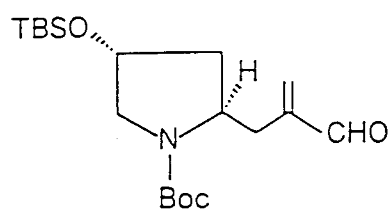

To a methylene chloride solution (40 ml) of the compound (3.14 g, 8.8 mmol) obtained by the above reaction, triethylamine (2.7 ml, 19.4 mmol) and N,N-dimethylmethyleneammonium chloride (1.65 g, 17.6 mmol) were added, and the mixture was stirred overnight at room temperature. The reaction solution was washed with water, dilute hydrochloric acid, a saturated sodium hydrogencarbonate aqueous solution and a saturated sodium chloride aqueous solution, then dried over anhydrous sodium sulfate and concentrated under reduced pressure. The residue was subjected to silica gel column chromatography [Wakogel™ C-300, heptane-ethyl acetate (4:1)] to obtain (2S,4R)-4-tert-butyldimethylsiloxy-N-tert-butoxycarbonyl-2-(2-formylallyl)pyrrolidine (2.33 g, yield: 71%).
NMR($CDCl_3$) δ:
0.03(6H,s),0.84(9H,s),1.46(9H,s),2.40(1H,m), 2.68(1H,m),3.24-3.48(2H,m),4.03(1H,m),4.30(1H,m), 6.06(1H,s)-,6.32(1H,m),9.53(1H,m)

3)

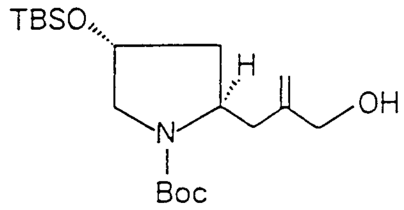

A methanol solution (10 ml) of cerium chloride heptahydrate (2.35 g, 6.3 mmol) was added to a methanol solution (25 ml) of the compound (2.33 g, 6.3 mmol) obtained by the above reaction, at 0°C, and sodium borohydride (240 mg, 6.3 mmol) was added thereto at the same temperature, and the mixture was stirred for 10 minutes. To the reaction solution, water (10 ml) was added, and the mixture was concentrated under reduced pressure. The residue was extracted with ethyl acetate. The organic layer was washed with dilute hydrochloric acid, a saturated sodium hydrogencarbonate aqueous solution and a saturated sodium chloride aqueous solution, dried over anhydrous sodium sulfate and concentrated under reduced pressure. The residue was subjected to silica gel column chromatography [Wakogel™ C-300, heptane-ethyl acetate (3:1)] to obtain (2S,4R)-4-tert-butyldimethylsiloxy-N-tert-butoxycarbonyl-2-[2-(hydroxymethyl)allyl]pyrrolidine (2.24 g, yield: 95%).
NMR($CDCl_3$) $\delta$:
0.06(6H,s),0.87(9H,s),1.45(9H,s),3.25-3.44(2H,m), 4.11(3H,m),4.37(1H,m),4.82(1H,m),5.01(1H,m)

4)

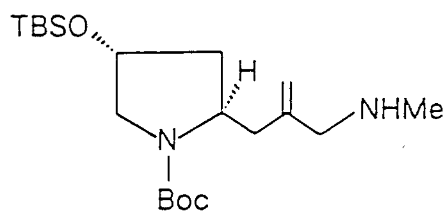

To a methylene chloride solution (30 ml) of the compound (2.24 g, 6.0 mmol) obtained by the above reaction, triethylamine (1.18 ml, 8.44 mmol) and methanesulfonyl chloride (0.56 ml, 7.25 mmol) were dropwise added under cooling with ice, and the mixture was stirred at the same temperature for 15 minutes. The reaction solution was washed with water, dilute hydrochloric acid, a saturated sodium hydrogencarbonate aqueous solution and a saturated sodium chloride aqueous solution, then dried over anhydrous sodium sulfate and concentrated under reduced pressure. To a methanol solution (100 ml) of the obtained oily substance, a 40% methylamine solution in methanol (30 ml) was added, and the mixture was stirred overnight at room temperature. The reaction solution was concentrated under reduced pressure, and the residue was extracted with chloroform (70 ml). The organic layer was washed with 1.5N sodium hydroxide (40 ml) and a saturated sodium chloride aqueous solution (30 ml), then dried over anhydrous sodium sulfate and concentrated under reduced pressure to obtain crude (2S,4R)-4-tert-butyldimethylsiloxy-N-tert-butoxycarbonyl-2-[2-(N-methylaminomethyl)allyl]pyrrolidine (2.24 g).
NMR($CDCl_3$) $\delta$:
0.04(6H,s),0.85(9H,s),1.45(9H,s),2.40(3H,s), 3.14(2H,m),3.20-3.40(2H,m),3.95-4.15(1H,m), 4.83(1H,s),4.96-(1H,s)

5)

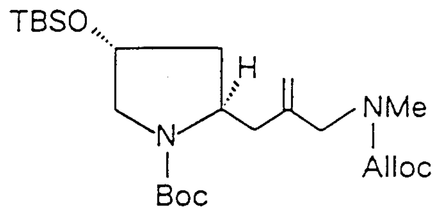

To a methylene chloride solution (40 ml) of the compound (2.24 g, 5.8 mmol) obtained by the above reaction, triethylamine (1.13 ml, 8.1 mmol) and allyl chlorocarbonate (0.74 ml, 7.0 mmol) were added under cooling with ice, and the mixture was stirred at the same temperature for 30 minutes The reaction solution was washed with dilute hydrochloric acid, a saturated sodium hydrogencarbonate aqueous solution and a saturated sodium chloride aqueous solution, then dried over anhydrous sodium sulfate and concentrated under reduced pressure. The residue was subjected to silica gel column chromatography [Wakogel™ C-300, heptane-ethyl acetate (3:1)] to obtain (2S,4R)-2-[2-(N-allyloxycarbonyl-N-methylaminomethyl)allyl]-4-tert-butyldimethylsiloxy-N-tert-butoxycarbonylpyrrolidine (2.46 g, yield: 90%).
NMR($CDCl_3$) $\delta$:
0.04(6H,s),0.86(9H,s),1.45(9H,s),1.71-2.08(3H,m), 2.50-2.70(1H,m),2.88(3H,s),3.37(2H,m), 3.72-4.20(4H,m)-,4.32(1H,m),4.59(2H,m),4.88(2H,m), 5.10-5.38(2H,m),5.91(1H,m)

6)

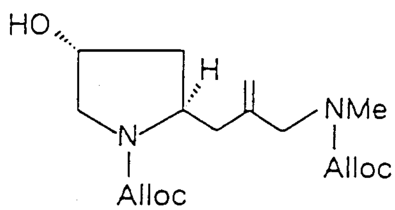

A 3N hydrogen chloride solution in methanol was added to the compound (2.46 g, 5.2 mmol) obtained by the above reaction, at room temperature, and the mixture was stirred overnight. The reaction solution was concentrated under reduced pressure, and to the residue, water (20 ml) and dioxane (40 ml) were added. Then, allyl chlorocarbonate (0.67 ml, 6.3 mmol) was dropwise added thereto while adjusting the pH to a level of from 9 to 10 with an aqueous sodium hydroxide solution under cooling with ice, and the mixture was stirred at the same temperature for 10 minutes. The reaction solution was extracted with ethyl acetate (150 ml), and the organic layer was washed with water and a saturated sodium chloride aqueous solution, then dried over anhydrous sodium sulfate and concentrated under reduced pressure. The residue was subjected to silica gel column chromatography [Wakogel™ C-300, heptane-ethyl acetate (2:1)→ethyl acetate] to obtain (2S,4R)-N-allyloxycarbonyl-2-[2-(N-allyloxycarbonyl-N-methylaminomethyl)allyl]-4-hydroxypyrrolidine (1.41 g, yield: 79%).
NMR($CDCl_3$) $\delta$:
1.72-2.24(3H,m),2.55-2.85(1H,m),2.86(3H,s), 3.40-3.72(2H,m),3.84(2H,m),4.17(1H,m),4.40(1H,m), 4.58(4H,m)-,4.89(2H,br s),5.10-5.38(4H,m),5.90(2H,m)

7)

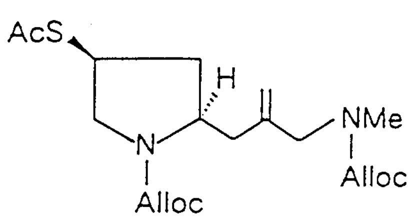

To a methylene chloride solution (30 ml) of the compound (1.41 g, 4.17 mmol) obtained by the above reaction, triethylamine (0.81 ml, 5.0 mmol) and methanesulfonyl chloride (0.39 ml, 5.0 mmol) were added under cooling with ice, and the mixture was stirred at the same temperature for 10 minutes. The reaction solution was washed with dilute hydrochloric acid, a saturated sodium hydrogencarbonate aqueous solution and a saturated sodium chloride aqueous solution, then dried over anhydrous sodium sulfate and concentrated under reduced pressure. Potassium thioacetate (1.42 g, 12.5 mmol) was added to a DMF solution (30 ml) of the obtained oily substance, and the mixture was stirred under a nitrogen stream at 70 °C for 3 hours. To the reaction mixture, ethyl acetate was added, and the organic layer was washed with water and a saturated sodium chloride aqueous solution, then dried over anhydrous sodium sulfate and concentrated under reduced pressure. The residue was subjected to silica gel column chromatography [Wakogel™ C-300, heptane-ethyl acetate (2:1→1:1)] to obtain (2S,4S)-4-acetylthio-N-allyloxycarbonyl-2-[2-(N-allyloxycarbonyl-N-methylaminomethyl)allyl]pyrrolidine (1.36 g, yield: 82%).

NMR($CDCl_3$) $\delta$:

1.62-1.80(1H,m),2.02-2.18(1H,m),2.32(3H,s), 2.86(3H,s),3.21(1H,m),3.86(3H,m),4.10(2H,m), 4.58(4H,br s),4.90(2H,br s),5.10-5.38(4H,m), 5.90(2H,m)

8)

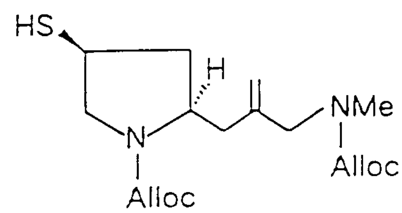

1N sodium hydroxide (3.5 ml) was dropwise added to a methanol solution (10 ml) of the compound (1.36 g, 3.43 mmol) obtained by the above reaction under cooling with ice, and the mixture was stirred at the same temperature for 10 minutes. 1N hydrochloric acid (3.7 ml) was added to the mixture at 0 °C, and the reaction mixture was concentrated. The residue was extracted with ethyl acetate. The organic layer was washed with a saturated sodium chloride aqueous solution, then dried over anhydrous sodium sulfate and concentrated under reduced pressure to obtain a crude product of the above-identified compound (1.22 g).

## INDUSTRIAL APPLICABILITY

The compounds of the present invention are novel compounds not disclosed any literatures and have strong antibacterial activities against sensitive and resistant gram positive bacteria and gram negative bacteria and excellent stability against $\beta$-lactamase and DHP-I. Therefore, they are useful as antibacterial agents.

## Claims

**1.** A compound of the formula (I):

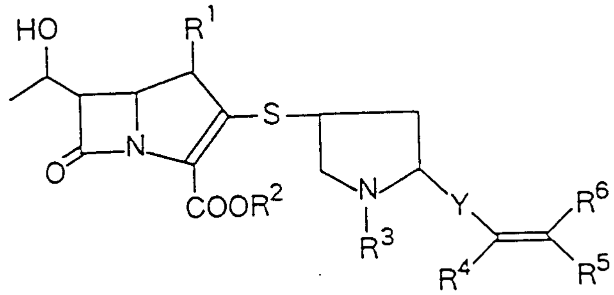

(I)

wherein $R^1$ is a hydrogen atom or a methyl group, $R^2$ is a hydrogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which

may have substituents or a group of the formula -X-N($R^7$)$R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula -X-N($R^7$)$R^8$ wherein $R^7$, $R^8$ and X are as defined above, and Y is a lower alkylene group or a single bond; or a pharmaceutically acceptable salt or ester thereof.

**2.** The compound according to Claim 1, which is a compound of the formula (I-a):

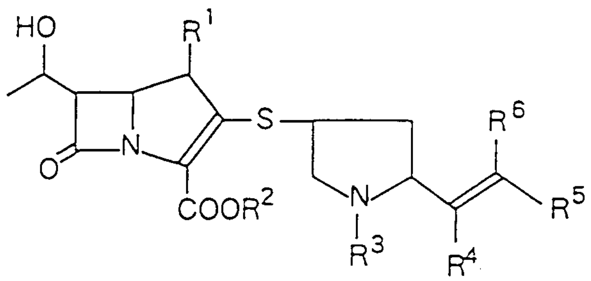

(I-a)

wherein $R^1$ is a hydrogen atom or a methyl group, $R^2$ is a hydrogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula -X-N($R^7$)$R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8- membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula -X-N($R^7$)$R^8$ wherein $R^7$, $R^8$ and X are as defined above; or a pharmaceutically acceptable salt or ester thereof.

**3.** The compound according to Claim 1, which is a compound of the formula (I-b):

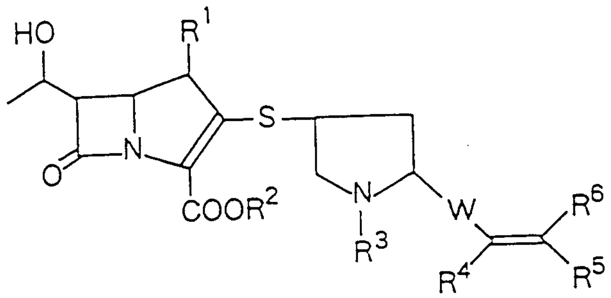

(I-b)

wherein $R^1$ is a hydrogen atom or a methyl group, $R^2$ is a hydrogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula -X-N($R^7$)$R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8- membered heterocyclic group which may be substituted

(provided that either the nitrogen atom adjacent to X or other nitrogen atom on the heterocyclic ring may form an ammonio group), and X is a lower alkylene group which may have substituents, provided that at least one of $R^4$, $R^5$ and $R^6$ is the group of the formula $-X-N(R^7)R^8$ wherein $R^7$, $R^8$ and X are as defined above, and W is a lower alkylene group; or a pharmaceutically acceptable salt or ester thereof.

**4.** The compound according to Claim 1, wherein $R^5$ or $R^6$ is a group of the formula $-(CH_2)_n-N(R^{70})R^{80}$ wherein each of $R^{70}$ and $R^{80}$ which may be the same or different, a hydrogen atom or a lower alkyl group, or $R^{70}$ and $R^{80}$ form, together with the adjacent nitrogen atom, a 3- to 8- heterocyclic group, and n is an integer of from 1 to 4.

**5.** The compound according to Claim 1, wherein $R^5$ or $R^6$ is a group of the formula $-CH_2-N(R^{77})R^{88}$ wherein each of $R^{77}$ and $R^{88}$ which may be the same or different, is a hydrogen atom or a lower alkyl group, or $R^{77}$ and $R^{88}$ form, together with the adjacent nitrogen atom, a 4- to 6- membered heterocyclic group.

**6.** The compound according to Claim 1, wherein each of $R^5$ and $R^6$ is a hydrogen atom.

**7.** The compound according to Claim 1, wherein $R^5$ or $R^6$ is a group of the formula $-CH_2-NH_2$, $-CH_2-NHCH_3$ or $-CH_2-N(CH_3)_2$.

**8.** The compound according to Claim 1, which is:

(5R,6S)-2-[(2S,4S)-2-[(E)-3-amino-1-propenyl]pyrrolidin-4-ylthio]-6-[(R)-1-hydroxyethyl]-1-carbapen-2-em-3-carboxylic acid,

(1R,5S,6S)-2-[(2S,4S)-2-[(E)-3-amino-1-propenyl]pyrrolidin-4-ylthio]-6-[(R)-1-hydroxyethyl]-1-methyl-1-carbapen-2-em-3-carboxylic acid,

(5R,6S)-2-[(2S,4S)-2-[(Z)-3-amino-1-propenyl]pyrrolidin-4-ylthio]-6-[(R)-1-hydroxyethyl]-1-carbapen-2-em-3-carboxylic acid,

(1R,5S,6S)-2-[(2S,4S)-2-[(Z)-3-amino-1-propenyl]pyrrolidin-4-ylthio]-6-[(R)-1-hydroxyethyl]-1-methyl-1-carbapen-2-em-3-carboxylic acid,

(5R,6S)-6-[(R)-1-hydroxyethyl]-2-[(2S,4S)-2-[(Z)-3-(N-methylamino)-1-propenyl]pyrrolidin-4-ylthio]-1-carbapen-2-em-3-carboxylic acid,

(1R,5S,6S)-6-[(R)-1-hydroxyethyl]-1-methyl-2-[(2S,4S)-2-[(Z)-3-(N-methylamino)-1-propenyl]-pyrrolidin-4-ylthio]-1-carbapen-2-em-3-carboxylic acid,

(5R,6S)-2-[(2S,4S)-2-[(Z)-3-(N,N-dimethylamino)-1-propenyl]pyrrolidin-4-ylthio]-6-[(R)-1-hydroxyethyl]-1-carbapen-2-em-3-carboxylic acid,

(1R,5S,6S)-2-[(2S,4S)-2-[(Z)-3-(N,N-dimethylamino)-1-propenyl]pyrrolidin-4-ylthio]-6-[(R)-1-hydroxyethyl]-1-methyl-1-carbapen-2-em-3-carboxylic acid,

(5R,6S)-6-[(R)-1-hydroxyethyl]-2-[(2S,4S)-2-[(Z)-3-(1-pyrrolidinyl)-1-propenyl]pyrrolidin-4-ylthio]-1-carbapen-2-em-3-carboxylic acid,

(1R,5S,6S)-6-[(R)-1-hydroxyethyl]-1-methyl-2-[(2S,4S)-2-[(Z)-3-(1-pyrrolidinyl)-1-propenyl]pyrrolidin-4-ylthio]-1-carbapen-2-em-3-carboxylic acid,

(5R,6S)-6-[(R)-1-hydroxyethyl]-2-[(2S,4S)-2-[(E)-3-(N-methylamino)-1-propenyl]pyrrolidin-4-ylthio]-1-carbapen-2-em-3-carboxylic acid,

(1R,5S,6S)-6-[(R)-1-hydroxyethyl]-1-methyl-2-[(2S,4S)-2-[(E)-3-(N-methylamino)-1-propenyl]-pyrrolidin-4-ylthio]-1-carbapen-2-em-3-carboxylic acid,

(5R,6S)-2-[(2S,4S)-2-[(Z)-3-amino-2-methyl-1-propenyl]pyrrolidin-4-ylthio]-6-[(R)-1-hydroxyethyl]-1-carbapen-2-em-3-carboxylic acid,

(1R,5S,6S)-2-[(2S,4S)-2-[(Z)-3-amino-2-methyl-1-propenyl]pyrrolidin-4-ylthio]-6-[(R)-1-hydroxyethyl]-1-methyl-1-carbapen-2-em-3-carboxylic acid,

(5R,6S)-2-[(2S,4S)-2-[(E)-3-amino-2-methyl-1-propenyl]pyrrolidin-4-ylthio]-6-[(R)-1-hydroxyethyl]-1-carbapen-2-em-3-carboxylic acid,

(1R,5S,6S)-2-[(2S,4S)-2-[(E)-3-amino-2-methyl-1-propenyl]pyrrolidin-4-ylthio]-6-[(R)-1-hydroxyethyl]-1-methyl-1-carbapen-2-em-3-carboxylic acid,

(5R,6S)-6-[(R)-1-hydroxyethyl]-2-[(2S,4S)-2-[(Z)-3-(N-methylamino)-2-methyl-1-propenyl]pyrrolidin-4-ylthio]-1-carbapen-2-em-3-carboxylic acid,

(1R,5S,6S)-6-[(R)-1-hydroxyethyl]-2-[(2S,4S)-2-[(Z)-3-(N-methylamino)-2-methyl-1-propenyl]-pyrrolidin-4-ylthio]-1-methyl-1-carbapen-2-em-3-carboxylic acid,

(5R,6S)-6-[(R)-1-hydroxyethyl]-2-[(2S,4S)-2-[(E)-3-(N-methylamino)-2-methyl-1-propenyl]pyrrolidin-4-ylthio]-1-carbapen-2-em-3-carboxylic acid,

(1R,5S,6S)-6-[(R)-1-hydroxyethyl]-2-[(2S,4S)-2-[(E)-3-(N-methylamino)-2-methyl-1-propenyl]-pyrrolidin-4-ylthio]-1-methyl-1-carbapen-2-em-3-carboxylic acid,

(5R,6S)-2-[(2S,4S)-2-[1-(aminomethyl)vinyl]pyrrolidin-4-ylthio]-6-[(R)-1-hydroxyethyl]-1-carbapen-2-em-3-carboxylic acid,

(1R,5S,6S)-2-[(2S,4S)-2-[1-(aminomethyl)vinyl]pyrrolidin-4-ylthio]-6-[(R)-1-hydroxyethyl]-1-methyl-1-carbapen-2-em-3-carboxylic acid,

(5R,6S)-6-[(R)-1-hydroxyethyl]-2-[(2S,4S)-2-[1-[(N-methylamino)methyl]vinyl]pyrrolidin-4-ylthio]-1-carbapen-2-em-3-carboxylic acid,

(1R,5S,6S)-6-[(R)-1-hydroxyethyl]-1-methyl-2-[(2S,4S)-2-[1-[(N-methylamino)methyl]vinyl]pyrrolidin-4-ylthio]-1-carbapen-2-em-3-carboxylic acid,

(5R,6S)-6-[(R)-1-hydroxyethyl]-2-[(2S,4S)-2-[2-[(N-methylamino)methyl]allyl]pyrrolidin-4-ylthio]-1-carbapen 2-em-3-carboxylic acid or

(1R,5S,6S)-6-[(R)-1-hydroxyethyl]-1-methyl-2-[(2S,4S)-2-[2-[(N-methylamino)methyl]allyl]pyrrolidin-4-ylthio]-1-carbapen-2-em-3-carboxylic acid.

**9.** The compound according to Claim 1, which is (1R,5S,6S)-6-[(R)-1-hydroxyethyl]-1-methyl-2-[(2S,4S)-2-[(E)-3-(N-methylamino)-1-propenyl]pyrrolidin-4-ylthio]-1-carbapen-2-em-3-carboxylic acid.

**10.** The compound according to Claim 1, wherein the steric configuration of the compound of the formula (I) is (5R,6S,8R) or (1R,5S,6S,8R).

**11.** A process for producing a compound of the formula (I):

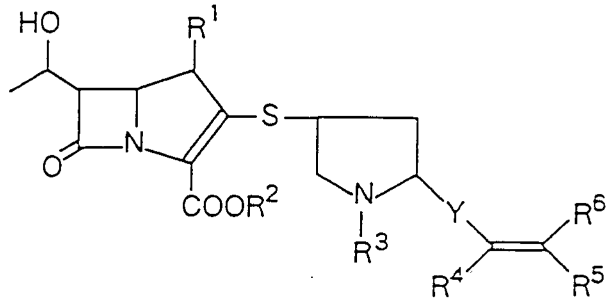

(I)

wherein $R^1$ is a hydrogen atom or a methyl group, $R^2$ is a hydrogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula $-X-N(R^7)R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula $-X-N(R^7)R^8$ wherein $R^7$, $R^8$ and X are as defined above, and Y is a lower alkylene group or a single bond; or a pharmaceutically acceptable salt or ester thereof, which comprises reacting a compound of the formula (II):

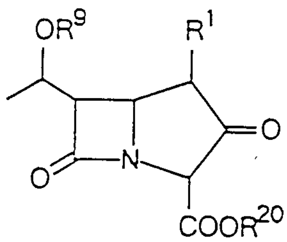

(II)

wherein $R^1$ is as defined above, $R^9$ is a hydrogen atom or a hydroxyl-protecting group, and $R^{20}$ is a hydrogen atom or a carboxyl-protecting group, or a reactive derivative thereof, with a compound of the formula (III):

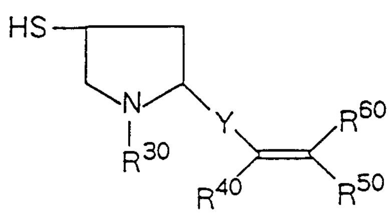

(III)

wherein $R^{30}$ is a hydrogen atom, a lower alkyl group or an imino-protecting group, each of $R^{40}$, $R^{50}$ and $R^{60}$ is a hydrogen atom, a cyano group, a methanesulfonyl group, a lower alkyl group which may have substituents, a carbamoyl group which may have substituents or a group of the formula $-X^1-N(R^{71})R^{81}$ {wherein each of $R^{71}$ and $R^{81}$ which may be the same or different, is a hydrogen atom, a lower alkyl group which may have substituents or an amino-protecting group, or $R^{71}$ and $R^{81}$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8- membered heterocyclic group which may be substituted (provided that either the nitrogen atom adjacent to $X^1$ or other nitrogen atom on the heterocyclic ring may form an ammonio group), and $X^1$ is a lower alkylene group which may have substituents}, provided that at least one of $R^{40}$, $R^{50}$ and $R^{60}$ is the group of the formula $-X^1-N(R^{71})R^{81}$ wherein $R^{71}$, $R^{81}$ and $X^1$ are as defined above, and Y is a lower alkylene group or a single bond (provided that the substituents of the lower alkyl group, the heterocyclic group and the lower alkylene group, as well as the carbamoyl group and the imino group on the heterocyclic ring, may be protected, as the case requires), to obtain a compound of the formula (IV):

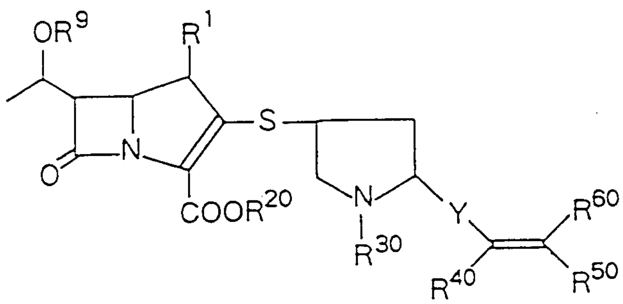

(IV)

wherein $R^1$, $R^9$, $R^{20}$, $R^{30}$, $R^{40}$, $R^{50}$ and $R^{60}$ are as defined above, and if necessary, removing any protecting group of the compound of the formula (IV).

**12.** An antibacterial agent comprising an antibacterially effective amount of the compound of the formula (I):

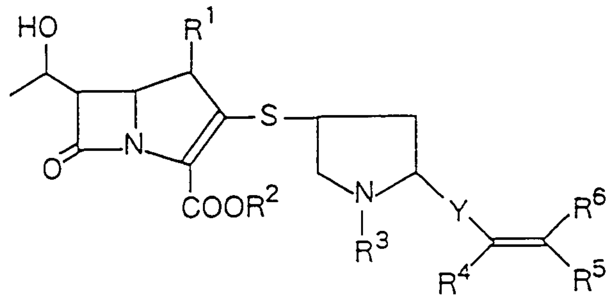

(I)

wherein $R^1$ is a hydrogen atom or a methyl group, $R^2$ is a hydrogen atom or a negative charge, $R^3$ is a hydrogen atom or a lower alkyl group, each of $R^4$, $R^5$ and $R^6$ is a hydrogen atom, a cyano group, a methanesulfonyl group, a lower alkyl group which may have substituents, a carbamoyl group which may have substituents or a group of the formula $-X-N(R^7)R^8$ {wherein each of $R^7$ and $R^8$ which may be the same or different, is a hydrogen atom or a lower alkyl group which may have substituents, or $R^7$ and $R^8$ form, together with the adjacent nitrogen atom and, if necessary, together with a hetero atom selected from the group consisting of a nitrogen atom, an oxygen atom and a sulfur atom in addition to the adjacent nitrogen atom, a 3- to 8-membered heterocyclic group which may be substituted (provided that either the nitrogen atom adjacent to X or other nitrogen atom on the heterocyclic ring may form an ammonio group), and X is a lower alkylene group which may have substituents}, provided that at least one of $R^4$, $R^5$ and $R^6$ is the group of the formula $-X-N(R^7)R^8$ wherein $R^7$, $R^8$ and X are as defined above, and Y is a lower alkylene group or a single bond; or a pharmaceutically acceptable salt or ester thereof, and a pharmaceutically acceptable carrier or diluent.

European Patent Office

# EUROPEAN SEARCH REPORT

Application Number

EP 92 11 1497

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-0 182 213 (SUMITOMO PHARMACEUTICALS CO., LTD.)<br>* claims *<br>--- | 1-12 | C07D477/00<br>A61K31/40 |
| D,Y | EP-A-0 411 664 (BANYU PHARMACEUTICAL CO. LTD.)<br>* claims *<br>----- | 1-12 | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| THE HAGUE | 18 SEPTEMBER 1992 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document